# EUROPEAN PATENT APPLICATION

(11) **EP 4 574 979 A1**
(43) Date of publication of application: **25.06.2025**
(21) Application number: 23854362.3
(22) Date of filing: 11.08.2023
(51) Int. Cl.: C12N 15/62, C12N 5/10, A61P 35/00

(54) **ENGINEERED IMMUNE CELL AND PREPARATION METHOD THEREFOR**

(30) Priority: 16.08.2022 CN 202210982818
(71) Applicant: Cells & Genes Biotech (Shanghai) Co., Ltd, Shanghai 201203 (CN)
(72) Inventor: LI, Linhong, Shanghai 201203 (CN); ZHU, Xiangyang, Shanghai 201203 (CN); YU, Shijun, Shanghai 201203 (CN); XIN, Pengcheng, Shanghai 201203 (CN); WANG, Li, Shanghai 201203 (CN); YANG, Xingxing, Shanghai 201203 (CN); ZHAN, Yifan, Shanghai 201203 (CN)
(74) Representative: Santoro, Sofia
(86) International application number: PCT/CN2023/112683
(87) International publication number: WO 2024/037461

(57) **Abstract**

Provided are an engineered immune cell and a preparation method therefor. The engineered immune cell is prepared by means of transfecting an isolated nucleic acid molecule. The isolated nucleic acid molecule comprises: a nucleic acid sequence encoding a chimeric antigen receptor (CAR); a nucleic acid sequence encoding IL-15 or a functional active fragment thereof; and a nucleic acid sequence encoding IL-21 or a functional active fragment thereof.

## Description

### TECHNICAL FIELD

The present application relates to the field of biomedicine, in particular to an engineered immune cell and preparation method thereof.

### BACKGROUND

Improving cell function through genetic modification is an important means to increase the efficacy of cell therapy. At present, the field of cell therapy mainly uses viral vectors to transfect cells to achieve the stable expression of various molecules, and at the same time have cellular functions including cell viability, expansion rate, and long-term specific functions of the expressed molecules so as to achieve the purpose of cell therapy. However, the design of viral vector transfection is time consuming, the preparation process is complicated, the test period is long, and the cost is high. Meanwhile, the introduction of viral sequences into human hosts possibly triggers host immunogenicity, insertional mutagenesis, and causes unpredictable safety hazards. For example, lentiviral vectors have been reported to cause insertion of exogenous nucleic acid sequences into thousands of sites in the T cell genome, which undoubtedly increases potential safety concerns.

It has been reported that there have been attempts to use non-viral systems for cell transformation or cell transfection, to achieve the purpose of cell therapy. However, the transfection efficiency mediated by the non-viral system is low, and it is highly toxic to most of the transfected cells required in cell therapies, which seriously affects the viability of the transfected cells. In particular, so far, non-viral transfection of immune cells, stem cells, fibroblasts, etc. (especially primary cells) has produced even more cytotoxicity, which results in high cell death rates and low expansion rates. Thus, the efficiency of transfection and the recovery rate of living cells cannot reach the level required by cell therapy. Under the limitations of the existing non-viral methods, in order to achieve the amount of cells required for cell therapy, it is necessary to carry out various remedial measures including long-term directed expansion of cells *in vitro.* However, long-term directed expansion *in vitro* will inevitably change the phenotype and function of the cells and will significantly limit the cell therapy effect of the transfected cells, making the current cellular therapeutic products produced by non-viral methods far less efficient than those produced by viral methods. Considering the limitations of viral methods, the development and maturation of cell therapy is greatly prevented.

Therefore, there is an urgent need of developing a non-viral cell modification method that is safe, has high transfection efficiency and good cell viability, and can produce enough cells for clinical treatment in a short period of time, and an engineered cell prepared through the method.

### SUMMARY OF THE INVENTION

The present application provides an isolated nucleic acid molecule having a novel design, and engineered immune cells obtained by transfection (e.g., electroporation transfection) with the molecule. The engineered immune cells of the present application show excellent safety and tumor cell killing activity.

In the first aspect, the present application provides an isolated nucleic acid molecule comprising: a nucleic acid sequence encoding a chimeric antigen receptor (CAR); a nucleic acid sequence encoding IL-15 or a functionally active fragment thereof; and a nucleic acid sequence encoding IL-21 or a functionally active fragment thereof.

In another preferred embodiment, the nucleic acid molecule further comprises a nucleic acid sequence encoding an additional exogenous protein selected from the group consisting of: IL-2 or a functionally active fragment thereof, IL-7 or a functionally active fragment thereof, a cytokine, BiTE, and a combination thereof.

In another preferred embodiment, the nucleic acid molecule comprises tandem expression units, wherein the expression units comprise:
(E1) a first expression unit for expressing the chimeric antigen receptor;
(E2) a second expression unit for expressing IL-15 or a functionally active fragment thereof or a fusion protein thereof;
(E3) a third expression unit for expressing IL-21 or a functionally active fragment thereof or a fusion protein thereof; and
(E4) optionally, a fourth expression unit for expressing the additional exogenous protein,
wherein the positions of the first, second, third, and fourth expression units are randomly interchangeable.

In the nucleic acid molecule, each independent expression unit is driven by an exogenous or endogenous promoter operably linked thereto, or is driven by a promoter further upstream and has a nucleic acid sequence of a cleavable moiety (such as a 2A nucleic acid sequence or an IRES nucleic acid sequence) at the 5' end.

In another preferred embodiment, the number of the first, second, and third expression units are each independently 1, 2, or 3.

In another preferred embodiment, the number of the fourth expression unit is 0, 1, 2, 3, 4, or 5.

In another preferred embodiment, the nucleic acid molecule encodes 1, 2, or 3 identical or different CAR molecules.

In another preferred embodiment, the nucleic acid molecule has a structure shown in the following Formula I:
ARM5-P1-CAR1-P2/L1-Z1-P3/L2-Z2-(P4/L3-Z3)m--ARM3 (Ia)
wherein,
ARM5 is absent or a 5' homologous arm;
ARM3 is absent or a 3' homologous arm;
P1 is absent, a splicing acceptor, or a first exogenous promoter;
CAR1 is a nucleic acid sequence encoding a first chimeric antigen receptor (CAR);
P2/L1 is a second exogenous promoter P2 or a nucleic acid sequence L1 encoding a cleavable moiety;
one of Z1 and Z2 is a nucleic acid sequence encoding IL-15 or a functionally active fragment thereof or a fusion protein thereof, and the other is a nucleic acid sequence encoding IL-21 or a functionally active fragment thereof or a fusion protein thereof;
L2 is a nucleic acid sequence L2 encoding a cleavable moiety;
P3 is a third exogenous promoter;
P4/L3 are each independently a fourth exogenous promoter P4 or a nucleic acid sequence L3 encoding a cleavable moiety;
Z3 is a nucleic acid sequence encoding an additional exogenous protein; and
m is 0, 1, 2, 3, 4, or 5.

In another preferred embodiment, each P4 is an identical or different exogenous promoter, and each L3 is a nucleic acid sequence encoding an identical or different cleavable moiety.

In another preferred embodiment, each Z3 is a nucleic acid sequence encoding an identical or different additional exogenous protein.

In another preferred embodiment, ARM5 is a 5' homologous arm, and ARM3 is a 3' homologous arm.

In another preferred embodiment, the nucleic acid molecule has a structure shown in the following Formula II:
ARM5-P1-CAR1-L1-Z1-L2-Z2-ARM3 (II)
wherein ARM5, P1, CAR1, L1, Z1, L2, Z2, ARM3, and P2 are as defined above.

In another preferred embodiment, P1, P2, and P3 are each independently a constitutive or inducible promoter.

In another preferred embodiment, P1 is an exogenous promoter, such as a PGK promoter.

In another preferred embodiment, P2 is a PGK promoter.

In another preferred embodiment, the sequence of PGK promoter is as shown in SEQ ID NO: 26.

In another preferred embodiment, the nucleic acid sequence encoding IL-15 or a functionally active fragment thereof encodes IL-15 or a fusion protein thereof, such as an IL-15-Fc fusion protein.

In another preferred embodiment, the nucleic acid sequence encoding IL-21 or a functionally active fragment thereof encodes IL-21 or a fusion protein thereof, such as an IL-21-Fc fusion protein.

In another preferred embodiment, the 5' homologous arm and the 3' homologous arm are homologous to a target region in the genome of an immune cell, thereby enabling the knock-in of the nucleic acid sequence between the homologous arms into a predetermined site.

In another preferred embodiment, the nucleic acid sequence is knocked in at:
(s1) an endogenous promoter or downstream thereto, such that the nucleic acid sequence encoding the chimeric antigen receptor (CAR) is operably linked to and driven by the endogenous promoter, or driven by an exogenous promoter;
(s2) a site of a target gene selected from the group consisting of: TRBC, TRAC, PD-1, CD52, CD95, AAVS1, and CCR5.

In certain embodiments, the nucleic acid molecule comprises, in order from 5' to 3': the nucleic acid sequence encoding the CAR, the nucleic acid sequence encoding IL-15 or a functionally active fragment thereof, and the nucleic acid sequence encoding IL-21 or a functionally active fragment thereof; or the nucleic acid sequence encoding the CAR, the nucleic acid sequence encoding IL-21 or a functionally active fragment thereof, and the nucleic acid sequence encoding IL-15 or a functionally active fragment thereof.

In certain embodiments, the CAR comprises a target-binding domain targeting a tumor associated antigen, a target-binding domain targeting a viral antigen, a target-binding domain targeting an immune-related antigen, or a combination thereof.

In certain embodiments, the tumor associated antigen is selected from the group consisting of: GPC3, CD19, BCMA, GCC (GUCY2C), Her2, Claudin18.2, and Mesothelin; and the viral antigen is selected from the group consisting of: EBV-gp350 and HBV s protein.

In certain embodiments, the nucleic acid molecule further comprises one or more nucleic acid sequences encoding a cleavable moiety.

In certain embodiments, the nucleic acid sequence encoding the cleavable moiety is located between any two sequences selected from the group consisting of: the nucleic acid sequence encoding the chimeric antigen receptor (CAR); the nucleic acid sequence encoding IL-15 or a functionally active fragment thereof; and the nucleic acid sequence encoding IL-21 or a functionally active fragment thereof.

In certain embodiments, the cleavable moiety comprises a 2A peptide.

In certain embodiments, the 2A peptide comprises P2A, T2A, F2A, or E2A.

In certain embodiments, the nucleic acid molecule further comprises a nucleic acid sequence encoding one or more additional exogenous proteins.

In certain embodiments, the one or more additional exogenous proteins comprise an antibody or an antigen-binding fragment thereof.

In certain embodiments, the antibody or antigen-binding fragment comprises a multispecific antibody or antigen-binding fragment thereof.

In certain embodiments, the multispecific antibody or antigen-binding fragment thereof comprises a bispecific T-cell engager (BiTE).

In certain embodiments, the BiTE comprises a CD3 binding domain.

In certain embodiments, the BiTE further comprises a tumor-associated antigen binding domain.

In certain embodiments, the tumor associated antigen is selected from the group consisting of: GPC3, CD19, BCMA, GCC (GUCY2C), Her2, Claudin18.2, and Mesothelin; and the viral antigen is selected from EBV-gp350 and HBV s protein.

In certain embodiments, the nucleic acid molecule further comprises a 5' homologous arm, wherein the 5' homologous arm is homologous to a target region in the genome of an immune cell.

In certain embodiments, the 5' homologous arm is located upstream to the nucleic acid sequence encoding the chimeric antigen receptor (CAR).

In certain embodiments, the nucleic acid molecule further comprises a 3' homologous arm, wherein the 3' homologous arm is homologous to a target region in the genome of an immune cell.

In certain embodiments, the 3' homologous arm is located downstream to the nucleic acid sequence encoding IL-21 or a functionally active fragment thereof.

In certain embodiments, the target region is located at a target gene selected from the group consisting of: TRBC, TRAC, PD-1, CD52, CD95, AAVS1, and CCR5.

In certain embodiments, the target-binding domain of the CAR comprises an amino acid sequence shown in SEQ ID NO: 1.

In certain embodiments, the target-binding domain of the CAR comprises an amino acid sequence encoded by the nucleotide sequences shown in SEQ ID NOs: 31 and 32.

In certain embodiments, the CAR comprises a hinge region, a transmembrane domain, a co-stimulatory domain, and an intracellular domain.

In certain embodiments, the hinge region is derived from CD8α.

In certain embodiments, the transmembrane domain is derived from CD8α.

In certain embodiments, the co-stimulatory domain is derived from 41BB.

In certain embodiments, the co-stimulatory domain comprises an amino acid sequence shown in SEQ ID NO: 3.

In certain embodiments, the intracellular domain is derived from CD3ζ.

In certain embodiments, the intracellular domain comprises an amino acid sequence shown in SEQ ID NO: 4.

In certain embodiments, the CAR comprises an amino acid sequence shown in SEQ ID NO: 5.

In certain embodiments, the IL-15 or a functionally active fragment thereof comprises an amino acid sequence shown in any one of SEQ ID NOs: 6-7 and 9.

In certain embodiments, the IL-21 or a functionally active fragment thereof comprises an amino acid sequence shown in any one of SEQ ID NOs: 10-11 and 13.

In certain embodiments, the cleavable moiety comprises an amino acid sequence shown in any one of SEQ ID NOs: 14-16.

In certain embodiments, the 5' homologous arm comprises a nucleic acid sequence shown in SEQ ID NO: 17.

In certain embodiments, the 3' homologous arm comprises a nucleic acid sequence shown in SEQ ID NO: 18.

In certain embodiments, the nucleic acid molecule comprises a nucleic acid sequence with a length of at least 3 kb.

In certain embodiments, the nucleic acid molecule comprises a nucleic acid sequence shown in any one of SEQ ID NOs: 20-21, 23-24, and 27-30.

In certain embodiments, the nucleic acid molecule includes a circular nucleic acid molecule, a supercoiled nucleic acid molecule, and/or a linear nucleic acid molecule.

In certain embodiments, the nucleic acid molecule includes a DNA molecule and/or a RNA molecule.

In certain embodiments, the nucleic acid molecule includes a single-stranded nucleic acid molecule and/or a double-stranded nucleic acid molecule.

In a second aspect, the present application provides a vector comprising the nucleic acid molecule according to the present application.

In certain embodiments, the vector is a non-viral vector.

In certain embodiments, the vector is a plasmid.

In another preferred embodiment, the plasmid is derived from a microorganism, and the content of genomic DNA of the microorganism accounts for less than 10 wt%, preferably ≤5 wt%, and more preferably ≤1 wt% of the total DNA in the plasmid.

In a third aspect, the present application provides a method for preparing a modified immune cell, wherein the method comprises transfecting an immune cell to be modified with the nucleic acid molecule according to the present application, or the vector according to the present application.

In another preferred embodiment, the method is an electrotransfection method based on a donor plasmid in combination with a targeting nuclease, or a targeting electrotransfection method based on a donor plasmid.

In another preferred embodiment, the method comprises:
(a) providing a donor plasmid, wherein the donor plasmid comprises a nucleic acid molecule of claim 1, the plasmid is derived from a microorganism and the content of genomic DNA of the microorganism accounts for less than 10 wt% (preferably ≤5 wt%, and more preferably ≤1 wt%) of the total DNA in the plasmid; and
(b) transfecting an immune cell to be modified with the plasmid, thereby allowing the cell to comprise and/or express the nucleic acid molecule.

In another preferred embodiment, in step (b), the transfection comprises electrotransfection.

In another preferred embodiment, in step (b), the transfection is carried out in the presence of a gene editing system (or a gene editing reagent), thereby integrating the nucleic acid molecule into a specific location in the genome of the cell.

In another preferred embodiment, the gene editing system comprises a nuclease and a guide RNA.

In another preferred embodiment, the nuclease comprises a Cas protein, preferably Cas9.

In another preferred embodiment, the gene editing system comprises a ribonucleoprotein complex (RNP), and the RNP comprises the Cas protein and the guide RNA.

In certain embodiments, the method comprises: transfecting the immune cell to be modified with a transfection composition comprising the nucleic acid molecule according to the present application or the vector according to the present application, thereby allowing the cell to comprise and/or express the nucleic acid molecule, wherein at least a portion of the nucleic acid molecule or the vector is obtained from a host cell; and in the portion of the nucleic acid molecule or the portion of the vector obtained from the host cell, the content of genomic DNA from the host cell is about 10% (w/w) or less.

In the present application, one or more additional genes may also be knocked out or knocked down in the modified immune cell. For example, the additional genes may include a gene expressing an immune checkpoint (e.g., PD-1). For example, the additional genes may include a gene related to Fas-FasL-induced cell death (e.g., CD95). For example, the transfection composition may comprise one or more guide RNAs targeting the additional gene to be knocked out or knocked down (e.g., ). For example, the guide RNA may target a gene expressing an immune checkpoint (e.g., PD-1), and/or a gene related to Fas-FasL-induced cell death (e.g., CD95).

In the present application, the host cell is not or does not include a mammalian immune cell.

In certain embodiments of the method of the present application, the immune cell comprises an immune effector cell.

In certain embodiments of the method of the present application, the immune cell comprises T lymphocyte, B lymphocyte, NK cell, macrophage, dendritic cell, monocyte, granulocyte and/or mast cell.

In certain embodiments of the method of the present application, the immune cell comprises peripheral blood lymphocyte.

In certain embodiments of the method of the present application, the immune cell is a primary cell.

In certain embodiments of the method of the present application, the immune cell comprises autologous and/or allogeneic cell derived from a subject.

In certain embodiments of the method of the present application, the immune cell is an activated cell.

In certain embodiments of the method of the present application, the activating comprises contacting the immune cells to be modified with an activation composition.

In certain embodiments of the method of the present application, the activation composition comprises an anti-CD3 and/or anti-CD28 antibody.

In certain embodiments of the method of the present application, the activating comprises contacting the immune cells to be modified with the activating composition for no more than about 4 days.

In certain embodiments, the method comprises performing the transfection by contacting the immune cells to be modified with the activation composition for a time period of about 2 days or less.

In certain embodiments of the method of the present application, the transfection comprises electroporating the immune cells to be modified.

In certain embodiments of the method of the present application, the concentration of the nucleic acid molecule or the vector in the transfection composition is from about 5 µg/mL to about 3000 µg/mL.

In certain embodiments of the method of the present application, the concentration of the nucleic acid molecule or the vector in the transfection composition is from about 200 µg/mL to about 800 µg/mL.

In certain embodiments of the method of the present application, the nucleic acid molecule or the vector is extracted and obtained from the host cell.

In certain embodiments of the method of the present application, the host is a microbial host.

In certain embodiments of the method of the present application, the host is selected from one or more of the following group: bacteria, fungi, actinomycetes, mycoplasmas, chlamydias, rickettsias, and spirochetes.

In certain embodiments of the method of the present application, the host comprises a gram-negative bacterium.

In certain embodiments of the method of the present application, the host comprises *E. coli.*

In certain embodiments of the method of the present application, the size of the genomic DNA of the host cell is at least about 10 kb.

In certain embodiments of the method of the present application, the content of genomic DNA of the host cell is less than about 1% (w/w).

In certain embodiments of the method of the present application, the content of genomic DNA of the host cell is less than about 9‰ (w/w).

In certain embodiments of the methods of the present application, the content of the genomic DNA of the host cell is determined by qPCR method.

In certain embodiments of the method of the present application, the nucleic acid molecule portion or the vector portion obtained from the host cell is treated such that the content of genomic DNA of the host cell therein is reduced; and/or the content of genomic DNA of the host cell in the nucleic acid molecule portion or the vector portion obtained from the host cell is determined, and based on this content, it is judged whether the nucleic acid molecule portion or the vector portion shall be treated to reduce the content of genomic DNA of the host cell therein.

In certain embodiments of the method of the present application, the content of genomic DNA of the host cell in the nucleic acid molecule portion or the vector portion obtained from the host cell is determined, and when the content of genomic DNA of the host cell is about 10% (w/w) or more, the nucleic acid molecule portion or the vector portion is treated to reduce the content of genomic DNA of the host cell therein.

In certain embodiments of the method of the present application, the treatment comprises contacting the nucleic acid molecule portion or the vector portion with one or more reagents selected from the group consisting of: deoxyribonuclease (DNase), SDS, TX-100, CTAB, and cesium chloride-ethidium bromide.

In certain embodiments of the method of the present application, the DNase is capable of non-specifically cleaving linear DNA.

In certain embodiments of the method of the present application, the DNase is an exonuclease.

In certain embodiments of the method of the present application, the treatment comprises contacting the transfection composition with the reagent in the presence of Mg²⁺ and Ca²⁺.

In certain embodiments of the method of the present application, the transfection composition also comprises a gene editing system capable of integrating the nucleic acid molecule into a specific location in the cell genome.

In certain embodiments of the method of the present application, the gene editing system comprises a site-specific enzyme or a nucleic acid molecule encoding the enzyme (e.g., mRNA encoding the site-specific enzyme).

In certain embodiments of the method of the present application, the site-specific enzyme is selected from: transcription activator-like effector nucleases (TALEN), zinc finger nucleases (ZFN), transposases, integrases, and Cas proteins.

In certain embodiments of the method of the present application, the Cas protein is Cas9 protein.

In certain embodiments of the method of the present application, the transposase comprises PiggyBac (PB) transposase, and/or Sleeping Beauty (SB) transposase.

In certain embodiments, the gene editing further comprises knocking out one or more genes in the cell. The gene to be knocked out may include, for example, a gene expressing immune checkpoint (such as PD-1) and/or a gene related to Fas-FasL-induced cell death (e.g., CD95).

In certain embodiments of the method of the present application, the gene editing system further comprises one or more guide RNAs.

For example, the gene editing system may comprise one or more guide RNAs targeting the gene(s) to be knocked out. For example, the gene to be knocked out may be selected from the group consisting of: TRBC, TRAC, PD-1, CD95, AAVS1 and CCR5. For example, the gene editing system may comprise a guide RNA targeting the gene expressing an immune checkpoint (such as PD-1). For example, the gene editing system may comprise a guide RNA targeting the gene related to Fas-FasL-induced cell death (e.g., CD95).

In certain embodiments of the method of the present application, the guide RNA is complementary to the nucleic acid sequence in the target region of the cell genome.

In certain embodiments of the method of the present application, the gene editing system comprises a ribonucleoprotein complex (RNP), and the RNP comprises the Cas protein and the guide RNA.

In certain embodiments, the target region is located in a gene region selected from the group consisting of: TRBC, TRAC, PD-1, CD52, CD95, AAVS1, and CCR5.

In the fourth aspect, the present application provides a kit, which comprises the nucleic acid molecule of the present application or the vector of the present application.

In the fifth aspect, the present application provides a kit for transfection, which comprises: 1) the nucleic acid molecule of the present application or the vector of the present application obtained from a host cell; and 2) a reagent capable of reducing or degrading the genomic DNA of the host cell.

In certain embodiments of the reagent kit of the present application, the reagent of 2) comprises one or more of the following: deoxyribonuclease (DNase), SDS, TX-100, CTAB, and cesium chloride-ethidium bromide.

In certain embodiments of the reagent kit of the present application, the DNase is capable of non-specifically cleaving linear DNA.

In certain embodiments of the reagent kit of the present application, the DNase is an exonuclease.

In certain embodiments of the reagent kit of the present application, the reagent kit also comprises a reagent containing Mg²⁺ and Ca²⁺.

In a sixth aspect, the present application provides a transfection composition, which comprises the nucleic acid molecule of the present application or the vector of the present application, wherein at least a portion of the nucleic acid molecule or the vector is obtained from a host cell; and in the portion of the nucleic acid molecule or the portion of the vector obtained from the host cell, the content of genomic DNA from the host cell is about 10% (w/w) or less.

In certain embodiments of the transfection composition of the present application, the concentration of the nucleic acid molecule or the vector is about 5 µg/mL to about 3000 µg/mL.

In certain embodiments of the transfection composition of the present application, the concentration of the nucleic acid molecule or the vector is about 200 µg/mL to about 800 µg/mL.

In certain embodiments of the transfection composition of the present application, the host is a microbial host.

In certain embodiments of the transfection composition of the present application, the host is selected from one or more of the following group: bacteria, fungi, actinomycetes, mycoplasmas, chlamydias, rickettsias, and spirochetes.

In certain embodiments of the transfection composition of the present application, the host comprises gram-negative bacteria.

In certain embodiments of the transfection composition of the present application, the host comprises *E. coli.*

In certain embodiments of the transfection composition of the present application, the size of the genomic DNA of the host cell is at least about 10 kb.

In certain embodiments of the transfection composition of the present application, the content of the genomic DNA of the host cell is less than about 1% (w/w).

In certain embodiments of the transfection composition of the present application, the content of the genomic DNA of the host cell is less than about 9‰ (w/w).

In certain embodiments of the transfection composition of the present application, the content of the genomic DNA of the host cell is determined by qPCR method.

In certain embodiments of the transfection composition of the present application, the nucleic acid molecule portion or the vector portion obtained from the host cell is treated such that the content of genomic DNA of the host cell therein is reduced; and/or the content of genomic DNA of the host cell in the nucleic acid molecule portion or the vector portion obtained from the host cell is determined, and based on this content, it is judged whether the nucleic acid molecule portion or the vector portion shall be treated to reduce the content of genomic DNA of the host cell therein.

In certain embodiments of the transfection composition of the present application, the content of genomic DNA of the host cell in the nucleic acid molecule portion or the vector portion obtained from the host cell is determined, and when the content of genomic DNA of the host cell is about 10% (w/w) or more, the nucleic acid molecule portion or the vector portion is treated to reduce the content of genomic DNA of the host cell therein.

In certain embodiments of the transfection composition of the present application, the treatment comprises contacting the nucleic acid molecule portion or the vector portion with one or more reagents selected from the group consisting of: deoxyribonuclease (DNase), SDS, TX-100, CTAB, and cesium chloride-ethidium bromide.

In certain embodiments of the transfection composition of the present application, the DNase is capable of non-specifically cleaving linear DNA.

In certain embodiments of the transfection composition of the present application, the DNase is an exonuclease.

In certain embodiments of the transfection composition of the present application, the treatment comprises contacting the transfection composition with the reagent in the presence of Mg²⁺ and Ca²⁺.

In certain embodiments, the transfection composition also comprises a gene editing system capable of integrating the nucleic acid molecule into a specific location in the cell genome.

In certain embodiments of the transfection composition of the present application, the gene editing system comprises a site-specific enzyme or a nucleic acid molecule encoding the enzyme.

In certain embodiments of the transfection composition of the present application, the site-specific enzyme is selected from: transcription activator-like effector nucleases (TALEN), zinc finger nucleases (ZFN), transposases, integrases, and Cas proteins.

In certain embodiments of the transfection composition of the present application, the Cas protein is Cas9 protein.

In certain embodiments of the transfection composition of the present application, the transposase comprises PiggyBac (PB) transposase, and/or Sleeping Beauty (SB) transposase.

In certain embodiments of the transfection composition of the present application, the gene editing system further comprises one or more guide RNAs.

In certain embodiments of the transfection composition of the present application, the guide RNA is complementary to the nucleic acid sequence in the target region of the cell genome.

In certain embodiments of the transfection composition of the present application, the gene editing system comprises a ribonucleoprotein complex (RNP), and the RNP comprises the Cas protein and the guide RNA.

In certain embodiments of the transfection composition of the present application, the target region is located at a gene selected from the group consisting of: TRBC, TRAC, PD-1, CD52, CD95, AAVS1, and CCR5.

In the seventh aspect, the present application further provides an immune cell prepared by the method of the present application.

In the eighth aspect, the present application provides a modified immune cell comprising the nucleic acid molecule of the present application, or the vector of the present application.

In certain embodiments of the modified immune cell of the present application, the nucleic acid molecule is integrated into the genome of the immune cell.

In certain embodiments of the modified immune cell of the present application, the nucleic acid molecule is integrated at a target gene in the genome of the immune cell.

In certain embodiments of the modified immune cell of the present application, the nucleic acid molecule is expressed under the regulation of endogenous regulatory sequences of the target gene.

In certain embodiments of the modified immune cell of the present application, the target gene is selected from the group consisting of: TRBC, TRAC, PD-1, CD95, AAVS1, and CCR5.

In certain embodiments of the modified immune cell of the present application, one or more additional genes are knocked down or knocked out.

In certain embodiments, the additional gene is different from the target gene into which the nucleic acid molecule or a functionally active fragment thereof is integrated.

In certain embodiments, the one or more additional genes include an immune checkpoint gene.

In certain embodiments, the one or more additional genes include a gene related to Fas-FasL-induced cell death.

In certain embodiments, the one or more additional genes include PD-1 and/or CD95.

In certain embodiments, the immune cell comprises immune effector cell.

In certain embodiments, the immune cell comprises T lymphocytes, B lymphocyte, NK cell, macrophage, dendritic cell, monocyte, granulocyte and/or mast cell.

In certain embodiments, the immune cell comprises peripheral blood lymphocyte.

In certain embodiments, the immune cell is a primary cell.

In certain embodiments, the immune cell comprises autologous and/or allogeneic cell derived from a subject..

In certain embodiments, the immune cell is an activated cell.

In certain embodiments of the modified immune cell of the present application, the activating comprises contacting the immune cells to be modified with an activation composition.

In certain embodiments of the modified immune cell of the present application, the activation composition comprises an anti-CD3 and/or anti-CD28 antibody.

In certain embodiments of the modified immune cell of the present application, the activating comprises contacting the immune cells to be modified with the activating composition for no more than about 4 days.

In certain embodiments, the immune cell is an isolated cell.

In the ninth aspect, the present application provides a cell population comprising the cell and/or progeny thereof described in the present application.

In the tenth aspect, the present application provides a pharmaceutical composition comprising the nucleic acid molecule of the present application, the vector of the present application, the cell of the present application, and/or the cell population of the present application.

In certain embodiments, the pharmaceutical composition further comprises a pharmaceutically acceptable adjuvant.

In the eleventh aspect, the present application provides the use of the nucleic acid molecule of the present application, the vector of the present application, the cell of the present application, the cell population of the present application, and/or the pharmaceutical composition of the present application for the preparation of a medicament.

In certain embodiments, the medicament is used for the prevention, treatment, and/or alleviation of a cancer.

In certain embodiments, the cancer includes tumor-associated antigen-positive cancer cells.

In certain embodiments, the tumor-associated antigen is selected from: GPC3, CD19, BCMA, Claudin18.2, and Mesothelin.

In certain embodiments, the cancer is liver cancer, hepatocellular cancer, or multiple myeloma.

In the twelfth aspect, the present application provides a method for preventing, treating, and/or alleviating a disease or disorder in a subject, comprising administering an effective amount of the nucleic acid molecule of the present application, the vector of the present application, the cell of the present application, the cell population of the present application, and/or the pharmaceutical composition of the present application to the subject.

In certain embodiments, the disease or disorder is a cancer.

In certain embodiments, the cancer includes tumor-associated antigen-positive cancer cells.

In certain embodiments, the tumor-associated antigen is selected from: GPC3, CD19, BCMA, Claudin18.2, and Mesothelin.

In certain embodiments, the cancer is liver cancer, hepatocellular cancer, or multiple myeloma.

In the thirteenth aspect, the present application provides the nucleic acid molecule of the present application, the vector of the present application, the cell of the present application, the cell population of the present application, and/or the pharmaceutical composition of the present application, for use in preventing, treating, and/or alleviating a disease or condition in a subject.

Those skilled in the art can easily discern other aspects and advantages of the present application from the detailed description below. The detailed description only shows and describes exemplary embodiments of the present application. As those skilled in the art will appreciate, the disclosure of the present application enables those skilled in the art to modify the specific embodiments disclosed without departing from the spirit and scope of the inventions involved in the present application. Accordingly, the descriptions in the drawings and specifications of the present application are merely illustrative and not limiting.

### DESCRIPTION OF DRAWINGS

The features and advantages of the invention involved in the present application may be better understood by referring to the detailed description of the exemplary embodiments and the drawings. A brief description of the drawings is as follows:
Figures 1A-1D show the expansion performance and biomarker expression of the engineered immune cells of the present application.
Figures 2A-2C show the performance of the engineered immune cells of the present application after cryopreservation and recovery.
Figures 3A-3B show the activation and expansion performance of the engineered immune cells of the present application after cryopreservation and recovery.
Figure 4 shows the proliferation of the engineered immune cells of the present application.
Figures 5A-5B show the killing effect of the engineered immune cells of the present application on tumor cells.
Figures 6A-6F show the *in vivo* tumor-inhibiting effect of the engineered immune cells of the present application.
Figures 7A-7B show the expression of exogenous genes by the engineered immune cells of the present application.
Figures 8A-8B show the *in vivo* tumor-inhibiting effect of the engineered immune cells of the present application.
Figures 9A-9B show the expression of exogenous genes by the engineered immune cells of the present application and their killing effect on tumor cells.
Figures 10A-10E show the performance comparison of different engineered immune cells of the present application.
Figures 11A-11B show the *in vivo* tumor-inhibiting effect of the engineered immune cells of the present application.
Figures 12A-12C show the detection of cell viability, cell proliferation ability, and protein expression ability of cells transfected with plasmids containing different amounts of host genomic DNA.
Figures 13A-13B show the inhibition of RPMI-8226 multiple myeloma cells and HepG2 hepatocellular carcinoma cells by the engineered immune cells regulated by exogenous promoters, wherein BCMA-CAR-15 represents the BCMA CAR-2A-IL15 structure; BCMA-CAR-15-21 represents the BCMA CAR-2A-IL15-2A-IL21 structure; BCMA-CAR-PGK-15-21 represents the PGK-BCMA CAR-2A-IL15-2A-IL21 structure; and PGK-GPC3 CAR represents the PGK-GPC3 CAR-2A-IL15-2A-IL21 structure.

### EMBODIMENTS

Through extensive and intensive research, the inventor has developed a method for highly efficient electrotransfection of immune cells based on the combination of a plasmid (or a donor plasmid) and a targeting nuclease for the first time,. Specifically, the present invention has developed a plasmid containing a nucleic acid molecule having the following elements: a nucleic acid sequence encoding a chimeric antigen receptor (CAR); a nucleic acid sequence encoding IL-15 or a functionally active fragment thereof and a nucleic acid sequence encoding IL-21 or a functionally active fragment thereof, and a highly efficient targeting electrotransfection method for immune cells based on this plasmid in combination with a targeting nuclease. On this basis, the present invention has been completed.

The inventor unexpectedly discovered that immune cells represented by T cells are extremely sensitive to the conventional plasmid-based electrotransfection method, resulting in a decrease in viability after transfection, making it impossible to efficiently prepare immune cells with targeted integration. However, when the plasmid is treated such that the content of the microbiome DNA in the plasmid is significantly decreased (e.g., ≤ 5wt%, more preferably ≤ 1wt%, more preferably ≤ 0.2%, more preferably ≤ 0.1%), the donor plasmid carrying a CAR-encoding sequence can be efficiently transfected into immune cells represented by T cells, and the prepared CAR-immune cells have high viability, high expansion ability, and high CAR expression rate.

In addition, the inventor has also developed a nucleic acid molecule or plasmid carrying a nucleic acid sequence encoding CAR, a nucleic acid sequence encoding IL21 (or a fusion protein or active fragment thereof), and a nucleic acid sequence encoding IL15 (or a fusion protein or active fragment thereof). Through the method of the present invention, the plasmid can be efficiently transfected into immune cells represented by T cells. When the CAR-T cells of the present invention are co-incubated with target cells *in vitro,* they exhibit excellent expansion ability, high survival rate, and high CAR expression rate without the addition of IL-2. However, the IL-15 and IL-21 expressed by the CAR-T cells of the present invention cannot provide sufficient support for cell survival during incubation when there are no target cells and no IL-2 cytokine, or when there are no target cells but only IL-2, which greatly reduces the survival time of CAR-T cells under such circumstances, indicating that there is no or extremely low tumorigenic risk.

The following specific embodiments illustrate the implementation of the present invention, and those skilled in the art can easily understand other advantages and effects of the present invention from the content disclosed in the present specification.

### Definitions on Terms

In the present application, the term "transfection efficiency" generally refers to the relative amount of materials introduced into and/or expressed by the transfected cells. In the present application, the transfection may refer to the introduction of one or more materials (e.g., polynucleotides) into cells. The introduced material may be stably or transiently maintained in the transfected cells. In the present application, the transfection efficiency may be measured by the amount of introduced materials.

In the present application, the term "deoxyribonuclease (DNase)" generally refers to an enzyme capable of cleaving phosphodiester bonds on the DNA backbone. The DNase may be a type of nuclease. The DNase may digest double-stranded DNA into deoxynucleotides (e.g., under weakly alkaline conditions). For example, the DNase may be substantially inactive (e.g., unable to cleave) circular or supercoiled nucleic acid molecules or fragments thereof (e.g., closed-circular double-stranded DNA and/or supercoiled DNA). For example, the DNase can cleave linear double-stranded DNA. For example, the DNase may be a DNA exonuclease. For example, the DNase may be an exonuclease V. For example, the DNase may be an ATP-Dependent DNase, such as Plasmid-Safe^{™} ATP-Dependent DNase.

In the present application, the term "nucleic acid molecule or its fragments" generally refers to nucleotides (e.g., ribonucleotides, deoxyribonucleotides, and/or modified forms of the foregoing) or fragments thereof. The nucleic acid molecule or fragment thereof may comprise the form of a polymer of nucleotides or fragment thereof. In certain cases, the nucleic acid molecule or fragment thereof may be interchangeable with "polynucleotides." The nucleic acid molecule may comprise DNA, RNA, cDNA, sense and antisense strands of genomic DNA, and synthetic forms, mixtures, and/or polymers thereof. The nucleic acid molecule or fragment thereof may include any topological conformation, such as single-stranded, double-stranded, partially double-stranded, triple-stranded, hairpin structures, circular, and/or catenated conformations. Nucleotides in the nucleic acid molecule or fragment thereof may be natural or modified. Nucleotides in the nucleic acid molecule or fragment thereof may be linked together by naturally occurring and/or non-naturally occurring nucleotide bonds.

In the present application, the term "exogenous nucleic acid molecule" typically refers to a nucleic acid molecule that is not directly produced within an organism, tissue, or cell. For example, the nucleic acid molecule is introduced into an organism, tissue, or cell in certain form from the outside. The structure, composition, or function of the nucleic acid molecule may be the same or different from the corresponding nucleic acid molecule endogenously expressed. For example, in certain cases, the exogenous nucleic acid molecule may have the same nucleotide sequence as the corresponding nucleic acid molecule endogenously expressed. In certain cases, the exogenous nucleic acid molecule is different from any endogenously expressed nucleic acid molecules.

In the present application, the term "transfection composition" generally refers to a mixture required for transfection. In the present application, the transfection composition may comprise one or more materials to be introduced (e.g., polynucleotides or exogenous nucleic acid molecules). For example, the transfection composition may comprise an exogenous nucleic acid molecule encoding an exogenous gene to be transfected. The transfection composition may comprise a vector containing the nucleic acid molecule. For example, the transfection composition may also comprise impurities (in certain cases, the impurities may comprise nucleic acid molecule or fragment thereof). In the present application, the impurities may comprise nucleic acid molecule or fragment thereof derived from microorganisms. The impurities carried by the vector may include impurities carried by the vector (e.g., nucleic acid molecule or fragment thereof homologous or heterologous to the vector backbone).

In the present application, the terms "total nucleic acid molecule content" and "total DNA content" can be used interchangeably, and generally refer to the total mass of all substances containing nucleotides in the transfection composition. For example, the substances with nucleotides may comprise the nucleic acid molecule or fragment thereof and the materials.

In the present application, the term "gene editing efficiency" typically refers to the proportion of nucleic acid molecules with cuts at the target location produced by gene editing means among all nucleic acid molecules treated by the gene editing means. The gene editing efficiency may reflect the ability of the gene editing to affect on the target location.

In the present application, the term "DNA homologous recombination efficiency" typically refers to the proportion of individuals (e.g., the number of cells) undergoing DNA homologous recombination among the total individuals used for DNA homologous recombination. The DNA homologous recombination efficiency can be verified by means such as gene sequencing and detection of corresponding protein expression.

In the present application, the term "cell viability" typically refers to the ability of a cell to survive and/or perform biological functions (e.g., division, proliferation, secretion, killing, preservation, and/or recovery) under certain conditions. In certain cases, cell viability may be measured by the proportion of live cells among the total number of live and dead cells at a specific time and condition. In the present application, cell viability can also be measured by the proportion of cells with a certain biological function and/or activity among the total number of cells at a specific time and condition.

In the present application, the term "host" typically refers to an organism used to carry, amplify, or produce the exogenous nucleic acid molecule to be transfected, which may comprise host cells, such as microbial or mammalian cells. In the present application, the host cell is not an immune cell.

In the present application, the term "microorganism" generally refers to eukaryotic and prokaryotic microbial species from the *Archaea, Bacteria,* and/or *Eucarya* domains. For example, the microorganism may comprise bacteria, viruses, fungi, actinomycetes, rickettsiae, mycoplasmas, chlamydiae, and/or spirochetes. In the present application, the term bacteria generally refers to any type of prokaryote, including prokaryotes from all phyla within the prokaryote domain. The bacteria may comprise cocci, bacilli, spirilla, protoplasts, and mycoplasmas. The bacteria may comprise Gram-positive and Gram-negative bacteria. "Gram-negative" and 'Gram-positive' refer to staining styles using Gram staining methods, which are well known in the art.

In the present application, the term "host cell genome DNA" typically refers to the DNA molecule or fragment thereof from host cell genome.

In the present application, the term "nucleic acid molecules or fragments thereof derived from the genome of a host (e.g., microorganism)" and "host genomic DNA" may be used interchangeably, generally referring to nucleic acid molecules or fragments thereof, which are derived from the genome of the host (e.g., microorganism). Information on the genomes of hosts (e.g., microorganisms) may be found in A genomic catalog of Earth's microbiomes, Nature Biotechnology (2020).

In the present application, the term "Gram-negative bacteria" generally refers to bacteria that do not retain the primary dye used in Gram staining but are stained by the counterstain. Therefore, Gram-negative bacteria usually appear red in the Gram staining method. The cell wall of the Gram-negative bacteria has a lower content of peptidoglycan and a higher content of lipids. For example, the cell wall of the Gram-negative bacteria may have a lipopolysaccharide layer. For example, the Gram-negative bacteria may comprise Escherichia coli, Pseudomonas aeruginosa, Proteus species, Shigella species, Klebsiella pneumoniae, Brucella species, Haemophilus influenzae, Haemophilus parainfluenzae, Moraxella catarrhalis, Acinetobacter species, Yersinia species, Legionella pneumophila, Bordetella pertussis, Bordetella parapertussis, Shigella species, Pasteurella species, Vibrio cholerae, Bacillus parahaemolyticus, and/or Plesimonas shigelloides.

In the present application, the term "Escherichia coli" generally refers to Escherichia coli. Escherichia coli belongs to the Enterobacteriaceae family, Escherichia genus.

In the present application, the term "transient transfection" generally refers to a transfection method in which the exogenous gene transfected into the cell is not integrated into the genome of the cell. The operation steps of the transient transfection can be known to those skilled in the art. For example, the transient transfection can be achieved via liposome mediated transfection. For example, the transient transfection may comprise electroporation transfection. The transient transfection can use transfection reagents, such as FuGENE6.

In the present application, the term "stable transfection" generally refers to that exogenous nucleic acid molecules are introduced and integrated into the genome of the transfected cells. For example, the exogenous gene is integrated into the genome of the transfected cells.

In the present application, the term "stem cell" generally refers to a class of undifferentiated cells that have the ability to self-renew and retain varying degrees of potential to form differentiated cells and tissues. The stem cell may be impotent, multipotent, or totipotent. Impotent stem cells are derived stem cells that have lost their differentiation ability. Totipotent stem cells can form all cells and tissues found in a complete organism. For example, totipotent stem cells can form a complete organism.

In the present application, the term "multipotent stem cell" and "pluripotent stem cell" are used interchangeably, and generally refer to stem cells that have the ability to form cells and tissues that are ultimately found in a complete organism but cannot form a complete organism.

In the present application, the term "mesenchymal stem cell" generally refers to cells that can produce mesenchymal lineages. The mesenchymal stem cells can be considered as part of multipotent stem cells. The mesenchymal stem cells can produce one or more mesenchymal lineage cells. The mesenchymal lineage cells can be derived from various tissues, for example, bone marrow tissue, adipose tissue, muscle tissue, reproductive tissue (e.g., amniotic, amniotic fluid, or umbilical cord tissue), skin tissue, bone tissue, and/or dental tissue.

In the present application, the term "immune cell" generally refers to cells that function in the immune response. The immune cells may comprise lymphocytes, monocytes, and/or granulocytes, as well as their precursors and/or mature derivatives. The immune cells may comprise T cells, B cells, Th cells, natural killer cells, monocytes, macrophages, eosinophils, basophils, mast cells, dendritic cells, and/or granulocytes. The immune cell may comprise immune effector cells. The immune effector cells may participate in immune responses, such as promoting responses of immune effectors. The immune effector cells may comprise T cells, for example, α/β T cells and γ/δ T cells, B cells, natural killer (NK) cells, natural killer T (NKT) cells, mast cells, and bone marrow-derived phagocytes.

In the present application, the term "fibrocyte" generally refers to functionally inactive fibroblasts. The fibrocytes may transform into fibroblasts (for example, they may participate in the repair process when tissue is damaged). In the present application, the term "muscle cell" generally refers to one cell or a group of cells derived from muscle. The muscle cells may be derived from cells and tissues of skeletal muscle, smooth muscle (for example, from the digestive tract, bladder, and blood vessels), and cardiac muscle.

In the present application, the term "plasmid" generally refers to a construct containing genetic material. The plasmid may be designed to deliver genetic material (e.g., one or more nucleic acid sequences) into cells. The plasmid may contain autonomously replicating sequences of single-stranded or double-stranded nucleic acids (e.g., DNA or RNA) from any source. The term "plasmid" may be used interchangeably with the term "vector" in the present application. The plasmid may have different conformations, such as linear plasmids, circular plasmids, or supercoiled plasmids. The linear plasmid may be a linear DNA molecule. The supercoiled plasmid may comprise two nucleic acid strands that maintain intact structures (e.g., covalently closed circular DNA, cccDNA), and exhibit a supercoiled conformation. The circular plasmid may have at least one nucleic acid strand maintaining a complete circular structure. The plasmid may be not integrated into the cell genome.

In the present application, the term "multiple cloning site" or "MCS" generally refers to a nucleic acid sequence containing at least one restriction site. The multiple cloning site may link the nucleic acid molecule to the vector of the present application, for example, through the restriction site to achieve insertion of the nucleic acid molecule at a specified position. The restriction site may be a restriction endonuclease recognition site. For example, the restriction endonuclease may be AclUHindIII, Sspl, MLuCI, Tsp509I, Pcil, AgeKBspMI, BfuAI, SexAI, MLuI, BceAI, HpyCH4IV, HpyCH4III, Bael, BsaXI, SpeI, Bsrl, Bmrl, BglII, AfeI, Alul, StuI, Seal, Clal, BspDI, PI-SceI, NsiI, Asel, Swal, CspCI, MfeI, BssSI, BmgBI, PmLl, Dralll, Alel, EcoP15I, PvuII, AlwNI or BtsMutI.

In the present application, the term "exogenous promoter" generally refers to a promoter not originating from the host in which it is placed. The exogenous promoter may be transfected and/or inserted into the host cell (e.g., the cell's genome) in which it is placed. The promoter may be a recognition site for RNA polymerase binding to a polynucleotide (DNA or RNA). The RNA polymerase may effectively catalyze the assembly of messenger RNA complementary to the appropriate DNA strand of the coding region. The number of promoters may be one or more.

The exogenous promoter may be constitutive or inducible. Representative examples include PGK promoter, EF1 promoter, etc. The sequence of PGK promoter may be as shown in SEQ ID NO: 26.

In the present application, the term "gene editing" generally refers to the manipulation of inserting, deleting, and/or replacing nucleic acids into the genome. The gene editing may be achieved through homology-directed repair (HDR), non-homologous end joining (NHEJ), or single-base changes. The gene editing can use gene editing tools familiar to those skilled in the art, such as the zinc finger nuclease system (ZFN), the TALEN system, and/or CRISPR technique.

In the present application, the term "gene editing knock-in" generally refers to a genetic engineering process (e.g., also called knock-in), which involves one-to-one replacement of DNA sequence information at a genetic locus or insertion of sequence information not found within the endogenous locus. The gene editing knock-in can utilize homologous recombination. For example, in certain cases, an exogenous functional gene (a gene not originally present in the genome or an inactivated gene) can be transferred into a cell by using homologous recombination and undergoes homologous recombination with homologous sequences in the genome, allowing it to be inserted into the genome and expressed in the cell. For example, the gene editing knock-in at least partially replace the cellular genome with an exogenous gene. The gene editing knock-in may use CRISPR technology to achieve "targeted knock-in." Additionally, the gene editing knock-in can use transposon and transposase systems, such as PiggyBac (PB) transposase and/or Sleeping Beauty (SB) transposase.

In the present application, the term "donor plasmid" generally refers to a plasmid that can transcribe and/or translate the encoded exogenous gene in the introduced cell. For example, the donor plasmid may be suitable for gene editing knock-in. For example, the donor plasmid may comprise a nucleic acid molecule encoding the exogenous gene. The donor plasmid may also comprise elements such as promoters to regulate the expression of the exogenous gene (for example, occurring at the transcriptional and/or translational levels of production and/or accumulation). The donor plasmid may be suitable for eukaryotic expression systems. Preferably, the donor plasmid of the present invention comprises the nucleic acid molecule of the first aspect of the present invention.

In the present application, the term "exogenous gene to be knocked-in" generally refers to a heterologous gene that may be introduced by gene editing knock-in. The exogenous gene to be knocked-in may be integrated into the introduced object (e.g., cells, such as within a subject). The exogenous gene to be knocked-in may comprise genes integrated into the genome of the introduced object. The exogenous gene to be knocked-in may be a natural gene from different species or an engineered gene (for example, a chimeric gene). In the present invention, representative exogenous genes to be knocked in include (but are not limited to): nucleic acid sequences encoding a chimeric antigen receptor (CAR), nucleic acid sequences encoding IL-15 or a functionally active fragment thereof, nucleic acid sequences encoding IL-21 or a functionally active fragment thereof, and nucleic acid sequences encoding additional exogenous proteins (e.g., cytokines, BITEs, antibodies), etc.

In the present application, the term "antibody" generally refers to an immunoglobulin that is reactive to a specific protein, peptide, or fragments thereof. Antibodies may be from any class, including but not limited to IgG, IgA, IgM, IgD, and IgE, and antibodies from any subclass (e.g., IgG1, IgG2, IgG3, and IgG4). Antibodies may have heavy chain constant regions selected from, for example, IgG1, IgG2, IgG3, or IgG4. Antibodies may also have light chains selected from, for example, kappa (κ) or lambda (λ). The antibodies of the present application may be derived from any species.

In the present application, the term "antigen-binding fragment" generally refers to a portion of an antibody molecule that comprises amino acid residues that interact with the antigen and confer specificity and affinity of the antibody to the antigen. Examples of antigen-binding fragments may comprise, but are not limited to, Fab, Fab', F(ab)2, Fv, F(ab')2, scFv, di-scFv and/or dAb. In the present application, the term "Fab" generally refers to a fragment containing the heavy chain variable domain and the light chain variable domain, as well as the light chain constant domain and the first constant domain of the heavy chain (CH1); the term "Fab'" generally refers to a fragment that differs from Fab by the addition of a small number of residues (including one or more cysteines from the antibody hinge region) at the carboxy terminus of the heavy chain CH1 domain; the term "F(ab')2" generally refers to a dimer of Fab', an antibody fragment containing two Fab fragments connected via disulfide bridges in the hinge region. The term "Fv" generally refers to the minimal antibody fragment containing the complete antigen recognition and binding site. In certain cases, this fragment may consist of a tight non-covalent dimer composed of a heavy chain variable region and a light chain variable region; the term "dsFv" generally refers to a disulfide-stabilized Fv fragment, in which the bond between a single light chain variable region and a single heavy chain variable region is a disulfide bond. The term "dAb fragment" generally refers to an antibody fragment composed of a VH domain. In the present application, the term "scFv" generally refers to a monovalent molecule in which one heavy chain variable domain and one light chain variable domain of an antibody are covalently linked together via a flexible peptide linker; such scFv molecules may have the general structure: NH₂-VL-linker-VH-COOH or NH₂-VH-linker-VL-COOH.

In the present application, the term "bispecific antibody" generally refers to an antibody having a variable region capable of recognizing more than one epitope on one or more antigens. Bispecific antibodies comprise, but are not limited to, full-length antibodies, antibodies with two or more VL and VH structural domains, antibody fragments, such as Fab, Fv, dsFv, scFv, bispecific antibodies, and covalently or non-covalently linked antibody fragments. In certain cases, the bispecific antibody may recognize two different epitopes on the same or different antigens. In certain cases, the bispecific antibody may recognize two different antigens. In the present application, multispecific antibodies comprise bispecific or trispecific antibodies, or their antigen-binding fragments.

In the present application, the term "antigen-binding domain" and "target-binding domain" may be used interchangeably and generally refer to a structural domain capable of binding to a target antigen. Antigen-binding domains may comprise specific antigen-binding antibodies and antigen receptors or fragments thereof, antibodies or their antigen-binding fragments. Antigen-binding domains may be domains capable of binding to tumor-associated antigens. In the present application, the tumor-associated antigen may comprise but is not limited to: CD19, CD20, CD22, CD123, CD33/IL3Ra, CD138, CD33, BCMA, CS1, C-Met, EGFRvIII, CEA, Her2, GD2, MAG3, GPC3, Claudin18.2, Mesothelin and NY-ESO-1.

In the present application, the term "chimeric antigen receptor" (CAR) generally refers to a fusion protein that comprises an extracellular domain capable of binding to an antigen and at least one intracellular domain. CAR is the core component of chimeric antigen receptor T cells (CAR-T), which may comprise an antigen-binding domain (e.g., tumor-specific antigen and/or tumor-associated antigen), a transmembrane domain, a co-stimulatory domain, and an intracellular signaling domain. In the present application, the CAR may be based on the antigen-specificity (e.g., CD19) of an antibody and the intracellular domain of a T cell receptor activation. Genetically modified T cells expressing CAR can specifically recognize and eliminate malignant cells expressing the target antigen. Descriptions of CAR and CAR-T cells can see, for example, Sadelain M, Brentjens R, Rivi'ere I. The basic principles of chimeric antigen receptor design. Cancer Discov.2013;3(4):388-398; Turtle CJ, Hudecek M, Jensen MC, Riddell SR.Engineered T cells for anti-cancer therapy. Curr Opin Immunol.2012;24(5):633-639; Dotti G,Gottschalk S, Savoldo B, Brenner MK. Design and development of therapies using chimeric antigen receptor-expressing T cells. Immunol Rev.2014;257(1):107-126. In the present invention, tumor-associated antigens (TAAs) include TAAs for hematological tumors and for solid tumors, with representative TAAs including, but not limited to, GPC3, CD19, BCMA, GCC (GUCY2C), Her2, Claudin 18.2, and Mesothelin.

In the present application, the term "BiTE" generally refers to bispecific T cell engagers. The BiTE may be a single polypeptide chain molecule having two antigen-binding domains, one of which binds to a T cell antigen and the second to an antigen present on the surface of a target cell (see WO 2005/061547; Baeuerle, P et al. (2008) "BiTE®: A New Class Of Antibodies That Recruit T Cells Drugs of the Future 33:137-147; or Bargou et al. (2008) "Tumor Regression in Cancer Patients by Very Low Doses of a T Cell-Engaging Antibody," Science 321:974-977).

In the present application, the term "polyprotein" generally refers to a polypeptide chain that comprises multiple protein molecules, which may exist in tandem in the polypeptide chain. In the polypeptide chain, any two protein molecules may be optionally separated by a cleavable moiety (e.g., 2A peptide).

In the present application, the term "homology arm" generally refers to a polynucleotide suitable for targeting an exogenous gene to be knocked into a donor plasmid to the genome by homologous recombination. Homologous recombination may refer to recombination occurring between sister chromatids or between DNA molecules on the same chromosome containing homologous sequences or within molecules. The homology arm may have two (e.g., it may have a 5' homology arm and/or a 3' homology arm). The homology arms may be located upstream and downstream of the exogenous gene to be knocked into the donor plasmid. In certain cases, the targeting site in the genome for the exogenous gene to be knocked in may be broken due to the effect of nucleases. The homology arms may be identical or have at least 80% identity to the DNA sequences at both ends (i.e., 5' and/or 3' ends) of the break at the target site. For example, the homology arms may have at least about 80%, at least about 85%, at least about 90%, at least about 91%, at least about 92%, at least about 93%, at least about 94%, at least about 95%, at least about 96%, at least about 97%, at least about 98%, at least about 99%, or 100% identity to the DNA sequences at both ends of the break at the target site. In the present application, the donor plasmid may comprise a 5' homology arm and/or a 3' homology arm.

In the present application, the term "cell survival rate" generally refers to the ability of cells to maintain their survival under certain conditions. The cell survival rate may be measured by the proportion of living cells to the total number of cells present at that time under those conditions within a certain period of time. The cell can reflect the effects of certain conditions on cells. For example, the higher the cell survival rate, the more favorable the condition for cell survival.

In the present application, the term "cell proliferation capacity" generally refers to the ability of cells to proliferate and/or self-replicate. The cell proliferation capacity may be measured by the total number of cells produced by cell proliferation within a certain period of time. For example, the more cells increase in number within a certain period of time, the higher the cell proliferation capacity. The cell proliferation capacity may also be reflected in the improvement of cell functions (e.g., proliferation).

In the present application, the term "cell killing capacity" generally refers to the killing capacity of cells against their target cells. For example, immune effector cells (e.g., T cells, NK cells) may mediate the killing of target cells (e.g., tumor cells) and kill target cells. For example, the mediation may comprise the localization of immune effector cells to target cells (e.g., through the mutual recognition of molecules and/or epitopes expressed by immune effector cells and target cells). The cell killing capacity may be measured by the number of target cells killed by cell-mediated killing within a certain period of time and under certain conditions.

In the present application, the term "cell secretion capacity" generally refers to the ability of cells to secrete and produce secretory factors. For example, the secretory factors may be molecules that leave the cell due to secretion. The secretory factors may comprise proteins encoded by exogenous genes. The cell secretion capacity may be measured by the quantity of secretory factors produced by cell secretion within a certain period of time and under certain conditions.

In the present application, the term "cell preservation capability" generally refers to the ability of cells to survive and/or retain their original function under preservation conditions. For example, the preservation conditions may comprise long-term storage at ambient temperature or low temperature (e.g., non-refrigerated temperature or refrigerated temperature, such as under liquid nitrogen conditions). Preservation may reduce the metabolic level of the cells and may temporarily suspend their growth state. The cells may be revived after preservation. The cell preservation capability may be measured by the ratio of the cell number after preservation for a certain time and under certain conditions to the cell number before preservation.

In the present application, the term "cell revival capability" generally refers to the ability of cells to recover growth after re-cultivation. The re-cultivation may refer to re-culturing cells after recovery them from liquid nitrogen or a -80°C freezer. The cell revival capability may be measured by the ratio of the number of cells that recover their growth ability after re-cultivation to the total number of cells subjected to re-cultivation under certain time and conditions.

In the present application, the term "cell line" generally refers to a clonal population of cells capable of continuous division. For example, the cell line may have acquired the ability to proliferate indefinitely *in vitro.*

In the present application, the term "complementary" generally refers to Watson-Crick base pairing between nucleotides, and specifically refers to nucleotides that hydrogen bond with each other, in which thymine or uracil residues are connected to adenine residues by two hydrogen bonds, and cytosine and guanine residues are connected by three hydrogen bonds. Typically, a nucleic acid includes a nucleotide sequence described as having "percent complementarity" to a specified second nucleotide sequence. Those skilled in the art will recognize that two complementary nucleotide sequences comprise a sense strand and an antisense strand.

In the present application, the terms "homology", "identity", or "similarity" generally refer to sequence similarity between two peptides or between two nucleic acid molecules. The term "homologous region" generally refers to a region on a donor molecule that has a certain degree of homology with the target sequence. Homology may be determined by comparing the positions in each sequence. For example, homology between sequences may be determined by performing sequence alignment. When the positions in the compared sequences are occupied by the same base or amino acid, the molecules are homologous at that position. The degree of homology between sequences is a function of the number of matching or homologous positions shared by the sequences. "Unrelated" or "non-homologous" sequences have less than 40% identity with one of the sequences of the present application, although preferably less than 25% identity. A polynucleotide or polynucleotide region (or polypeptide or polypeptide region) has a certain percentage (e.g., 60%, 65%, 70%, 75%, 80%, 85%, 90%, 95%, 98%, or 99%) of "sequence identity" or "homology" with another sequence, meaning that in alignment, the percentage of bases (or amino acids) in the two sequences being compared are the same. This alignment and percentage homology or sequence identity may be determined using software programs known in the art, such as those described in Ausubel et al. (2007), Current Protocols in Molecular Biology.

In the present application, the term "transfection" generally refers to methods for introducing biologically active substances (e.g., nucleic acids, proteins, enzymes, or small molecules) into cells. Nucleic acids may be DNA (as plasmid or oligomer delivery) and/or RNA or combinations thereof.

In the present application, the term "electroporation" generally refers to a transfection method that involves applying an external electric field to cells. In certain embodiments, the electroporation method used is electrostatic poration.

In the present application, the terms "IL15" and "IL-15" are used interchangeably and generally refer to the cytokine interleukin-15 and/or a functionally active fragment thereof. IL-15 binds to IL-15 receptors on a variety of different cells of the immune system, thereby mediating relevant signaling. The amino acid sequence of human IL-15 can be found in UniprotKB: P40933. As used herein, the term "IL-15" includes human IL-15 (hIL-15), variants, isoforms and species homologs of hIL-15, and analogs sharing at least one epitope with hIL-15 (e.g., IL-15-containing fusion proteins, such as fusion proteins of IL-15 and Fc).

In the present application, the terms "IL21" and "IL-21" are used interchangeably and generally refer to the cytokine interleukin-21 and/or a functionally active fragment thereof. IL-21 binds to IL-21 receptors on a variety of different cells of the immune system, thereby mediating relevant signaling. The amino acid sequence of human IL-21 can be found in UniProtKB: Q9HBE4. As used herein, the term "IL-21" includes human IL-21 (hIL-21), variants, isoforms and species homologs of hIL-21, and analogs sharing at least one epitope with hIL-21 (e.g., IL-21-containing fusion proteins, such as fusion proteins of IL-21 and Fc).

In the present application, the term "functionally active fragment" generally refers to a fragment that has a partial region of a full-length protein or nucleic acid, but retains or partially retains the biological activity or function of the full-length protein or nucleic acid. For example, the functionally active fragment may retain or partially retain the ability of the full-length protein to bind another molecule. For example, a functionally active fragment of IL-15 may retain or partially retain the receptor binding function or one or more other biological activities of full-length IL-15. For example, a functionally active fragment of IL-21 may retain or partially retain the receptor binding function or one or more other biological activities of full-length IL-21.

Unless otherwise specified in the context, in the present application, the terms "comprise," "have," and "comprise" may be used interchangeably, generally referring to the possibility of comprising other components, elements, values, steps, etc. In certain cases, "comprise" covers the cases of "for" and "composed of," for example, based on a record of a composition "comprising" component "A," those skilled in the art can infer that, in certain cases, the composition may be composed solely of component A.

In the present application, the term "about" generally refers to within 30%, 25%, 20%, 15%, 10%, 9%, 8%, 7%, 6%, 5%, 4%, 3%, 2%, 1%, 0.5%, or 0.05 % of the specified value or numerical range involved in the present application. In the present application, when the term "about" precedes the first value in a series of two or more values, it applies to each value in the series.

### Detailed Description of the Invention

### Isolated nucleic acid molecule and vector

In the first aspect, the present application provides an isolated nucleic acid molecule comprising: a nucleic acid sequence encoding a chimeric antigen receptor (CAR); a nucleic acid sequence encoding IL-15 or a functionally active fragment thereof; and a nucleic acid sequence encoding IL-21 or a functionally active fragment thereof.

In certain embodiments, the nucleic acid molecule comprises, in order from 5' to 3': the nucleic acid sequence encoding the CAR, the nucleic acid sequence encoding IL-15 or a functionally active fragment thereof, and the nucleic acid sequence encoding IL-21 or a functionally active fragment thereof. For example, a nucleic acid sequence encoding IL-15 or a functionally active fragment thereof may be comprised on the 3' side (or downstream) of the nucleic acid sequence encoding CAR, and one or more segments of a first spacer nucleic acid sequence may be comprised between the two. For example, the 3' end of the nucleic acid sequence encoding CAR may be joined to the 5' end of the first spacer nucleic acid sequence, and the 3' end of the first spacer nucleic acid sequence may be joined to the 5' end of the nucleic acid sequence encoding IL-15 or a functionally active fragment thereof. For example, a nucleic acid sequence encoding IL-21 or a functionally active fragment thereof may be comprised on the 3' side (or downstream) of the nucleic acid sequence encoding IL-15 or a functionally active fragment thereof, with one or more segments of a second spacer nucleic acid sequence included between the two. For example, the 3' end of the nucleic acid sequence encoding IL-21 or a functionally active fragment thereof may be joined to the 5' end of the second spacer nucleic acid sequence, and the 3' end of the second spacer nucleic acid sequence may be joined to the 5' end of the nucleic acid sequence encoding IL-21 or a functionally active fragment thereof. The first spacer nucleic acid sequence and the second spacer nucleic acid sequence may be identical or different. In certain embodiments, the first spacer nucleic acid sequence and/or the second spacer nucleic acid sequence may comprise a nucleic acid sequence encoding one or more additional proteins.

In the present application, the chimeric antigen receptor CAR may comprise a target-binding domain that targets a tumor-associated antigen. For example, the chimeric antigen receptor may comprise at least one target-binding domain targeting an antigen. For example, the chimeric antigen receptor may specifically bind to one antigen. For example, the chimeric antigen receptor may specifically bind to two antigens, and/or, may specifically bind to at least two different epitopes of one antigen. In the present application, the antigen may be a tumor-associated antigen (TAA). The tumor-associated antigen may be a related antigen of the following tumor cells: for example, breast cancer cells, B-cell lymphoma, Hodgkin lymphoma cells, ovarian cancer cells, prostate cancer cells, mesothelioma, lung cancer cells (e.g., small cell lung cancer cells), non-Hodgkin B-cell lymphoma (B-NHL) cells, ovarian cancer cells, prostate cancer cells, mesothelioma cells, lung cancer cells (e.g., small cell lung cancer cells), melanoma cells, chronic lymphocytic leukemia cells, glioma, glioblastoma, neurotube cell tumor, colorectal cancer cells, etc. Cancer cell-associated antigens can also be expressed by non-cancer cells.

In the present application, the tumor-associated antigens may be selected from: GPC3, CD19, BCMA, Claudin18.2, and Mesothelin. For example, the antigen may be CD19. For example, the antigen may be GPC3, or BCMA.

In certain cases, the target-binding domain may be a single-chain antibody (scFv), or a cAb VHH (camelid antibody variable domain) and its humanized variants, IgNAR VH (shark antibody variable domain) and its humanized variants, sdAb VH (single-domain antibody variable domain) and "camelid-derived" antibody variable domains. The target-binding domain may also be a recognition domain based on T-cell receptor (TCR), such as single-chain TCR (scTv, a single-chain two-domain TCR containing VαVβ).

In certain cases, the target-binding domain of the CAR may comprise an amino acid sequence shown in SEQ ID NO: 1.

In certain cases, the target-binding domain of the CAR may comprise an amino acid sequence encoded by the nucleotide sequences shown in SEQ ID NOs: 31 or 32.

In the present application, the chimeric antigen receptor may comprise a transmembrane domain. The N-terminus of the transmembrane domain may be directly or indirectly connected to the C-terminus of the target-binding domain. In the present application, any transmembrane (TM) structure provided for inserting a peptide into the cell membrane of a eukaryotic cell (e.g., mammalian cell) may be applicable to the transmembrane domain.

For example, the transmembrane domain may comprise a transmembrane domain derived from a protein selected from the following: T-cell receptor α, β, or ζ chain, CD28, CD3e, CD45, CD4, CD5, CD8a, CD9, CD16, CD22, CD33, CD37, CD64, CD80, CD86, CD134, CD137, and CD154.

In certain embodiments, the chimeric antigen receptor may comprise a hinge region. The hinge region may be located between the target-binding domain and the transmembrane domain. The hinge region may be an immunoglobulin heavy chain hinge region, or the hinge region may be a hinge region polypeptide from a receptor (e.g., from the hinge region of CD8).

The length of the hinge region may be about 4 to about 50 amino acids. For example, it may be about 4 to about 10 amino acids, about 10 to about 15 amino acids, about 15 to about 20 amino acids, about 20 to about 25 amino acids, about 25 to about 30 amino acids, about 30 to about 40 amino acids, or about 40 to about 50 amino acids.

The hinge region may comprise at least one cysteine. Natl. Acad. The amino acid sequence of the hinge region may be known in the art, see for example Tan et al. (1990) Proc. Sci. USA 87:162; and Huck et al. (1986) Nucl. Acids Res.14:1779.

In the present application, the hinge region may comprise the amino acid sequence of a human IgG1, IgG2, IgG3, or IgG4 hinge region. For example, compared to the wild-type (naturally occurring) hinge region, the hinge region may comprise one or more amino acid substitutions and/or insertions and/or deletions.

In the present application, the chimeric antigen receptor may comprise co-stimulatory structural domains. The length of the co-stimulatory structural domain may be about 30 to about 70 amino acids. The co-stimulatory structural domain may be derived from a peptide of the receptor. For example, the co-stimulatory structural domain may be the intracellular part of a transmembrane protein. For example, the co-stimulatory structural domain may comprise co-stimulatory structural domains derived from the following proteins: 4-1BB (CD137), CD28, ICOS, OX-40, BTLA, CD27, CD30, GITR, and/or HVEM. For example, the co-stimulatory structural domain may comprise co-stimulatory structural domains derived from the following proteins: CD28, 4-1BB, OX-40, and/or ICOS.

In the present application, the chimeric antigen receptor may also comprise a linker. The linker may be located between the transmembrane structural domain and the co-stimulatory structural domain. The linker may be a linker peptide. For example, the linker peptide may have a length of about 6 to about 40 amino acids. The linker peptide may have any amino acid sequence as long as it can have a flexible structure.

In the present application, the chimeric antigen receptor may comprise an intracellular signal transduction structural domain. For example, the intracellular signal transduction structural domain may comprise intracellular signal transduction structural domains derived from the following proteins: CD8β, CD4, CD3ζ, CD28, CD134, and/or CD7. For example, the intracellular signal transduction structural domain may comprise signal transduction structural domains derived from CD3ζ.

In the present application, the intracellular signal transduction structural domain may comprise a part having an ITAM motif from a polypeptide containing an ITAM motif. For example, the intracellular signal transduction structural domain may comprise DAP12; FCER1G (Fcε receptor Iγ chain); CD3D (CD3δ); CD3E (CD3ε); CD3G (CD3γ); CD3Z (CD3ζ); and CD79A (antigen receptor complex-associated protein α chain).

In certain embodiments, the CAR may comprise a hinge region, a transmembrane domain, a co-stimulatory domain, and an intracellular domain. For example, the hinge region may be derived from CD8α. In certain cases, the hinge region may comprise a hinge region sequence in the amino acid sequence shown in SEQ ID NO: 2. For example, the transmembrane domain may be a transmembrane domain derived from CD8α. In certain cases, the transmembrane domain may comprise a transmembrane domain portion of the amino acid sequence shown in SEQ ID NO: 2. In certain embodiments, the hinge region and the transmembrane domain may comprise the amino acid sequence shown in SEQ ID NO: 2. For example, the co-stimulatory domain may be a co-stimulatory domain derived from 41BB. In certain cases, the co-stimulatory domain may comprise the amino acid sequence shown in SEQ ID NO: 3. For example, the intracellular domain may be an intracellular domain derived from CD3ζ. In certain cases, the intracellular domain may comprise the amino acid sequence shown in SEQ ID NO: 4. In certain embodiments, the CAR comprises an amino acid sequence shown in SEQ ID NO: 5.

In the present application, the IL-15 or a functionally active fragment thereof may comprise the endogenous signal peptide thereof or a signal peptide derived from another protein. For example, the IL-15 or a functionally active fragment thereof may comprise a signal peptide derived from IL-7. In the present application, the IL-15 or a functionally active fragment thereof may comprise the amino acid sequence shown in any one of SEQ ID NOs: 6-7, 9.

In the present application, the IL-21 or a functionally active fragment thereof may comprise the endogenous signal peptide thereof or a signal peptide derived from another protein. For example, the IL-21 or a functionally active fragment thereof may comprise a signal peptide derived from CCL19. In the present application, the IL-21 or a functionally active fragment thereof may comprise the amino acid sequence shown in any one of SEQ ID NOs: 10-11, 13.

The nucleic acid molecules of the present application may further comprise one or more nucleic acid sequences encoding a cleavable moiety. The nucleic acid sequence encoding the cleavable moiety is located between any two sequences selected from the group consisting of: the nucleic acid sequence encoding the chimeric antigen receptor (CAR); the nucleic acid sequence encoding IL-15 or a functionally active fragment thereof; and the nucleic acid sequence encoding IL-21 or a functionally active fragment thereof. For example, the nucleic acid sequence encoding a cleavable moiety may be comprised between a nucleic acid sequence encoding the CAR and a nucleic acid sequence encoding IL-15 or a functionally active fragment thereof. For example, the nucleic acid sequence encoding a cleavable moiety may be comprised between a nucleic acid sequence encoding IL-15 or a functionally active fragment thereof and a nucleic acid sequence encoding IL-21 or a functionally active fragment thereof. In certain embodiments, the nucleic acid sequence encoding a first cleavable moiety may be comprised between a nucleic acid sequence encoding the CAR and a nucleic acid sequence encoding IL-15 or a functionally active fragment thereof, and the nucleic acid sequence encoding a second cleavable moiety may be comprised between a nucleic acid sequence encoding IL-15 or a functionally active fragment thereof and a nucleic acid sequence encoding IL-21 or a functionally active fragment thereof. The first cleavable moiety and the second cleavable moiety may be identical or different.

For example, the CAR, the IL-15 or a functionally active fragment thereof, and the IL-21 or a functionally active fragment thereof may be expressed as a polyprotein. For example, the cleavable moiety may be comprised between 2 or more proteins in the polyproteins.

In the present application, the cleavable part may comprise 2A peptides. The 2A peptides may comprise P2A, T2A, F2A, or E2A.

For example, the cleavable moiety may comprise an amino acid sequence shown in any one of SEQ ID NOs: 14-16.

In certain cases, the nucleic acid molecule of the present application may further comprise a nucleic acid sequence encoding one or more additional exogenous proteins.

In the present application, the additional exogenous protein may comprise any one or more functional proteins. For example, the additional exogenous protein may be selected from the following group: cytokines, chemokines, and antibodies or their antigen-binding fragments. For example, the functional protein may be used to treat diseases related to gene defects. For example, the functional protein may be a protein that is absent and/or mutated in the host of the transfected cell.

In certain cases, the one or more additional exogenous proteins may comprise an antibody or an antigen-binding fragment thereof. For example, the antibody or antigen-binding fragment thereof may comprise multispecific antibodies or their antigen-binding fragments. For example, the multispecific antibodies or their antigen-binding fragments may simultaneously target immune effector cells (e.g., T cells, NK cells) and tumor cells.

For example, the multispecific antibodies or their antigen-binding fragments may comprise bispecific T cell engagers (BiTEs). For example, the BiTE may specifically bind at least one tumor-associated antigen (TAA) and T-cells. For example, the BiTE may specifically bind an antigen expressed on the surface of T-cells, for example, it may specifically bind to CD3 receptor. For example, the BiTE may specifically target MHC-independent tumor-associated antigens (TAAs). For example, the BiTE may comprise a CD3-binding domain. The BiTE may comprise at least one antigen-binding part, such as at least one single-chain antibody scFv. For example, the BiTE may comprise a tumor-associated antigen-binding domain. For example, the tumor-associated antigens may be selected from: GPC3, CD19, BCMA, Claudin18.2, and Mesothelin.

In the present application, when the isolated nucleic acid molecule encodes both a CAR and a BiTE, the tumor-associated antigen targeted by the BiTE and the tumor-associated antigen targeted by the CAR may be identical or different. In certain embodiments, the BiTE and the CAR target the same epitope. In certain embodiments, the BiTE and the CAR target the same target but specifically recognize or bind to different epitopes of that target. In certain embodiments, the BiTE and the CAR target different targets.

In the present application, the isolated nucleic acid molecule may also comprise one or more homologous regions. For example, each of the homologous regions may comprise at least 10 nucleotides (e.g., at least 20 nucleotides, at least 30 nucleotides, at least 40 nucleotides, at least 50 nucleotides, at least 60 nucleotides, at least 70 nucleotides, at least 80 nucleotides, at least 90 nucleotides, at least 100 nucleotides, at least 110 nucleotides, at least 120 nucleotides, at least 130 nucleotides, at least 140 nucleotides, at least 150 nucleotides, at least 160 nucleotides, at least 170 nucleotides, at least 180 nucleotides, at least 190 nucleotides, at least 200 nucleotides or more). For example, in the isolated nucleic acid molecule, the homology region may be located at the 3' end and/or the 5' end of the nucleic acid sequence encoding the CAR, the IL-15 or functionally active fragment thereof, or the IL-21 or functionally active fragment thereof. In certain embodiments, at least one the homologous region is located at the 5' end of a nucleic acid sequence encoding the CAR and at least another homologous region is located at the 3' end of a nucleic acid sequence encoding the IL-21 or a functionally active fragment thereof.

For example, the nucleic acid sequence of the homologous region (or homology arms) may have at least about 80%, at least about 85%, at least about 90%, at least about 91%, at least about 92%, at least about 93%, at least about 94%, at least about 95%, at least about 96%, at least about 97%, at least about 98%, at least about 99%, or 100% identity to the 5' and/or 3' ends of the genomic DNA sequences at the intended knock-in locus in the immune cell to be modified. For example, the homology arms may be 100-1000 bp in size. For example, the homology arm may be about 150 bp-1000 bp, may be about 200 bp-1000 bp, may be about 300 bp-1000 bp, may be about 400 bp-1000 bp, or may be about 800 bp-1000 bp.

In certain embodiments, the isolated nucleic acid molecule of the present application further comprises a 5' homologous arm, wherein the 5' homologous arm may be homologous to a target region in the genome of an immune cell. The target region may be located at a target gene (e.g., the coding region, the non-coding region, or other regulatory regions of that target gene). The target gene may be selected from the group consisting of: TRBC, TRAC, PD-1, CD95, AAVS1 and CCR5. In certain embodiments, in the isolated nucleic acid molecule, the 5' homology arm is located upstream of the nucleic acid sequence encoding a chimeric antigen receptor (CAR). For example, the 5' homologous arm may comprise a nucleic acid sequence shown in SEQ ID NO: 17.

In certain embodiments, the isolated nucleic acid molecule of the present application further comprises a 3' homologous arm, wherein the 3' homologous arm may be homologous to a target region in the genome of an immune cell. The target region may be located at a target gene (e.g., the coding region, the non-coding region, or other regulatory regions of that target gene). The target gene may be selected from the group consisting of: TRBC, TRAC, PD-1, CD95, AAVS1 and CCR5. In certain embodiments, in the isolated nucleic acid molecule, the 3' homologous arm is located downstream of the nucleic acid sequence encoding IL-21 or a functionally active fragment thereof. For example, the 3' homologous arm may comprise a nucleic acid sequence shown in SEQ ID NO: 18.

For example, the isolated nucleic acid molecule of the present application may comprise (in order from 5' to 3'): a 5' homology arm - a nucleic acid sequence encoding a CAR - a nucleic acid sequence encoding a cleavable moiety (e.g., a 2A peptide) - a nucleic acid sequence encoding IL-15 or a functionally active fragment thereof - a nucleic acid sequence encoding a cleavable moiety (e.g., 2A peptide) - a nucleic acid sequence encoding IL-21 or a functionally active fragment thereof - 3' homology arm.

For example, the isolated nucleic acid molecule of the present application may comprise (in order from 5' to 3'): a 5' homology arm - a nucleic acid sequence encoding a CAR - a nucleic acid sequence encoding a cleavable moiety (e.g., a 2A peptide) - a nucleic acid sequence encoding IL-15 or a functionally active fragment thereof - a nucleic acid sequence encoding a cleavable moiety (e.g., 2A peptide) - a nucleic acid sequence encoding IL-21 or a functionally active fragment thereof - poly A - 3' homology arm.

For example, the isolated nucleic acid molecule of the present application may comprise (in order from 5' to 3'): a 5' homology arm - a nucleic acid sequence encoding a CAR - a nucleic acid sequence encoding a cleavable moiety (e.g., a 2A peptide) - a nucleic acid sequence encoding a signal peptide of IL-7 - a nucleic acid sequence encoding IL-15 or a functionally active fragment thereof - a nucleic acid sequence encoding a cleavable moiety (e.g., 2A peptide) - a nucleic acid sequence encoding a signal peptide of CCL19 - a nucleic acid sequence encoding IL-21 or a functionally active fragment thereof - poly A - 3' homology arm.

In certain embodiments, the isolated nucleic acid molecule comprises a nucleic acid sequence selected from the group consisting of SEQ ID NOs: 20-21, 23-24, and 27-30.

The isolated nucleic acid molecule of the present application may be integrated into the genome of the immune cell. For example, the expression of the isolated nucleic acid molecule can be regulated by an endogenous promoter of the genome of the immune cell. For example, the expression of the isolated nucleic acid molecule may be regulated by an endogenous promoter after it has been integrated into the genome of the cell. For example, the homologous regions may be homologous to the target region of the immune cellular genomic DNA. For example, the target region may be located at a target gene (e.g., the coding region, the non-coding region, or other regulatory regions of that target gene). The target gene may be selected from: TCR-β subunit constant gene (TRBC), TCR-α subunit constant gene (TRAC), PD-1, CD95, AAVS1 and CCR5.

In the present application, the size of the isolated nucleic acid molecule may be at least about 1000bp (e.g., at least about 1500bp, at least about 2000bp, at least about 2500bp, at least about 3000bp, at least about 3500bp, at least about 4000bp, at least about 4500bp, at least about 5000bp, at least about 5500bp, at least about 6000bp, at least about 6500bp, at least about 7000bp, at least about 7500bp, at least about 8000bp, at least about 8500bp, at least about 9000bp, at least about 9500bp or more), wherein the isolated nucleic acid molecule is integrated into the genome of the transfected cell. For example, the size of the isolated nucleic acid molecule may be at least about 1 kb, for example, at least about 1.5 kb, at least about 2 kb, at least about 2.5 kb, at least about 3 kb, at least about 3.5 kb, at least about 4 kb, at least about 4.5 kb, at least about 5 kb, at least about 5.5 kb, at least about 6 kb, at least about 6.5 kb, at least about 7 kb, at least about 7.5 kb, at least about 8 kb, at least about 8.5 kb, at least about 9 kb, or larger.

In the present application, the isolated nucleic acid molecule may comprise circular nucleic acid molecules, supercoiled nucleic acid molecules, and/or linear nucleic acid molecules.

In the present application, the isolated nucleic acid molecule may comprise DNA molecule and/or RNA molecule.

In the present application, the isolated nucleic acid molecule may comprise single-stranded nucleic acid molecules and/or double-stranded nucleic acid molecules.

The present application provides a vector comprising the isolated nucleic acid molecule according to the present application. In certain embodiments, the vector is a non-viral vector (e.g., does not contain any viral component or any characteristic nucleic acid sequence derived from a virus). For example, the vector may be a plasmid.

In the present application, the term "vector" may be used to refer to a carrier isolated nucleic acid molecule into which a heterologous nucleic acid sequence may be inserted for introduction into a cell where it may be replicated and/or expressed. The nucleic acid sequence may be "heterologous," meaning that it is foreign to the cell to be transfected or the inserted nucleic acid sequence. Vectors comprise DNA, RNA, plasmids, cosmid, and artificial chromosomes (e.g., YACs). Those skilled in the art can construct vectors using recombinant preparation techniques (e.g., Sambrook *et al.*, 2001; Ausubel *et al.*, 1996). Vectors may be used to transfect cells to produce antibodies or other isolated proteins (e.g. CAR, cytokines, etc.).

In the present application, the vector may comprise regulatory sequences, such as promoters. Promoters are generally regions in the nucleic acid sequence that control the initiation and rate of transcription. Promoters can contain gene elements that can bind regulatory proteins and molecules (e.g., RNA polymerase and other transcription factors). Promoters may be used in conjunction with or without "enhancers," which are cis-acting regulatory sequences involved in the transcriptional activation of nucleic acid sequences.

Vectors or constructs generally comprise at least one termination signal. The "termination signal" or "terminator" is a DNA sequence that specifies the specific termination of RNA transcripts by RNA polymerase. Therefore, in certain embodiments, the termination signal for terminating RNA transcript production is considered. In eukaryotic systems, the terminator region may also contain specific DNA sequences that allow site-specific cleavage to expose polyadenylation sites. This sends a signal to a specific endogenous polymerase to add an extension sequence of about 200 A residues (polyA) to the 3' end of the transcript. RNA molecules modified with this polyA tail appear to be more stable and are translated more efficiently. Thus, in other embodiments involving eukaryotes, the terminator may comprise signals for cleaving RNA, e.g., the terminator signal may promote polyadenylation information.

The isolated nucleic acid molecule or vector of the present application may also comprise polyadenylation signals that affect the appropriate transcript polyadenylation.

In certain embodiments, the vector may comprise one or more replication origin sites (commonly called "ori"), which are specific nucleic acid sequences where replication begins. Alternatively, if the host cell is yeast, an autonomously replicating sequence (ARS) may be used.

In the present application, the plasmid may comprise circular plasmids. For example, the plasmid may be enzymatically degraded without being affected by DNase treatment. For example, the plasmid may be a circular plasmid, supercoiled plasmid, and/or linear plasmid. In the present application, the plasmid may comprise multiple cloning sites.

For example, the isolated nucleic acid molecule may be obtained by extraction from the host cell. Common plasmid extraction methods are well known to those skilled in the art.

### Method, kit and transfection composition for preparing immune cell

In another aspect, the present application provides a method for preparing a modified immune cell. The method comprises, transfecting (e.g., transfecting by electroporation) an immune cell to be modified with the isolated nucleic acid molecule of the present application (e.g., which comprises: a nucleic acid sequence encoding the chimeric antigen receptor (CAR); a nucleic acid sequence encoding the IL-15 or a functionally active fragment thereof; and a nucleic acid sequence encoding the IL-21 or a functionally active fragment thereof), or the vector of the present application (e.g., a plasmid vector comprising the nucleic acid molecule).

The method comprises, transfecting (e.g., transfecting by electroporation) an immune cell to be modified with the isolated nucleic acid molecule of the present application (e.g., which comprises in order from 5' to 3': a nucleic acid sequence encoding the chimeric antigen receptor (CAR); a nucleic acid sequence encoding the IL-15 or a functionally active fragment thereof; and a nucleic acid sequence encoding the IL-21 or a functionally active fragment thereof), or the vector of the present application (e.g., a plasmid vector comprising the nucleic acid molecule).

In certain embodiments, the method may comprise: transfecting an immune cell to be modified with a transfection composition comprising the nucleic acid molecule of the present application or the vector of the present application, so as to allow the cell to comprise and/or express the nucleic acid molecule, wherein at least a portion (e.g., at least 1w/w%, at least 5w/w%, at least 10w/w%, at least 15w/w%, at least 20w/w%, at least 25w/w%, at least 30w/w%, at least 35w/w%, at least 40w/w%, at least 45w/w%, at least 50w/w%, at least 55w/w%, at least 60w/w%, at least 65w/w%, at least 70w/w%, at least 75w/w%, at least 80w/w%, at least 85w/w%, at least 90w/w%, at least 95w/w%, at least 99w/w%, at least 100w/w%) of the nucleic acid molecule or the vector is derived from a host cell (e.g., at least a portion of the plasmid is derived from a microbial host cell); and in the portion of the nucleic acid molecule or the portion of the vector derived from the host cell, the amount of genomic DNA of the host cell is about 10% (w/w) or less (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In certain embodiments, the method may comprise: treating the isolated nucleic acid molecule with DNase. In certain embodiments, the DNase is capable of non-specifically cleaving linear DNA. In certain embodiments, the DNase comprises an exonuclease. In certain embodiments, the treatment can comprise contacting the isolated nucleic acid molecules with the DNase in the presence of Mg²⁺ and Ca²⁺.

In certain embodiments, the method may comprise: reducing the content of the host cell genomic DNA in the isolated nucleic acid molecule portion obtained from a host cell. For example, after the reduction, the content of genomic DNA of the host cell may be about 10% (w/w) or less (for example, about 10% or less, about 9% or less, about 8% or less, about 7% or less, about 6% or less, about 5% or less, about 4% or less, about 3% or less, about 2.5% or less, about 2% or less, about 1.5% or less, about 1% or less, about 0.9‰ or less, about 0.8‰ or less, about 0.7‰ or less, about 0.6‰ or less, about 0.55‰ or less, about 0.5‰ or less, about 0.45‰ or less, about 0.4‰ or less, about 0.35‰ or less, about 0.3‰ or less, about 0.25‰ or less, about 0.2‰ or less, about 0.15‰ or less, about 0.1‰ or less, about 0.05‰ or less, about 0.03‰ or less, about 0.01‰ or less, about 0.001‰ or less or lower; all are mass percentages).

In certain embodiments, the method may comprise: treating the isolated nucleic acid molecule portion derived from a host cell with DNase. In certain embodiments, the DNase is capable of non-specifically cleaving linear DNA. In certain embodiments, the DNase comprises an exonuclease. In certain embodiments, the treatment comprises contacting the portion of isolated nucleic acid molecule derived from the host cell with the DNase in the presence of Mg²⁺ and Ca²⁺.

In the present application, compared with isolated nucleic acid molecules not treated with deoxyribonuclease (DNase), the transfection efficiency of isolated nucleic acid molecules treated with deoxyribonuclease (DNase) may be increased by at least about 10%, at least about 20%, at least about 30%, at least about 40%, at least about 50%, at least about 60%, at least about 70%, at least about 80%, at least about 90%, at least about 100%, at least about 110%, at least about 120%, at least about 130%, at least about 140%, at least about 150%, at least about 200%, at least about 300%, at least about 400%, at least about 500%, at least about 1000% or more.

For example, the methods of the present application may be *in vitro* or *ex vivo* methods.

In another aspect, the present application provides a transfection (e.g., electrotransfection) reagent kit. The reagent kit comprises: 1) the portion of isolated nucleic acid molecule derived from the host cell according to any aspect of the present application (e.g., plasmids derived from microbial host cells); and 2) a reagent capable of reducing or degrading the genomic DNA of the host cell. In certain embodiments, the reagent of 2) comprises one or more selected from the group consisting of deoxyribonuclease (DNase), SDS, TX-100, CTAB, and cesium chloride-ethidium bromide. In certain embodiments, the DNase is capable of non-specifically cleaving linear DNA. In certain embodiments, the DNase comprises an exonuclease. In certain embodiments, the reagent kit also comprises a reagent containing Mg²⁺ and Ca²⁺.

In the present application, the reagent kit may also comprise reagents and/or instruments required for obtaining the transformed state of cells required for the electrotransfection. For example, the transformed state of cells may be stored at ultra-low temperature (e.g., -70°C). In the present application, during the electrotransfection, the voltage of the electroporator may be about 1500-2500V. In the present application, for the electrotransfection, the electroporation cuvette may be suitable for ultra-low temperature conditions.

In the present application, the DNase may cleave single-stranded DNA and/or double-stranded DNA. For example, the DNase may non-specifically cleave linear DNA. For example, the DNase may comprise a nucleic acid exonuclease. For example, the DNase may not have any RNase. In certain embodiments, the DNase may not cleave circular DNA. In certain embodiments, the DNase may not cleave single-stranded DNA.

In the present application, the reagent kit may also comprise a buffer solution, which may comprise Mg²⁺ and Ca²⁺. For example, the buffer solution may be the Reaction Buffer corresponding to the DNase. In the present application, the reagent kit may also comprise deionized water (e.g., DEPC-treated) required for DNase digestion of DNA.

In another aspect, the present application provides a reagent kit, wherein the reagent kit comprises: 1) isolated nucleic acid molecules derived from host cells as described in any aspect of the present application; and 2) an instruction, which describes the processing of the isolated nucleic acid molecules derived from host cell in 1) (e.g., plasmids derived from microbial host cells) by the methods of the present application and/or determining the quality of the transfection composition containing the isolated nucleic acid molecules derived from host cell (e.g., plasmids derived from microbial host cells) by the method of the present application.

In another aspect, the present application provides a transfection composition containing isolated nucleic acid molecules treated by the method of the present application.

In the present application, the transfection efficiency of the transfection composition (isolated nucleic acid molecules or vectors, such as plasmids) to cells may be determined by the following methods: 1) measuring the proportion of cells expressing isolated nucleic acid molecules in the transfected cells, wherein the transfection composition (or plasmid) contains the isolated nucleic acid molecules; and/or 2) measuring the proportion of cells containing the isolated nucleic acid molecules in the transfected cells, wherein the transfection composition contains isolated nucleic acid molecules.

In another aspect, the present application provides immune cells transfected with the isolated nucleic acid molecules, vectors or transfection composition of the present application.

In another aspect, the present application provides an immune cell prepared by the method of the present application.

In another aspect, the present application provides a cell population comprising the cells of the present application and/or their progeny. For example, the cell population may comprise at least 10³ cells (e.g., at least 10⁴ cells, at least 10⁵ cells, at least 10⁶ cells, at least 10⁷ cells, at least 10⁸ cells, at least 10⁹ cells, at least 10¹⁰ cells, or more).

In another aspect, the present application provides a pharmaceutical composition comprising the isolated nucleic acid molecules, the vectors, the transfection composition, the cells, and/or the cell population of the present application. In certain embodiments, the pharmaceutical composition further comprises a pharmaceutically acceptable adjuvant. The pharmaceutically acceptable adjuvants may comprise, for example, materials that do not cause significant irritation to the organism and do not significantly negatively affect the biological activity and properties of the active ingredient(s) (e.g., the modified cells or cell population). For example, the pharmaceutically acceptable adjuvants may comprise but are not limited to: diluents, buffers, binders, surfactants, wetting agents, adsorbents, lubricants, fillers, and/or disintegrants.

In another aspect, the present application provides the use of the isolated nucleic acid molecule of the present application, the vector of the present application, the cell of the present application, the cell population of the present application, and/or the pharmaceutical composition of the present application for the preparation of a medicament. In certain embodiments, the medicament is for the prevention, treatment, and/or alleviation of cancer.

In another aspect, the present application provides a method for preventing, treating, and/or alleviating a disease or disorder in a subject, comprising administering an effective amount of the isolated nucleic acid molecule of the present application, the vector of the present application, the transfection composition of the present application, the cell of the present application, the cell population of the present application, and/or the pharmaceutical composition of the present application to the subject. In certain embodiments, the disease or disorder is a cancer.

In another aspect, the present application provides an electrotransfection method (e.g., plasmid electrotransfection method), which comprises: electrotransfecting an immune cell (e.g., a T cell) with a plasmid comprising nucleic acid sequences encoding a CAR, IL-15 or a functionally active fragment thereof and IL-21 or a functionally active fragment thereof, thereby allowing the immune cell to comprise and/or express the CAR, IL-15 or a functionally active fragment thereof and IL-21 or a functionally active fragment thereof, wherein the plasmid is extracted from a host cell (e.g., a microorganism host cell), and the content of the host cell genomic DNA comprised in the plasmid accounts for about 10% (w/w) or less of the plasmid DNA content (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001%o (w/w) or less, or lower).

In another aspect, the present application provides an electrotransfection method (e.g., plasmid electrotransfection method), which comprises, in order from 5' to 3': electrotransfecting an immune cell (e.g., a T cell) with a plasmid comprising nucleic acid sequences encoding a CAR, IL-15 or a functionally active fragment thereof and IL-21 or a functionally active fragment thereof, thereby allowing the immune cell to comprise and/or express the CAR, IL-15 or a functionally active fragment thereof and IL-21 or a functionally active fragment thereof, wherein the plasmid is extracted from a host cell, and the content of the host cell genomic DNA comprised in the plasmid accounts for about 10% (w/w) or less of the plasmid DNA content (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower).

In another aspect, the present application provides an one-step electrotransfection method, which comprises: electrotransfecting an immune cell (e.g., a T cell) with a plasmid comprising nucleic acid sequences encoding a CAR, IL-15 or a functionally active fragment thereof and IL-21 or a functionally active fragment thereof, thereby allowing the immune cell to comprise and/or express the CAR, IL-15 or a functionally active fragment thereof and IL-21 or a functionally active fragment thereof (for example, encoding sequences of the CAR, IL-15 or a functionally active fragment thereof and IL-21 or a functionally active fragment thereof is knocked in to the genome of the immune cell and one or more endogenous genes in the genome of the immune cell are knocked out, for example, the endogenous genes may comprise CD3, PD-1, CD95, TRBC, TRAC, AAVS1 or CCR5, etc.), wherein the plasmid is extracted from a host cell, and the content of the host cell genomic DNA comprised in the plasmid accounts for about 10% (w/w) or less of the plasmid DNA content (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2‰ (w/w) or less, about 1.5‰ (w/w) or less, about 1‰ (w/w) or less, about 0.5‰ (w/w) or less, about 0.3‰ (w/w) or less, about 0.1‰ (w/w) or less, about 0.01‰ (w/w) or less, about 0.001‰ (w/w) or less, or lower). For example, the location of the nucleic acid sequence knocked-in may not be at the location of the gene desired to be knocked out.

In another aspect, the present application provides an one-step electrotransfection method, which comprises: electrotransfecting an immune cell (e.g., a T cell) with a plasmid comprising nucleic acid sequences encoding a CAR, IL-15 or a functionally active fragment thereof and IL-21 or a functionally active fragment thereof in order from 5' to 3', thereby allowing the immune cell to comprise and/or express the CAR, IL-15 or a functionally active fragment thereof and IL-21 or a functionally active fragment thereof (for example, encoding sequences of the CAR, IL-15 or a functionally active fragment thereof and IL-21 or a functionally active fragment thereof is knocked in to the genome of the immune cell and one or more endogenous genes in the genome of the immune cell are knocked out), wherein the plasmid is extracted from a host cell, and the content of the host cell genomic DNA comprised in the plasmid accounts for about 10% (w/w) or less of the plasmid DNA content (e.g., about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1.5% (w/w) or less, about 1% (w/w) or less, about 9‰ (w/w) or less, about 8‰ (w/w) or less, about 7‰ (w/w) or less, about 6‰ (w/w) or less, about 5‰ (w/w) or less, about 4‰ (w/w) or less, about 3‰ (w/w) or less, about 2%o (w/w) or less, about 1.5‰ (w/w) or less, about 1‰ (w/w) or less, about 0.5‰ (w/w) or less, about 0.1‰ (w/w) or less, about 0.01‰ (w/w) or less, about 0.001‰ (w/w) or less, or lower). For example, the location of the nucleic acid sequence knocked-in may not be at the location of the gene desired to be knocked out.

In certain embodiments, the plasmid may further comprise a nucleic acid sequence encoding one or more additional proteins (such as those described in other aspects of the present application), e.g., the one or more additional proteins may comprise a multispecific antibody or an antigen-binding fragment thereof (e.g., BiTE).

In certain embodiments, the electrotransfection method of the present application is a one-step electrotransfection method, of which the knock-in and/or knock-out of the exogenous gene is completed in a single electrotransfection step. For example, the location of the nucleic acid sequence knocked-in may not be at the location of the gene desired to be knocked out.

### Host genomic DNA

The host cell of the present application may be a prokaryotic cell or a eukaryotic cell, which comprises any transformable organism capable of replicating or expressing a heterologous gene encoded by a vector or expression vector. In the present application, the host cell is not or does not comprise an immune cell.

For example, the host may be a microbial host. For example, the host may be selected from one or more of the following group: bacteria, fungi, actinomycetes, mycoplasma, chlamydia, rickettsia, and spirochetes. For example, the host may comprise gram-negative bacteria. For example, the host may comprise Escherichia coli (for example, competent E. coli cells).

In the present application, the genomic DNA of the host cell may not be comprised in the isolated nucleic acid molecule.

In the present application, the content of the host cell genomic DNA may be determined by qPCR method.

In the present application, the size of the genomic DNA of the host cell may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger. For example, the size of the genomic DNA of the host cell may be at least about 10 kb. In the present application, the size of the genome DNA derived from the host may have a size of at least about 10kb (for example, may be at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger).

In the present application, the genomic DNA of the host cell may be derived from a microorganism. For example, the microorganism may be selected from one or more of the following group: bacteria, fungi, actinomycetes, mycoplasma, chlamydia, rickettsia, and spirochetes.

In the present application, the microorganism may comprise Gram-negative bacteria. For example, the microorganism may be suitable for preparing a skeletal vector of the vector. For example, the microorganism may comprise Escherichia coli.

In the present application, a content of the genomic DNA of the host cell of at least about 10kb (e.g., at least about 15kb, at least about 20kb, at least about 30kb, at least about 35kb, at least about 40kb, at least about 48kb, or larger) may account for about 10 (w/w)% or less, about 9 (w/w)% or less, about 8 (w/w)% or less, about 7 (w/w)% or less, about 6 (w/w)% or less, about 5 (w/w)% or less, about 4 (w/w)% or less, about 3 (w/w)% or less, about 2.5 (w/w)% or less, about 2 (w/w)% or less, about 1.5 (w/w)% or less, about 1 (w/w)% or less, about 9 (w/w)‰ or less, about 8 (w/w)‰ or less, about 7 (w/w)‰ or less, about 6 (w/w)‰ or less, about 5 (w/w)‰ or less, about 4 (w/w)‰ or less, about 3 (w/w)‰ or less, about 2 (w/w)‰ or less, about 1 (w/w)‰ or less, about 0.5‰ (w/w) or less, about 0.3‰(w/w) or less, about 0.1 (w/w)‰ or less, about 0.01 (w/w)‰ or less, about 0.001 (w/w)‰ or less, or lower, in the content of isolated nucleic acid molecules of the present application (e.g., plasmids) derived from the host cell in the transfection composition. For example, the content of the genomic DNA of the host cell at least about 10kb (e.g., at least about 15kb, at least about 20kb, at least about 30kb, at least about 35kb, at least about 40kb, at least about 48kb, or larger) may account for about 2% or less of the plasmid content in the transfection composition; may account for about 5‰ or less of the plasmid content in the transfection composition; may account for about 1‰ or less of the plasmid content in the transfection composition.

### Transfection

Transfection is a method of intentionally introducing nucleic acids into cells. In certain embodiments, transfection is non-viral, meaning that the sequences used in the plasmid context are non-viral, and the isolated nucleic acid molecules do not enter cells via viral mechanisms. Transfection of animal cells typically involves the opening of transient pores or "holes" in the cell membrane to allow uptake of the material. Transfection may be carried out using methods known in the art and those described below.

In the present application, the transfection may comprise electrotransfection. For example, the transfection may comprise electroporation of the cell to be modified. The electrotransfection may refer to cell electrotransfection or cell electroporation. The electrotransfection may utilize the effect of a strong instantaneous electric field to allow charged substances entering a cell by passing through a cell membrane with a certain permeability. The electrotransfection may produce little cytotoxicity. The cytotoxicity produced by the electrotransfection is significantly reduced compared to chemical transfection methods and/or viral transfection methods. The electrotransfection can be applied to almost all kinds of eukaryotic cells. The electrotransfection can be used for transient or stable expression of exogenous proteins.

In the present application, the transfection may comprise transfection of transposon systems (e.g., may comprise Sleeping beauty transposon system, or PiggyBac (PB) transposon system).

In the present application, the transfection may comprise transfection of cells (e.g., the cell to be transfected or to be modified according to the present application). In the present application, the cell may comprise eukaryotic cell. The eukaryotic cells may be animal cells. Among them, the animal cells may comprise mammalian cells (e.g., human cells, such as immune cells, such as T cells, such as human PBMCs).

In the present application, other transfection methods known in the art can also be comprised, such as chemically-based transfection methods and non-chemically-based transfection methods. Chemically-based transfection methods may comprise, for example, calcium phosphate, dendrimers, lipofection, and cationic polymers (e.g., DEAE-dextran or polyethyleneimine) methods. Non-chemical methods may comprise cell squeezing, sonoporation, optoporation, impalefection, and hydrodynamic delivery, etc. Particle-based methods, such as gene gun transfection, magnetofection (i.e., magnetically-assisted transfection), and particle bombardment, are also comprised.

In certain embodiments, electroporation is used to assist one or more isolated nucleic acid molecules in entering host cells. In the present application, "electroporation" or "electroloading" generally refers to applying current or electric field to cells to facilitate the entry of isolated nucleic acid molecules into cells. For example, flow electroporation may be performed using a flow electroporation instrument. In certain embodiments, static electroporation methods may be employed.

In the methods of the present application, transfection efficiencies of greater than about 35%, greater than about 40%, greater than about 50%, greater than about 60%, greater than about 70%, greater than about 80%, or greater than about 90% (or any range derivable therefrom) may be achieved after electroporating cells. Transfection efficiency may be measured by the percentage of cells expressing gene products or by the secretion levels of products expressed by the gene. During and after the electroporation process, cells maintain high viability. The viability is generally greater than about 40% or higher. The viability of electroporated cells may be at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or higher compared to the initial non-electroporated cell population or electroporated cell population transfected with a control construct.

Cell squeezing is a transfection method that delivers molecules to cells by gently squeezing the cell membrane. Cell squeezing is a high-throughput, carrier-free microfluidic platform for intracellular delivery. Cell squeezing does not rely on exogenous materials or electric fields.

Sonoporation uses high-intensity ultrasound to induce pore formation in the cell membrane. This pore formation is mainly attributed to bubble cavitation interacting with the nearby cell membrane, enhanced by the addition of ultrasound contrast agents (a source of cavitation nuclei).

Optoporation is a method of using highly focused lasers to instantaneously create tiny (approximately 1µm in diameter) pores in the cell's plasma membrane. In this technique, cells are processed one at a time, making it particularly suitable for single-cell analysis.

Chemically-based transfection may be divided into several categories: cyclodextrins, polymers, liposomes, or nanoparticles (with or without chemical or viral processes acting). For example, in the calcium phosphate method, a HEPES-buffered saline solution (HeBS) containing phosphate ions is combined with a calcium chloride solution containing the DNA to be transfected. When the two are combined, a fine precipitate of positively charged calcium and negatively charged phosphate forms, with the DNA to be transfected binding to its surface. The suspension of the precipitate is then added to the cells to be transfected (usually monolayer cell cultures). Cells take up some of the precipitate along with the associated DNA. Other methods use highly branched organic compounds (so-called dendrimers) to bind DNA and facilitate its entry into cells. A very effective method is to encapsulate the DNA to be transfected in liposomes, which are small membrane-coated vesicles that, in some ways, resemble cell structures and can actually fuse with the cell membrane, releasing DNA into the cell. For eukaryotic cells, the use of cationic liposomes (or mixtures thereof) achieves better transfection because cells are more sensitive. Another method is to use cationic polymers, such as DEAE-dextran or polyethyleneimine. Negatively charged DNA binds to polycations, and the complex is accepted by cells through endocytosis.

In certain embodiments, a direct transfection method is using a gene gun, in which DNA is coupled to inert solid nanoparticles (typically gold), and then directly "shot" into the nucleus of the target cell.

Magnetofection or magnet-assisted transfection is a transfection method that uses magnetic force to deliver DNA to target cells. First, nucleic acids are associated with magnetic nanoparticles. Then, magnetic force is applied to deliver the nucleic acid-particle complexes to the target cells and into the target cells, where the payload is released.

Puncture transfection is performed by piercing cells with elongated nanostructures and arrays of such nanostructures, such as carbon nanofibers or silicon nanowires functionalized with plasmid DNA.

Another particle-based transfection method is called particle bombardment. Nucleic acids, typically connected to microprojectiles, are delivered at high speed through membrane penetration.

In the present application, the transfection may comprise stable transfection. For example, the transfection may stably express the exogenous protein encoded by the exogenous gene (such as the isolated nucleic acid molecules of the present application) in the transfected cell. For example, the transfection may result in exogenous genes being incorporated into the genome of the transfected cell (e.g., an immune cell, such as a T cell). In the present application, the integration may occur at a specific location in the genome.

### Cell to be transfected or modified

In the present application, the terms "cell", "cell line", and "cell culture" may be used interchangeably. All these terms also comprise freshly isolated cells, as well as cells that have been cultured, activated, or expanded *ex vivo.* All these terms also comprise their progeny, i.e., any and all descendants. It should be understood that progeny cells may be not identical due to intentional or unintentional mutations.

In the present application, the cell to be transfected or modified may be a eukaryotic cell, such as an animal cell. For example, the cell may be a mammalian cell. For example, the cell may be a human cell.

In the present invention, the cell may preferably be an immune effector cell. For example, the cell may be a T lymphocyte, B lymphocyte, NK cell, macrophage, dendritic cell, monocyte, granulocyte, and/or mast cell. For example, the cell may be a peripheral blood lymphocyte.

For example, the cell may be a primary cell. For example, the cell may comprise an allogeneic cell and/or an autologous cell derived from the subject. For example, in certain cases, the cell is an autologous cell derived from the subject. For example, in certain cases, the cell is an allogeneic cell derived from other donors.

For example, the cell may be an activated cell. For example, the activation may comprise contacting the cell to be modified with an activation composition.

For example, the cell may comprise immune cells (e.g., immune effector cells, such as T cells or NK cells, e.g., PBMCs, e.g., primary or autologous T cells or NK cells, e.g., primary or autologous PBMCs), and the activation composition may comprise anti-CD3 and/or anti-CD28 antibodies (e.g., the antibodies may be provided on magnetic beads).

Reagents or kits for activating T cells are also commercially available. Exemplary kits comprise streptavidin particles (e.g., MACSiBead or Dynabead) and biotinylated antibodies against human CD2, CD3, and CD28. Streptavidin particles loaded with biotinylated antibodies are used to simulate antigen-presenting cells and activate resting T cells from PBMCs as well as purified T cells. T cell expansion is achieved by culturing and reactivating on day 14 of cultivation. For example, T cells may also be activated by mitogens (e.g., ConA, PHA, and PWM).

For example, the activation may comprise contacting the cell to be modified with the activation composition for no more than about 4 days (e.g., within about 96 hours, 90 hours, 85 hours, 80 hours, 75 hours, 72 hours, 70 hours, 65 hours, 60 hours, 55 hours, 50 hours, 48 hours, 45 hours, 40 hours, 36 hours, 30 hours, 24 hours, 20 hours, 15 hours, 12 hours, 8 hours, or shorter times). For example, the activation may comprise contacting the cell to be modified with the activation composition for about 10 hours to about 48 hours (e.g., about 12 hours to about 24 hours). For example, the method may comprise performing the transfection while the cell to be transfected is in contact with the activation composition for about 2 days or shorter periods.

In certain embodiments, transfection may be carried out on any prokaryotic or eukaryotic cells. In certain aspects, electroporation involves transfecting human cells. In other aspects, electroporation involves transfecting animal cells. In certain aspects, transfection involves transfecting cell lines or hybrid cell types. In certain aspects, the cell to be transfected is a cancer cell, tumor cell, or immortalized cell. In certain cases, tumors, cancers, immortalized cells, or cell lines are induced, while in other cases, tumors, cancers, immortalized cells, or cell lines naturally enter their respective states or conditions. In certain aspects, cells or cell lines may comprise A549, B cells, B16, BHK-21, C2C12, C6, CaCo-2, CAP/, CAP-T, CHO, CHO2, CHO-DG44, CHO-K1, COS-1, Cos-7, CV-1, dendritic cells, DLD-1, embryonic stem (ES) cells or derivatives, H1299, HEK, 293, 293T, 293FT, Hep G2, hematopoietic stem cells, HOS, Huh-7, induced pluripotent stem cells (iPSC) or derivatives, Jurkat, K562, L5278Y, LNCaP, MCF7, MDA-MB-231, MDCK, mesenchymal cells, Min-6, monocytes, Neuro2a, NIH 3T3, NIH3T3L1, K562, NK-cells, NS0, Panc-1, PC12, PC-3, peripheral blood cells, plasma cells, primary fibroblasts, RBL, Renca, RLE, SF21, SF9, SH-SY5Y, SK-MES-1, SK-N-SH, SL3, SW403, Stimulus-triggered Acquisition of Pluripotency (STAP) cells or derivatives SW403, T-cells, THP-1, tumor cells, U2OS, U937, peripheral blood lymphocytes, expanded T-cells, hematopoietic stem cells, or Vero cells. In certain specific embodiments, the cell is a peripheral blood lymphocyte, expanded T-cell, natural killer cell (NK cell), stem cell, hematopoietic stem cell, or primary cell. In certain specific embodiments, the cell is a hematopoietic stem cell. In other specific embodiments, the cell is peripheral blood lymphocytes and/or peripheral blood mononuclear cells (PBMC).

In certain embodiments, the cell is a difficult-to-transfect cell known in the art. Such cells are known in the art and comprise, for example, primary cells, insect cells, SF9 cells, Jurkat cells, CHO cells, stem cells, slowly dividing cells, T-cells, and non-dividing cells. In certain embodiments, the cell is a T-cell. In certain embodiments, the cell is a primary cell. In certain embodiments, the cell is a stem cell. In certain embodiments, the cell is a hematopoietic stem cell, comprising bone marrow and lymphoid progenitors. In certain embodiments, the cell is a mesenchymal stem cell. In certain embodiments, the cell is a germ cell, such as an oocyte or sperm cell.

In certain embodiments, cells may be cultured before or after transfection. For example, cells may be cultured during a post-transfection selection stage, during a maintenance and cloning selection and initial expansion stage, during a screening stage, and/or during a large-scale production stage. Methods for culturing suspension and adherent cells are known to those skilled in the art. In certain embodiments, cells may be cultured using commercially available cell culture containers and cell culture media.

In the present application, the stem cell may comprise hematopoietic stem cell and/or mesenchymal stem cell. The hematopoietic stem cell may differentiate into blood cell (e.g., blood cell of the bone marrow lineage and blood cell of the lymphoid lineage). The hematopoietic stem cell may have multipotency and self-renewal characteristics. The hematopoietic stem cells may differentiate into cells selected from the following group: monocytes, macrophages, neutrophils, basophils, eosinophils, erythrocytes, megakaryocytes, platelets, T cells, B cells, and NK cells.

The mesenchymal stem cells may be adult stem cells with self-renewal, multilineage differentiation potential, and the ability to maintain their biological characteristics after large-scale *in vitro* expansion, derived from the early mesoderm of embryonic development. The mesenchymal stem cell may express HLA-I class antigens. The mesenchymal stem cell may not express or may have low expression of HLA-II class antigens. The mesenchymal stem cells may have the ability to differentiate into adipocytes, osteoblasts, and chondrocytes, and may support the differentiation of hematopoietic stem cells into granulocytes, macrophages, and megakaryocytes. The mesenchymal stem cells may secrete cytokines, for example, they may secrete CSF-1, GM-CSF, G-CSF, IL-6, c-kit ligand, and/or IL-3.

In the present application, the immune cell may comprise immune effector cell. For example, the immune effector cell may comprise lymphocytes (e.g., cytotoxic T cells, memory T cells), macrophages, dendritic cells, and NK cells. For example, the immune cell may be selected from the following group: T lymphocytes (e.g., may be activated T lymphocytes or non-activated T lymphocytes), B lymphocytes, NK cells, macrophages, dendritic cells, monocytes, granulocytes, and mast cells.

### Transfection composition

In the present application, the transfection composition may comprise the isolated nucleic acid molecules (e.g., plasmids). For example, the plasmid may be a circular plasmid, supercoiled plasmid, or linear plasmid. In the present application, the plasmid (e.g., linear plasmid) may be treated (e.g., with a deoxyribonuclease, such as an exonuclease, such as Exonuclease V) to satisfy the conditions of the present application for the content of host genomic DNA with a size of at least about 10kb (e.g., at least about 15kb, at least about 20kb, at least about 30kb, at least about 35kb, at least about 40kb, at least about 48kb or larger). In the present application, the treatment may not affect the structure or function of an isolated nucleic acid molecule of the present application with a circular structure.

For example, the transfection composition of the present application may substantially not or even totally not comprise any viral vectors, such as the content of viral vectors being less than about 2 (w/w)%, less than about 1 (w/w)%, less than about 0.9 (w/w)%, less than about 0.8 (w/w)%, less than about 0.7 (w/w)%, less than about 0.6 (w/w)%, less than about 0.5 (w/w)%, less than about 0.4 (w/w)%, less than about 0.3 (w/w)%, less than about 0.2 (w/w)%, or less than about 0.1 (w/w)%.

In the present application, the plasmid may be a DNA plasmid. For example, the DNA plasmid may be a double-stranded, circular DNA molecule. For example, the DNA plasmid may be a double-stranded, linear DNA molecule.

In the present application, the genomic DNA of the host (for example, with a size of at least about 10kb (for example, at least about 15kb, at least about 20kb, at least about 30kb, at least about 35kb, at least about 40kb, at least about 48kb, at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger)) and the plasmid may exist in different nucleic acid molecules. For example, the genomic DNA of the host may be freely present in the transfection composition. For example, the genomic DNA of the host may be out of the plasmid. The freedom may be present in a separated manner without attachment to the plasmid.

In the present application, the concentration of the isolated nucleic acid molecule (e.g., the vector) in the transfection composition may be about 5 µg/mL to about 3000 µg/mL (e.g., about 5 µg/mL to about 2500 µg/mL, about 10 µg/mL to about 2000 µg/mL, about 10 µg/mL to about 1500 µg/mL, about 5 µg/mL to about 1000 µg/mL, about 10 µg/mL to about 1200 µg/mL, about 8 µg/mL to about 1000 µg/mL, about 15 µg/mL to about 950 µg/mL, about 20 µg/mL to about 900 µg/mL, about 30 µg/mL to about 900 µg/mL, about 40 µg/mL to about 950 µg/mL, about 50 µg/mL to about 950 µg/mL, about 60 µg/mL to about 950 µg/mL, about 70 µg/mL to about 950 µg/mL, about 80 µg/mL to about 950 µg/mL, about 90 µg/mL to about 950 µg/mL, about 100 µg/mL to about 950 µg/mL, about 110 µg/mL to about 950 µg/mL, about 120 µg/mL to about 950 µg/mL, about 130 µg/mL to about 950 µg/mL, about 140 µg/mL to about 950 µg/mL, about 150 µg/mL to about 950 µg/mL, about 180 µg/mL to about 950 µg/mL, about 200 µg/mL to about 950 µg/mL, about 200 µg/mL to about 850 µg/mL, about 200 µg/mL to about 800 µg/mL, about 250 µg/mL to about 850 µg/mL, about 300 µg/mL to about 750 µg/mL, about 350 µg/mL to about 700 µg/mL, about 400 µg/mL to about 650 µg/mL, about 450 µg/mL to about 600 µg/mL, about 500 µg/mL to about 550 µg/mL, etc.). In certain embodiments, the concentration of the isolated nucleic acid molecule (e.g. the plasmid) in the transfection composition is about 200 µg/mL to about 800 µg/mL.

In another aspect, the present application provides a transfection composition prepared by the method according to the present application.

In the present application, the transfection composition can significantly increase the transfection efficiency, and in particular can significantly increase the transfection efficiency of the nucleic acid molecules of the present invention to immune cells. The transfection composition may significantly reduce cytotoxicity caused by transfection. The transfection composition may be directly applicable for cell transfection. The method according to the present application may serve as a quality control method for the preparation and/or quality inspection of the transfection composition.

### Optimization of nucleic acid molecule

For example, the method may also comprise treating the isolated nucleic acid molecules (for example, plasmid) derived from the host cell to reduce the content of the host cell's genomic DNA therein.

For example, the treatment may comprise contacting the isolated nucleic acid molecules (for example, plasmid) derived from the host cells with one or more reagents selected from the following group: deoxyribonuclease (DNase), SDS, TX-100, CTAB, and cesium chloride-ethidium bromide. For example, the DNase may non-specifically cleave linear DNA. For example, the DNase may comprise an exonuclease. For example, the treatment may comprise contacting the isolated nucleic acid molecules (for example, plasmid) derived from the host cells with the reagent in the presence of Mg²⁺ and Ca²⁺.

The method of the present application may comprise the following steps: reducing the content of host genomic DNA in the transfection composition. In the present application, the reduction may refer to reducing the content of the host genomic DNA in the transfection composition to meet the requirements of the method according to the present application.

In the present application, the reduction may comprise purifying the isolated nucleic acid molecule. That is, through the purification, the content of genomic DNA from the host cell in the isolated nucleic acid molecule (e.g., a plasmid) can be reduced to conform to the requirements of the methods of the present application. In the present application, the host genomic DNA of about 10kb (e.g., at least about 15kb, at least about 20kb, at least about 30kb, at least about 35kb, at least about 40kb, at least about 48kb or larger) in size may be removed by the purification; and/or, the genomic DNA derived from the host (e.g., microorganism) may be removed. For example, the purification may comprise reducing the content of genomic DNA of host cell in the isolated nucleic acid molecule (e.g., plasmid) using methods for purifying DNA known to those skilled in the art. In the present application, the purification may not affect the integrity and/or activity of the DNA of the isolated nucleic acid molecule (such as the plasmid). In the present application, the reduction may comprise purifying the isolated nucleic acid molecule (such as the plasmid) using a reagent selected from the group consisting of: DNAase, SDS, TX-100, CTAB, and cesium chloride-ethidium bromide. For example, the reduction may use DNAase. For example, the DNAase may not affect the integrity and/or activity of the circular plasmid DNA.

In the present application, the DNase may cleave double-stranded DNA. For example, the DNase may non-specifically cleave linear DNA.

In the present application, the DNase may (e.g., non-specifically) cleave linear (e.g., double-stranded linear) DNA. For example, the DNase may not affect the structure and/or activity of the plasmid (e.g., circular plasmid). In the present application, the DNase may comprise deoxyribonuclease. In the present application, the DNase may comprise DNA exonuclease. In the present application, the DNase may comprise exonuclease V. In the present application, the DNase may comprise ATP-Dependent DNase, such as Plasmid-Safe^{™} ATP-Dependent DNase.

For example, the method of the present application may comprise the following steps: contacting the DNase with the plasmid (or the isolated nucleic acid molecule) of the present application. In the present application, the contact may comprise contacting in the presence of a buffer (e.g., the buffer may be from the kit sold with the DNase). In the present application, the buffer may comprise Mg²⁺ and Ca²⁺. For example, the buffer may contain Mg²⁺ and Ca²⁺ and no other additional cations. The buffer may improve the enzymatic efficiency of the DNase.

In the method of the present application, the DNase treatment may comprise the following steps: purifying the plasmid or the isolated nucleic acid molecule after the contact.

After treatment with the DNase, the content of host genomic DNA of at least about 10 kb (e.g., at least about 15 kb, at least about 20 kb, at least about 30 kb, at least about 35 kb, at least about 40 kb, at least about 48 kb, or greater) in size in the isolated nucleic acid molecule (or the vector, e.g., the plasmid) may be less than about 10% of the total content of the isolated nucleic acid molecule.

In the present application, the contact may also comprise further contact with an RNase.

In the present application, a nuclease or DNase is an enzyme that hydrolyzes nucleic acids. For example, the DNase may be an exonuclease. Exonucleases are any group of enzymes that catalyze the hydrolysis of individual nucleotides from the ends of DNA or RNA chains. Nucleases may also be classified based on whether they specifically digest DNA or RNA. Nucleases that specifically catalyze the hydrolysis of DNA may be called deoxyribonucleases or DNAases, while nucleases that specifically catalyze the hydrolysis of RNA may be called ribonucleases or RNAases. Some nucleases have specificity for single-stranded or double-stranded nucleic acid sequences. Some enzymes have both exonuclease and endonuclease characteristics. In addition, some enzymes are capable of digesting both DNA and RNA sequences.

In different nucleases, optimal reaction conditions are different. Factors to consider comprise temperature, pH, enzyme cofactors, salt composition, ionic strength, and stabilizers. Suppliers of commercially available nucleases (e.g., Promega Corp.; New England Biolabs, Inc.) provide information on the optimal conditions for various enzymes. As measured at incubation temperature, most nucleases are used between pH 7.2 and pH 8.5. In addition, most nucleases exhibit maximum activity at 37°C; however, a few enzymes require higher or lower temperatures for optimal activity (e.g., Taq I, 65°C; Sma I, 25°C). DNA concentration may also be a factor, as high DNA concentration may reduce enzyme activity, and too diluted DNA concentration may be below the enzyme's Km and also affect enzyme activity. Non-limiting examples of nucleases comprise DNAse I, Benzonase, exonuclease I, exonuclease III, Mung Bean Nuclease, nuclease BAL 31, RNase I, S1 nuclease, Lambda Exonuclease, RecJ, and T7 Exonuclease. DNAse I is an endonuclease that non-specifically cleaves DNA to release dinucleotides, trinucleotides, and oligonucleotides with 5'-phosphorylated and 3'-hydroxylated termini. DNAse I acts on single-stranded and double-stranded DNA, chromatin, and RNA:DNA hybrids. Exonuclease I catalyzes the removal of nucleotides from single-stranded DNA in the 3' to 5' direction. Exonuclease III catalyzes the stepwise removal of mononucleotides from the 3'-hydroxyl termini of double-stranded DNA. Exonuclease III also acts on nicks in double-stranded DNA to produce single-stranded gaps. Single-stranded DNA is resistant to exonuclease III. Mung Bean Nuclease degrades single-stranded extensions from DNA ends. Mung Bean Nuclease is also an RNA endonuclease. Nuclease BAL 31 degrades both 3' and 5' ends of double-stranded DNA. Nuclease BAL 31 is also a highly specific single-stranded endonuclease that cleaves at nicks, gaps, and single-stranded regions in double-stranded DNA and RNA. RNase I is a single-strand-specific RNA endonuclease that cleaves at all RNA dinucleotide sites. S1 nuclease degrades single-stranded DNA and RNA by endonucleolysis to obtain 5'-phosphorylated termini products. Double-stranded nucleic acids (DNA:DNA, DNA:RNA, or RNA:RNA) are resistant to S1 nuclease degradation, except at very high enzyme concentrations. Lambda Exonuclease catalyzes the removal of 5' mononucleotides from double-stranded DNA. Its preferred substrate is 5'-phosphorylated double-stranded DNA, although Lambda Exonuclease also degrades single-stranded and non-phosphorylated substrates at a greatly reduced rate. Lambda Exonuclease cannot initiate DNA digestion at breaks or nicks. RecJ is a single-stranded DNA-specific exonuclease that catalyzes the removal of deoxynucleotide monophosphates from DNA in the 5' to 3' direction. T7 Exonuclease catalyzes the removal of 5' mononucleotides from double-stranded DNA. T7 Exonuclease catalyzes the removal of nucleotides from the 5' end or at nicks and gaps in double-stranded DNA.

### Gene editing system

In the present application, the transfection composition may also comprise a gene editing system capable of integrating the nucleic acid molecule into a specific location in the genome of the cell (e.g., an immune cell, e.g., a T cell). For example, the gene editing system may comprise a site-specific enzyme (or referred to as a localizing nuclease) or an isolated nucleic acid molecule encoding the same (e.g., a DNA molecule or an RNA molecule, such as an mRNA encoding the enzyme). For example, the site-specific enzyme may be selected from: transcription activator-like effector nucleases (TALEN), zinc-finger nucleases (ZFN), transposases, integrases, and Cas proteins. For example, the Cas protein is Cas9 protein. For example, the gene editing system further comprises one or more guide RNAs. For example, the guide RNA is complementary to the nucleic acid sequence in the target region of the cell genome.

For example, the gene editing system may also comprise one or more guide RNAs targeting one or more genes to be knocked out.

In the method and composition of the present application, the site-specific enzymes may be provided in the form of a protein or a nucleic acid molecule (e.g., a DNA molecule or an RNA molecule). The site-specific enzyme and the guide RNA may be provided simultaneously (e.g., in the same molecule, the same complex or the same composition) or separately (e.g., in different molecules or different compositions).

For example, the gene editing system comprises a ribonucleoprotein complex (RNP), and the RNP comprises the Cas protein and the guide RNA.

For example, the gene editing systems and methods may be known to those skilled in the art, as long as the purpose of gene editing may be achieved, and not limited to a specific method. In the present application, the gene editing method may be selected from one or more of the following groups: CRISPR/Cas system, RNA editing system ADAR, RNA-guided endonucleases, zinc-finger proteinases, Mega-TAL nucleases, TALENs, and Meganucleases. For example, the gene editing method may employ the CRISPR/Cas system.

In the present application, the gene editing may comprise gene editing knockout and/or gene editing knock-in. For example, the gene editing may comprise gene editing knock-in. For example, CRISPR/Cas system may be used for the gene editing knock-in.

For example, the CRISPR-CAS system may comprise a class of clustered regularly interspaced short palindromic repeats (CRISPRs) and some functionally related proteins (CRISPR-associated, Cas). The Cas protein-encoding genes may comprise Cas9, Cas1, Cas2, and Csn2. For example, the Cas protein may be Cas9. The CRISPR/CAS system (such as the CRISPR/Cas9 system) may have targeted cleavage specificity to DNA molecules.

For example, the gene editing knock-in may be a process where Cas protein targets a specific DNA sequence in the cell for DNA sequence insertion. The gene editing knock-in may be a Cas protein (such as Cas 9 protein) mediated homologous recombination between the exogenous gene to be knocked-in in the donor plasmid and the specific DNA sequence in the targeted cell.

In the present application, the isolated nucleic acid molecule (e.g., plasmid) can serve as a donor plasmid in gene editing knock-in. For example, the donor plasmid may be double-stranded DNA or single-stranded DNA. The donor plasmid may serve as a donor template for the HDR repair mechanism.

The site-specific enzyme of the present application can cleave the bonds between specific nucleotide subunits in the nucleic acid sequence (i.e., phosphodiester bonds). In a specific embodiment, the site-specific enzyme is encoded on RNA. In other embodiments, the site-specific enzyme is an enzyme-active protein, enzyme, or small molecule mimics. In certain embodiments, the site-specific enzyme is encoded on DNA. In a specific embodiment, the site-specific enzyme is encoded on plasmid DNA. In certain embodiments, the site-specific enzyme and donor DNA are encoded on the same plasmid.

In one embodiment, the site-specific enzyme is a transposase. The transposase may be a Sleeping Beauty transposase. In certain cases, the transposase may be a PiggyBac (PB) transposase.

For example, a synthetic DNA transposon can be used, such as the "Sleeping Beauty" transposon system, which is designed for introducing a precisely defined DNA sequence into the chromosome of a vertebrate. The Sleeping Beauty transposon system consists of the Sleeping Beauty (SB) transposase and a transposon designed to insert a specific DNA sequence into the vertebrate genome. DNA transposons translocate from one DNA site to another in a simple cut-and-paste manner. Transposition is a precise process in which a defined DNA fragment is excised from one DNA molecule and moved to another site within the same or different DNA molecule or genome. The SB transposase inserts the transposon into the TA dinucleotide base pairs of the recipient DNA sequence. The insertion site may be another location within the same DNA molecule or within another DNA molecule (or chromosome). In mammalian (comprising human) genomes, there are approximately 200 million TA sites. During the transposon integration process, the TA insertion site is duplicated. The duplication of the TA sequence is a signature of transposition and is used to determine the mechanism in some experiments. The transposase may be encoded within the transposon, or the transposase may be provided by another source, in which case the transposon becomes a non-autonomous element. Non-autonomous transposons are the most useful genetic tools because they cannot independently excise and reinsert after integration. All DNA transposons identified in the human genome and other mammalian genomes are non-autonomous because although they contain transposase genes, these genes are non-functional and cannot produce a transposase capable of moving the transposon.

In the present application, the transposon system may comprise a transposon. The transposon may comprise an isolated nucleic acid molecule capable of being incorporated into nucleic acids by transposase. The transposon may contain two transposon ends (also known as "arms") connected by a sequence long enough to form a loop in the presence of transposase. The transposon may be double-stranded, single-stranded, or mixed, containing single-stranded and double-stranded regions, depending on the transposase used for inserting the transposon. The transposase may comprise Mu, Tn3, Tn5, Tn7, and/or Tn10. The ends of the transposon may be double-stranded. In the present application, the transposon may be inserted into double-stranded DNA through transposition events. In the present application, the transposon may be considered to be the exogenous gene. The plasmid may contain the transposon. The plasmid may also contain the isolated nucleic acid molecules necessary for the transposition event. For example, the transposon system may comprise the Sleeping Beauty transposon system. The Sleeping Beauty transposon system is a member of the Tc1/mariner transposon superfamily. For example, the transposon system may comprise the Piggy Bac transposon system.

In one embodiment, the site-specific enzyme is an integrase. For example, phiC31 integrase is a sequence-specific recombinase encoded within the genome of bacteriophage phiC31. PhiC31 integrase mediates the recombination between two 34 base pair sequences called attachment sites (att), one obtained from the bacteriophage and the other obtained from the bacterial host. It has been shown that this serine integrase works efficiently in many different cell types (comprising mammalian cells). In the presence of phiC31 integrase, donor plasmids containing attB may be unidirectionally integrated into the target genome by recombination at sites with sequences similar to the natural attP site (called pseudo-attP sites). PhiC31 integrase can integrate any size plasmid as a single copy without the need for cofactors. The integrated transgenes are stably expressed and heritable.

In one embodiment, the site-specific nuclease is a Cas nuclease. In a related embodiment, the Cas nuclease is Cas9. In another embodiment, the nuclease is cas9 and the composition further comprises guide RNA. Another example of a sequence-specific nuclease system that may be used in conjunction with the methods and compositions described herein comprises the Cas9/CRISPR system (Wiedenheft, B. et al. Nature 482, 331-338 (2012); Jinek, M. et al. Science 337, 816-821 (2012); Mali, P. et al. Science 339, 823-826 (2013); Cong, L. et al. Science 339, 819-823 (2013)). The Cas9/CRISPR (Clustered Regularly Interspaced Short Palindromic Repeat) system exploits RNA-guided DNA binding and sequence-specific target DNA cleavage. The guide RNA/Cas9 complex confers site specificity to the nuclease. The guide RNA (gRNA) comprises about 20 nucleotides complementary to the target genomic DNA sequence, which is upstream of the genomic PAM (Protospacer Adjacent Motif) site (NNG) and constant RNA scaffold region. The Cas (CRISPR-associated)9 protein binds to gRNA and the target DNA bound to gRNA, and introduces double-strand breaks at a defined position upstream of the PAM site. Cas9 has two independent nuclease domains homologous to HNH and RuvC endonucleases, and by mutating either of the two domains, Cas9 protein may be converted into a nickase that introduces single-strand breaks (Cong, L. et al. Science 339, 819-823 (2013)). In particular, the methods and compositions of the present invention may be used with both single-strand and double-strand induction forms of Cas9, as well as other RNA-guided DNA nucleases (e.g., Cas9-like systems from other bacteria).

The site-specific nucleases of the methods and compositions described herein may be modified, chimeric, or isolated from an organism. Sequence-specific nucleases may be introduced into cells in the form of RNA encoding a sequence-specific nuclease (e.g., mRNA).

In one embodiment, the site-specific enzyme is a site-specific nuclease, such as a zinc finger nuclease. Zinc finger nucleases typically comprise a DNA binding domain (i.e., zinc finger) and a cleavage domain (i.e., nuclease). The zinc finger binding domain may be engineered to recognize and bind any selected nucleic acid sequence. Compared to naturally occurring zinc finger proteins, engineered zinc finger binding domains may have new binding specificities. Engineering methods comprise, but are not limited to, rational design and various types of selection. In certain embodiments, the zinc finger nuclease may also comprise a nuclear localization signal or sequence (NLS). The NLS is an amino acid sequence that facilitates targeting of the zinc finger nuclease protein to the nucleus to introduce double-strand breaks at the target sequence on the chromosome. Nuclear localization signals are known in the art. See, for example, Makkerh et al. (1996) Current Biology 6: 1025-1027. The part of cleavage domain of the zinc finger nuclease may be obtained from any endonuclease or exonuclease. Zinc finger nucleases also comprise a cleavage domain. Non-limiting examples of cleavage domains that may be derived from endonucleases comprise, but are not limited to, restriction endonucleases and homing endonucleases.

In another aspect, the targeted endonuclease may be a meganuclease. Meganucleases are characterized by large recognition sites of endonucleases, that is, recognition sites typically ranging from about 12 base pairs to about 40 base pairs. As a result of this requirement, recognition sites typically appear only once in any given genome. Naturally occurring meganucleases recognize 15 to 40 base pair cleavage sites and are generally divided into four families: LAGLIDADG family, GIY-YIG family, His-Cyst box family, and HNH family. Meganucleases may be targeted to specific chromosomal sequences by modifying their recognition sequences using techniques well-known to those skilled in the art.

In another aspect, the targeted endonuclease may be a transcription activator-like effector (TALE) nuclease. TALEs are transcription factors derived from the plant pathogen Xanthomonas, which may be easily modified to bind new DNA targets. TALEs or their truncated forms may be connected to the catalytic domains of endonucleases (e.g., Fok1) to produce targeted endonucleases called TALE nucleases or TALENs.

In another aspect, the nuclease may be a homing nuclease. Homing endonucleases comprise 1-5'cel, l-Ceul, l-Pspl, Vl-Sce, l-SceTV, I-Csml, l-Panl, l-Scell, l-Ppol, l-Scelll, l-Crel, l-Tevl, 1-Tev, and I-7evIII. Their recognition sequences are known. See also U.S. Patent No. 5,420,032; U.S. Patent No. 6,833,252.

In certain embodiments, the site-specific enzyme comprises a modified (non-naturally occurring) homing endonuclease (meganuclease). The recognition sequences of homing endonucleases and meganucleases (e.g., l-Scel, l-Ceul, VI-Pspl, Vl-Sce, l-ScelN, l-Csml, l-Panl, l-Scell, l-Ppol, l-Scelll, l-Crel, l-Tevl, l-Tevll, and I-7evIII) are known. See also U.S. Patent No. 5,420,032; U.S. Patent No. 6,833,252. In addition, the DNA-binding specificity of homing endonucleases and large-scale nucleases can be modified to bind non-natural target sites. See, e.g., U.S. Patent Publication No. 20070117128. The DNA-binding domains of homing endonucleases and meganucleases may be altered as a whole in the context of a nuclease (i.e., the nuclease comprises a homologous cleavage domain) or may be fused to a heterologous cleavage domain.

In one embodiment, the site-specific enzyme is a site-specific nucleic acid enzyme selected from or consisting of a group comprising omega, zinc finger, TALE, and CRISPR/Cas9.

### Transfected cells or cell populations

In another aspect, the present application provides a cell (e.g., an immune cell) or cell population obtained using the methods according to the present application.

In another aspect, the present application provides a modified cell (e.g., an immune cell) comprising the isolated nucleic acid molecule of the present application, or the vector of the present application.

In another aspect, the present application provides a cell population which may comprise the cells of the present application and/or their progeny.

For example, the isolated nucleic acid molecule may be integrated into the genome of the cell (e.g., immune cell).

For example, the isolated nucleic acid molecule may be integrated at a target gene in the genome of the cell (e.g., an immune cell). For example, the isolated nucleic acid molecule may be expressed under the regulation of an endogenous regulatory sequence of the target gene. The target gene may be selected from: TRBC, TRAC, PD-1, AAVS1 and CCR5.

The cell may comprise an immune cell, e.g., an immune effector cell. For example, the immune cell may comprise a T lymphocyte, B lymphocyte, NK cell, macrophage, dendritic cell, monocyte, granulocyte, and/or mast cell. For example, the immune cell may comprise a peripheral blood lymphocyte.

For example, the cell may be a primary cell. For example, the cell may comprise an allogeneic cell and/or an autologous cell derived from the subject. For example, in certain cases, the cell is an allogeneic cell derived from other donors. For example, in certain cases, the cell is an autologous cell derived from the subject. For example, in certain cases, the cells comprise autologous cells derived from the subject and allogeneic cells derived from other donors.

For example, the cell may be an activated cell. In certain embodiments, the cells comprise immune cells (e.g., T cells), which may be activated, and the activation may comprise contacting the cells with an activation composition.

For example, the activation composition may comprise anti-CD3 and/or anti-CD28 antibodies.

In certain embodiments, the activation may comprise contacting the immune cell to be modified with the activation composition for no more than about 4 days.

In certain cases, the cell of the present application is an isolated cell.

For example, the present application provides a cell and/or cell line obtained using the methods according to the present application.

In the present application, the cell and/or cell line may have a significantly increased transfection positive ratio (e.g., may also have a significantly increased gene editing knock-in positive ratio (e.g., target cell activation can increase the positive rate of CAR, antibody, cytokine, and/or chemokine knock-in), significantly increased DNA homologous recombination efficiency, and/or significantly increased cell viability). The cell line of the present application may maintain the ability to have a significantly increased transfection positive ratio during the proliferation process of multiple generations (e.g., passaging at least about 5 generations, at least about 10 generations, at least about 15 generations, at least about 20 generations, or longer).

In certain embodiments, the transfection positive ratio is greater than about 2%, 3%, 4%, 5%, 6%, 7%, 8%, 9%, 10%, 15%, 20%, 25%, 30%, 35%, 40%, 45%, 50%, 55%, 60%, 65% or 70%. The positive proportion of the CAR, the IL-15 or a functionally active fragment thereof, and the IL-21 or a functionally active fragment thereof may be measured by determining the number of modified cells comprising them and dividing it by the total number of cells. Integration of the exogenous gene into the cellular genomic DNA may be determined by methods known in the art, such as direct genomic DNA sequencing, differential restriction digestion (if gene editing is adding, removing, or changing restriction enzyme sites), gel electrophoresis, array capillary electrophoresis, MALDI-TOF MS, allele-specific oligonucleotide hybridization, molecular beacons, restriction fragment length polymorphism, primer extension, temperature gradient gel electrophoresis, etc.

In certain embodiments, the cell (e.g., an immune cell, such as a T cell) is capable of expressing an appropriate amount of the IL-15 (or a functionally active fragment thereof) and the IL-21 (or a functionally active fragment thereof). For example, the expression of the IL-15 (or a functionally active fragment thereof) and the IL-21 (or a functionally active fragment thereof) is not excessively high thereby causing safety concerns associated with cytokine expression. Furthermore, the expression of the IL-15 (or functionally active fragment thereof) and the IL-21 (or functionally active fragment thereof) is not too low so as to fail to produce a therapeutic effect.

For example, the cells (e.g., immune cells, such as T cells) or cell populations of the present application may express about 4 pg/mL to about 100 pg/mL (e.g., about 5 pg/mL to about 90 pg/mL, about 5 pg/mL to about 80 pg/mL, about 5 pg/mL to about 70 pg/mL, about 5 pg/mL to about 60 pg/mL, about 5 pg/mL to about 50 pg/mL, about 6 pg/mL to about 50 pg/mL, about 7 pg/ mL to about 45 pg/mL, about 8 pg/mL to about 40 pg/mL, about 9 pg/mL to about 35 pg/mL, about 10 pg/mL to about 30 pg/mL, about 15 pg/mL to about 25 pg/mL or about 20 pg/mL to about 60 pg/mL) or higher amounts of IL-15 or a functionally active fragment thereof. For example, 1x10⁶/mL to 2x10⁶/mL of the cells of the present application may express about 4 pg/mL to about 100 pg/mL (e.g., about 5 pg/mL to about 90 pg/mL, about 5 pg/mL to about 80 pg/mL, about 5 pg/mL to about 70 pg/mL, about 5 pg/mL to about 60 pg/mL, about 5 pg/mL to about 50 pg/mL, about 6 pg/mL to about 50 pg/mL, about 7 pg/ mL to about 45 pg/mL, about 8 pg/mL to about 40 pg/mL, about 9 pg/mL to about 35 pg/mL, about 10 pg/mL to about 30 pg/mL, about 15 pg/mL to about 25 pg/mL or about 20 pg/mL to about 60 pg/mL) or higher amounts of the IL-15 or a functionally active fragment thereof after culture for about 2-4 days (such as 3 days).

For example, the cells (e.g., immune cells, such as T cells) or cell populations of the present application may express about 4 pg/mL to about 300 pg/mL (e.g., about 5 pg/mL to about 290 pg/mL, about 5 pg/mL to about 280 pg/mL, about 5 pg/mL to about 270 pg/mL, about 5 pg/mL to about 260 pg/mL, about 5 pg/ mL to about 250 pg/mL, about 6 pg/mL to about 250 pg/mL, about 7 pg/mL to about 245 pg/mL, about 8 pg/mL to about 240 pg/mL, about 9 pg/mL to about 235 pg/mL, about 10 pg/mL to about 230 pg/mL, about 15 pg/mL to about 225 pg/mL, about 20 pg/ mL to about 220 pg/mL, about 20 pg/mL to about 210 pg/mL, about 20 pg/mL to about 200 pg/mL, about 20 pg/mL to about 180 pg/mL, about 20 pg/mL to about 170 pg/mL, about 20 pg/mL to about 160 pg/mL, about 20 pg/mL to about 150 pg/mL, about 20 pg/mL to about 140 pg/mL, about 20 pg/mL to about 130 pg/mL, about 20 pg/mL to about 120 pg/mL, about 20 pg/mL to about 110 pg/mL, about 20 pg/mL to about 100 pg/mL, about 20 pg/mL to about 90 pg/mL, about 10 pg/mL to about 80 pg/mL, the about 10 pg/mL to about 70 pg/mL, about 10 pg/mL to about 60 pg/mL, about 10 pg/mL to about 50 pg/mL, about 10 pg/mL to about 40 pg/mL, about 10 pg/mL to about 30 pg/mL, or about 5 pg/mL to about 25 pg/mL) or higher amounts of IL-21 or a functionally active fragment thereof. For example, 1x10⁶/mL to 2x10⁶/mL of the cells of the present application may express about 4 pg/mL to about 300 pg/mL (e.g., about 5 pg/mL to about 290 pg/mL, about 5 pg/mL to about 280 pg/mL, about 5 pg/mL to about 270 pg/mL, about 5 pg/mL to about 260 pg/mL, about 5 pg/ mL to about 250 pg/mL, about 6 pg/mL to about 250 pg/mL, about 7 pg/mL to about 245 pg/mL, about 8 pg/mL to about 240 pg/mL, about 9 pg/mL to about 235 pg/mL, about 10 pg/mL to about 230 pg/mL, about 15 pg/mL to about 225 pg/mL, about 20 pg/ mL to about 220 pg/mL, about 20 pg/mL to about 210 pg/mL, about 20 pg/mL to about 200 pg/mL, about 20 pg/mL to about 180 pg/mL, about 20 pg/mL to about 170 pg/mL, about 20 pg/mL to about 160 pg/mL, about 20 pg/mL to about 150 pg/mL, about 20 pg/mL to about 140 pg/mL, about 20 pg/mL to about 130 pg/mL, about 20 pg/mL to about 120 pg/mL, about 20 pg/mL to about 110 pg/mL, about 20 pg/mL to about 100 pg/mL, about 20 pg/mL to about 90 pg/mL, about 10 pg/mL to about 80 pg/mL, the about 10 pg/mL to about 70 pg/mL, about 10 pg/mL to about 60 pg/mL, about 10 pg/mL to about 50 pg/mL, about 10 pg/mL to about 40 pg/mL, about 10 pg/mL to about 30 pg/mL, or about 5 pg/mL to about 25 pg/mL) or higher amounts of IL-21 or a functionally active fragment thereof after culture for about 2-4 days (such as 3 days).

In other embodiments, cell viability after electroporation is at least 20%, 25%, 30%, 35%, 40%, 45%, 50%, 55%, 60%, 65%, 70%, 75%, 80%, or 85%. Cell viability may be measured by methods known in the art. For example, cell counting may be performed before and after electroporation using a cell counter device. In other embodiments, apoptosis may be measured. The introduction of large amounts of nucleic acids is considered to induce apoptosis. The methods described herein are believed to result in less apoptosis than other methods in the art. In certain embodiments, the amount of cells showing apoptosis after electroporation is less than 50%, 45%, 40%, 35%, 30%, 25%, 20%, 15%, 10%, or 5%. Apoptosis refers to a specific process of programmed cell death and may be measured by methods known in the art. For example, apoptosis may be measured by annexin V assay.

### Therapeutic Uses

In another aspect, the present application provides a pharmaceutical composition comprising the nucleic acid molecule of the present application, the vector of the present application, the cell of the present application, and/or the cell population of the present application. The pharmaceutical composition may further comprise a pharmaceutically acceptable adjuvant.

In another aspect, the present application provides the use of the nucleic acid molecule of the present application, the vector of the present application, the cell of the present application, the cell population of the present application, and/or the pharmaceutical composition of the present application for the preparation of a medicament. The medicament may be used for the prevention, treatment, and/or alleviation of cancer. The cancer may include tumor-associated antigen-positive cancer cells. For example, the tumor-associated antigen may be selected from: GPC3, CD19, BCMA, Claudin18.2, and Mesothelin. For example, the tumor-associated antigen may be GPC3. The cancer may be liver cancer or hepatocellular cancer.

In another aspect, the present application provides a method for preventing, treating, and/or alleviating a disease or disorder in a subject, comprising administering an effective amount of the nucleic acid molecule of the present application, the vector of the present application, the cell of the present application, the cell population of the present application, and/or the pharmaceutical composition of the present application to the subject.

In another aspect, the present application provides the nucleic acid molecule of the present application, the vector of the present application, the cell of the present application, the cell population of the present application, and/or the pharmaceutical composition of the present application, for use in preventing, treating, and/or alleviating a disease or condition in a subject.

In the present application, the disease or disorder may be a cancer. The cancer may include tumor-associated antigen-positive cancer cells. The tumor-associated antigen may be selected from: GPC3, CD19, BCMA, Claudin18.2, and Mesothelin. For example, the tumor-associated antigen may be GPC3 or BCMA. The cancer may be liver cancer, hepatocellular cancer, or multiple myeloma.

In certain embodiments, the cells and cell lines produced by the methods described herein are cells and cell lines that provide therapeutic effects after editing the genomic DNA of the cells. Primary cells may be isolated and modified by the methods described herein and used *in vitro* for reintroduction into the subject to be treated. Suitable primary cells comprise peripheral blood mononuclear cells (PBMCs), peripheral blood lymphocytes (PBLs), and other blood cell subpopulations (e.g., but not limited to CD4+ T cells or CD8+ T cells). Other suitable primary cells comprise progenitor cells, such as bone marrow or lymphoid progenitors. Suitable cells also comprise stem cells, such as, for example, embryonic stem cells, induced pluripotent stem cells, hematopoietic stem cells, neuronal stem cells, mesenchymal stem cells, muscle stem cells, and skin stem cells. For example, iPSCs may be obtained *in vitro* from patients with known gene mutations associated, and the mutation may be modified using the methods described herein to a wild-type allele. The modified iPSCs may then be differentiated into dopaminergic neurons and reimplanted into the patient. In another *in vitro* therapeutic application, hematopoietic stem cells may be isolated from patients with known gene mutations and then modified to correct the gene mutation. The HSCs may then be administered back to the patient for therapeutic effects or differentiated into more mature hematopoietic cells in culture before administration to the patient.

Another example of therapeutic use of the methods of the present application is site-specific integration of chimeric antigen receptors (CARs). The term "chimeric antigen receptor" or "CAR" refers to such modified receptors that transplant any specificity onto immune effector cells. These receptors are used to transplant the specificity of monoclonal antibodies onto T cells. The receptors are called chimeras because they are composed of parts from different sources. The most common form of these molecules is a fusion protein consisting of a single-chain variable fragment (scFv) derived from a monoclonal antibody fused with the following: CD3ζ transmembrane and endodomain; CD28 or 41BB intracellular domain; and a combination thereof. Such molecules respond to recognition by their target scFv, leading to signal transduction. An example of such a construct is GPC3-CAR, which recognizes GPC3 (Glypican 3) specifically expressed on the surface of hepatocellular carcinoma cells. When T cells express this molecule, they recognize and kill target cells expressing GPC3 (e.g., hepatoma cells). To target malignant B cells, researchers have redirected T-cell specificity using chimeric immune receptors specific for the B-lineage molecule CD19. The variable parts of the immunoglobulin heavy and light chains are fused to form an scFv by a flexible linker. A signal peptide precedes the scFv to guide the initial protein to the endoplasmic reticulum and subsequent surface expression (which is cleaved). The flexible spacer region allows the scFv to orient in different directions for antigen binding. The transmembrane domain is a typical hydrophobic α-helix, usually derived from the original molecule of the signal transduction intracellular domain that penetrates the cell and transmits the desired signal.

Artificial T cell receptors are being investigated as a treatment for cancer, using a technique called adoptive cell transfer. T cells are removed from the patient and modified to express receptors with specificity for a particular form of cancer. The T cells can then recognize and kill cancer cells when reintroduced into the patient. Modification of T cells derived from donors other than the patient is also under investigation.

These modified CAR T cells may be expanded *ex vivo,* and the expanded CAR T cell population may be infused into the patient. After infusion, the T cells proliferate in the patient's body and, under the guidance of their modified receptors, recognize and kill cancer cells expressing the antigen on their surface. Many existing treatment methods involve the introduction of CAR via viral infection. However, there are always safety concerns when using viral infection in treatment methods. Therefore, the methods described herein are non-viral methods for gene therapy and genome engineering. Previously, it was impossible to transfect immune cells with plasmid DNA, as doing so resulted in severe toxicity to the cells. The inventors of the present application have discovered that by treating the transfection composition prior to cell transfection (for example, reducing the content of host genome DNA), the problems affecting cell viability are overcome, and long DNA fragments (and/or high concentrations) may be transfected into cells while maintaining high levels of viability. Using the methods described herein, CAR may be integrated into specific sites of immune cells. In certain embodiments, the cells are autologous immune cells. Long-term expression of CAR in T cells or natural killer (NK) cells may be used for leukemia treatment or treatment of tumors associated with certain antigens.

In certain embodiments, using the method of the present application, the modified cells (e.g., immune cells, e.g., T-cells) obtained by non-viral transfection (e.g., plasmid electrotransfection) of the isolated nucleic acid molecules of the present application (which encode CARs described in the present application, such as, e.g., GPC3-CAR, IL-15 or a functionally active fragment thereof, and IL-21 or a functionally active fragment thereof) surprisingly have both favorable safety and tumor cell killing ability. For example, in certain embodiments, the isolated nucleic acid molecule, after being transferred into the cell, is inserted site-specifically into a specific locus (e.g., a TRAC locus) of the genomic DNA of the cell such that the exogenous protein (e.g., the CAR, the IL-15 or a functionally active fragment thereof, and the IL-21 or a functionally active fragment thereof) is expressed in a specific manner (e.g., for timing and amount of expression) under the regulation of an endogenous regulatory sequence (e.g., an endogenous promoter) at that insertion site, thereby rendering that modified cell both safe and therapeutically effective.

In certain aspects, the methods described herein involve improved methods for *ex vivo* therapy. Cell populations may be separated from the subject, and cells may be activated using methods known in the art and/or methods described herein, and the genomic DNA of the cells may be modified in a manner that corrects defects or integrates target genes in a site-specific manner. The cell population can then be transplanted into the subject for therapeutic purposes. In certain cases, the separated cell population may contain cell subpopulations sensitive to certain *ex vivo* manipulations (e.g., conventional transfection and/or electroporation methods) or cell subpopulations resistant to conventional transfection and/or electroporation methods or genomic DNA manipulation. It is contemplated that modifying genomic DNA using the methods described herein results in more efficient sequence modification in such populations.

Furthermore, cells and cell lines produced by the methods described herein may be used for drug development and/or reverse genetics research. Such cells and animals may display phenotypes associated with specific mutations or sequence modifications and may be used to screen drugs that interact specifically with the discussed mutations or mutant proteins, or drugs that may be used to treat diseases in affected animals. These cell lines can also provide tools for studying the effects of specific mutations, as the cell lines and their corresponding "modified" cell lines represent "genetically identical" controls, thus providing a powerful tool for repairing disease-specific mutations, drug screening and discovery, and studying disease mechanisms.

The route of administration for the immune cells of the present application can be , for example, intratumoral, intradermal, subcutaneous, intravenous, intralymphatic, and intraperitoneal administration. In certain embodiments, administration is intratumoral or intralymphatic. In certain embodiments, the immune cells are administered directly to the cancer tissue or lymph node.

In certain embodiments, the immune cells are T cells. T cells may be cells that have already contacted the antigen or antigen-presenting cells. For example, APCs may be cultured with tumor antigens specific to a patient's cancer to differentiate them into, for example, CD8-positive cytotoxic T lymphocytes (CTLs) or CD4-positive helper T cells. The established T cells may be administered to an individual with cancer.

The source of the initial T cells is not particularly limited and may be , for example, from peripheral blood of a vertebrate. The initial T cells used may be CD8-positive cells or CD4-positive cells separated from PBMC fractions. In certain embodiments, in terms of inducing CTL efficiency, the initial T cells are CD8-positive cells or CD4-positive cells mixed with other cells and components without being separated from PBMC fractions. For example, when cells from PBMC fractions are cultured in a medium supplemented with serum and tumor antigens, the PBMCs differentiate into dendritic cell precursors. The dendritic cell precursors then bind to the peptide and differentiate into dendritic cells as antigen-presenting cells presenting the peptide/tumor antigen. Antigen-presenting cells stimulate CD8-positive T cells in PBMCs to differentiate into CTLs. Thus, CTLs capable of recognizing the added peptide may be obtained. The obtained CTLs may be isolated and used directly as a cancer vaccine. Alternatively, before being used as a cancer vaccine, they may be further cultured in the presence of leukocyte interleukins (e.g., IL-2), antigen-presenting cells, and tumor antigens. Their route of administration is not particularly limited, and examples comprise intradermal, subcutaneous, intravenous, and intratumoral administration.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

### Examples

Plasmids used in the following examples are described in the following table:

| **Plasmid name** | **Plasmid backbone** | **Encoded Exogenous protein** | **5' Homology arm** | **3' Homology arm** |
|---|---|---|---|---|
| Molecule 2 | pUC57-mini | GPC3 CAR-2A-IL15-2A-IL21 (SEQ ID NO: 19) | SEQ ID NO: 17 (TRAC) | SEQ ID NO: 18 (TRAC) |
| Molecule 3 | pUC57-mini | GPC3 CAR-2A-IL7/15-2A-CCR19/IL21 (SEQ ID NO: 22) | SEQ ID NO: 17 (TRAC) | SEQ ID NO: 18 (TRAC) |

In the GPC3 CAR, the amino acid sequence of the scFv targeting GPC3 is shown in SEQ ID NO: 1. IL17/15 represents IL15 linked with an IL7 signal peptide. CCR19/IL21 represents IL21 linked with a CCR19 signal peptide.

### Example 1 Impact of Host Genomic DNA Content on the Performance of Electrotransfected Immune Cells

Plasmid Molecules 2 were obtained from different sources (referred to as plasmid 2-1, plasmid 2-2, and plasmid 2-3 respectively), and the content of genomic DNA from the microorganisms that produced the plasmid therein was detected. The results (the amount of host genomic DNA contained in 1 mg of total DNA, µg) are shown in the following table:

| Plasmid | Content of Genomic DNA from Microorganism (µg) |
|---|---|
| 2-1 | 168 |
| 2-2 | 5 |
| 2-3 | 0.3 |

Plasmid 2-1, 2-2, and 2-3 were taken. A chemically synthesized sgRNA targeting the human TRAC gene was designed (5'TCAGGGTTCTGGATATCTGT (SEQ ID NO: 25, with 3 sulfur and oxygen methylation modifications at both the 5' and 3' ends of the sgRNA, purchased from GenScript Biotech, Nanjing)). The Cas9 protein was purchased from Yiqiao China (Product No.: 40572-A08B). The sgRNA and Cas9 protein were prepared as ribonucleoprotein RNP for subsequent cell modification.

Plasmids 2-1, 2-2, and 2-3 were separately introduced into 2 donor human T cells activated for two days with Dynabead (from ThermoFisher, comprising anti-CD3 and anti-CD28 antibodies) by electrotransfection, and the prepared RNP was also introduced into the cells. The nucleic acid molecule encoding the Molecule 2 was integrated into the human T cell TRAC gene and could be expressed through the endogenous promoter of the TRAC gene.

The survival rate and total count of viable cells of T - cells transfected with plasmid 2-1, 2-2, or 2-3 were detected by an NC-200 cell counter. The expression level of CAR in the transfected T-cells was detected by flow cytometry and GPC3 protein assay.

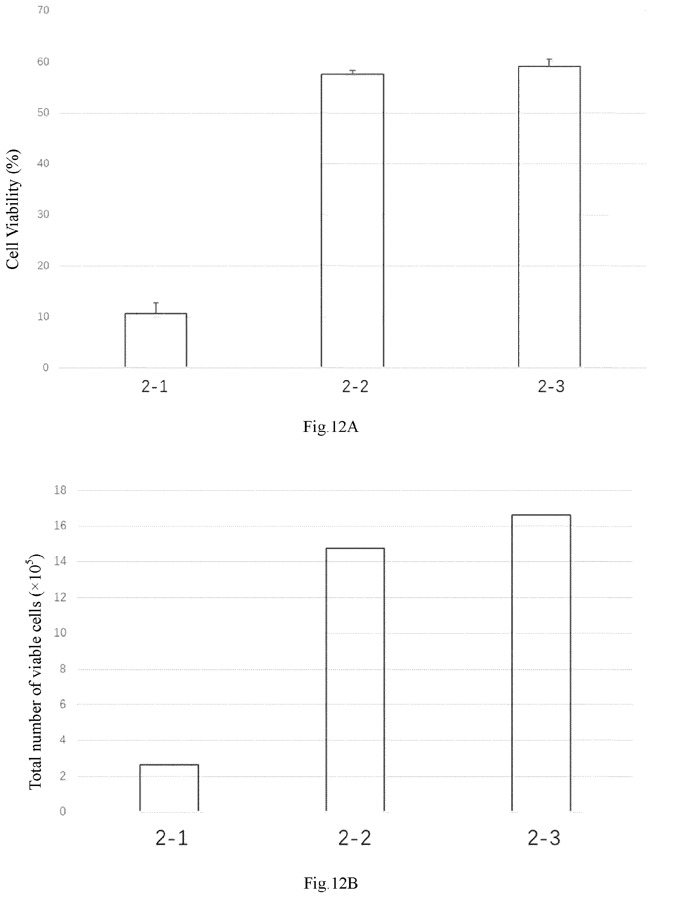

The results are shown in Figs. 12A-12C.

The results of Fig. 12A indicate that, compared to plasmid 2-1 containing more nucleic acid molecules or fragments thereof from the host (e.g., microorganisms), the survival rate of T cells transfected with plasmid 2-2 or 2-3 is significantly increased, with lower host genomic DNA content corresponding to higher survival rates of transfected T cells.

The results of Fig.12B indicate that, compared to plasmid 2-1 containing more host genomic DNA, the total count of viable cells in T cells transfected with plasmid 2-2 or 2-3 is significantly increased, with lower host genomic DNA content corresponding to higher total count of viable cells in T cells.

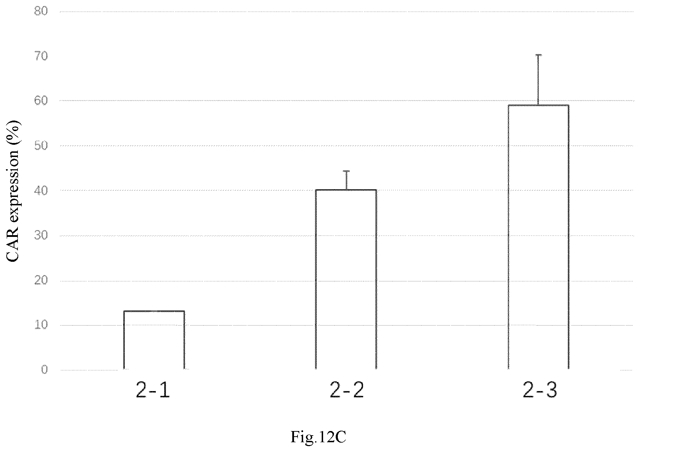

The results of Fig. 12C indicate that, compared to plasmid 2-1 containing more host genomic DNA, the expression level of CAR in T cells transfected with plasmid 2-2 or 2-3 is significantly increased, with lower host genomic DNA content corresponding to higher CAR expression levels in transfected T cells.

### Example 2 Performance of Engineered Immune cells

A chemically synthesized sgRNA targeting the human TRAC gene was designed (5'TCAGGGTTCTGGATATCTGT (SEQ ID NO: 25, with 3 sulfur and oxygen methylation modifications at both the 5' and 3' ends of the sgRNA, purchased from GenScript Biotech, Nanjing)). The Cas9 protein was purchased from Yiqiao China (Product No.: 40572-A08B). The sgRNA and Cas9 protein were prepared as ribonucleoprotein RNP for subsequent cell modification.

Plasmid Molecule 2 were separately introduced into human T cells activated for 2-3 days with Dynabead (from ThermoFisher, comprising anti-CD3 and anti-CD28 antibodies) by electrotransfection, and the prepared RNP was also introduced into the cells. The nucleic acid molecule encoding Molecule 2 was integrated into the human T cell TRAC gene and could be expressed through the endogenous promoter of the TRAC gene. The obtained cells are Molecule 2 cells. The obtained cells were detected.

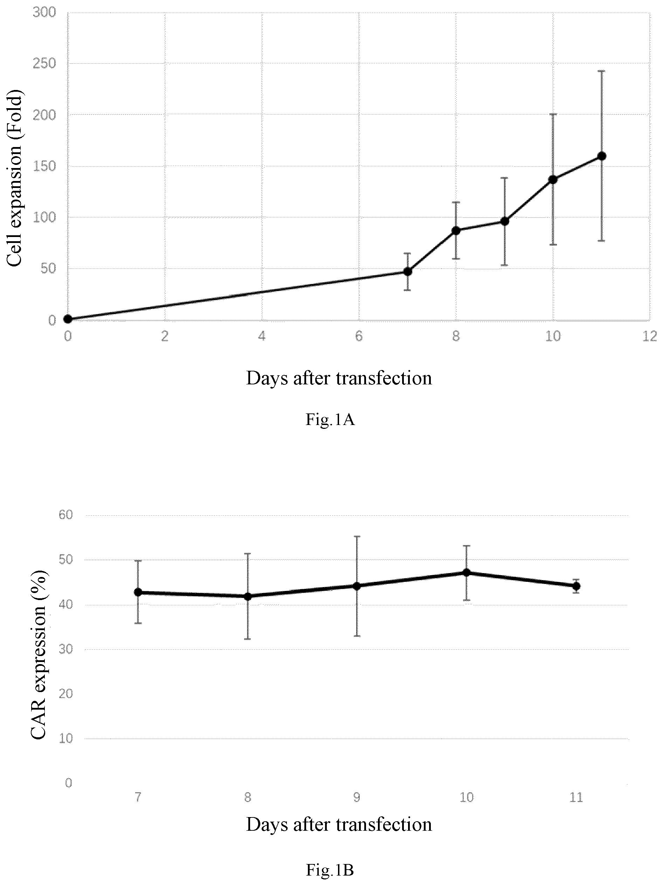

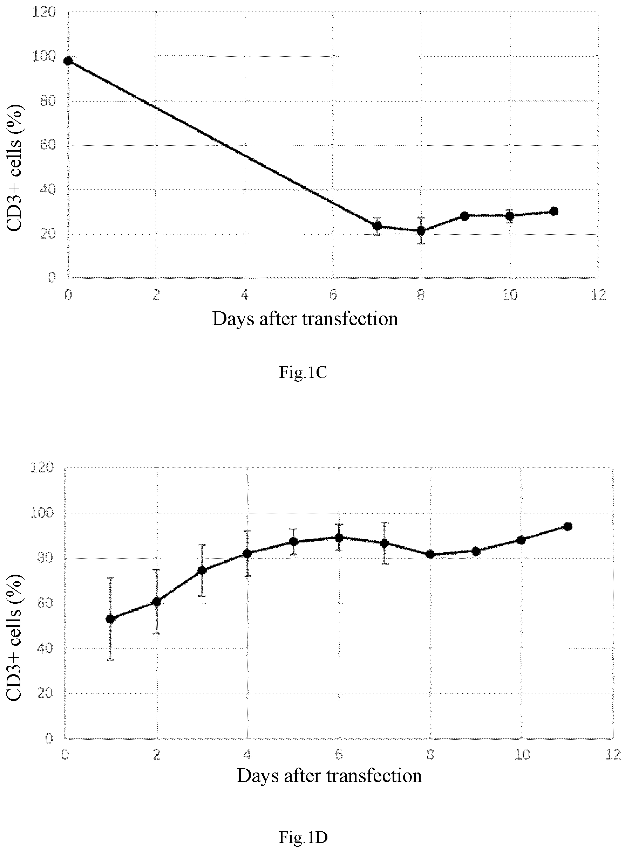

The results are shown in Figs. 1A-1D. Fig. 1A shows that after 7-day expansion of the Molecule 2 cells in Xvivo-15 medium (containing 5% SR + 100 IU/ml rhIL-2, 10 ng/mL rhIL-7 and 5 ng/mL rhIL-15), the cell number increased by at least 50-fold. Fig. 1B shows that the Molecule 2 cells have a stable positive expression rate of CAR molecules. Fig. 1C shows that after TRAC knockout, CD3 in the Molecule 2 cells was effectively knocked-out. Fig. 1D shows that there is a relatively high proportion of CD8-positive cells in the Molecule 2 cells.

### Example 3 Performance of Engineered Immune Cells after Cryopreservation and Recovery

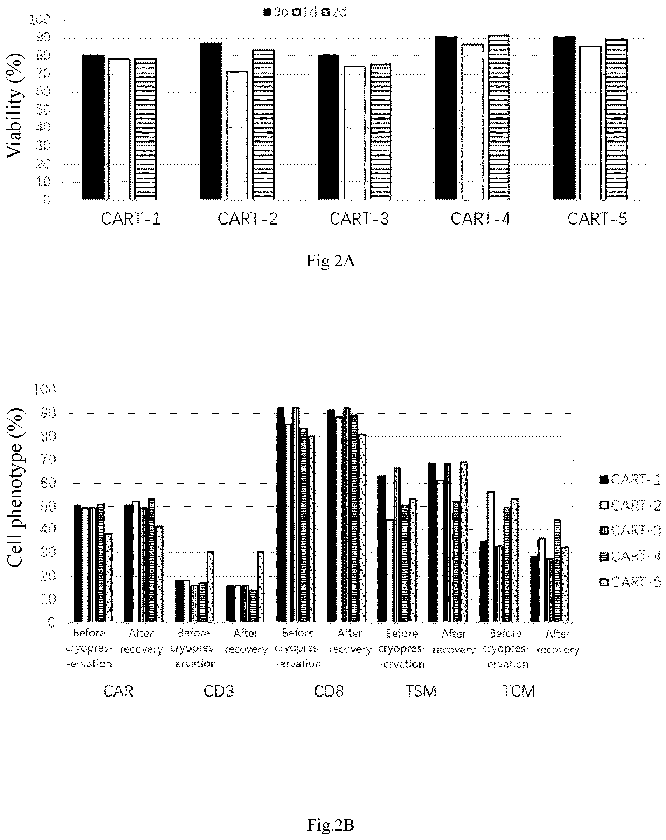

The viability, cell phenotypic characteristics, and tumor-cell killing ability of the Molecule 2 cells described in Example 1 after cryopreservation and recovery were measured. The results are shown in Figs. 2A-2C.

Fig. 2A shows the cell viability of 5 different batches of Molecule 2 cells (referred to as CART-1, CART-2, CART-3, CART-4, and CART-5, respectively) after cryopreservation and recovery. The Molecule 2 cells were cryopreserved using CryoStor CS10 7 days after electrotransfection. The cell viability was measured with NC-200 on Day 0, Day 1, and Day 2 after cryopreservation and recovery. The results indicate that the Molecule 2 cells maintain a cell viability of at least 70% within 0-2 days after cryopreservation and recovery.

In addition, the proportion of CAR-positive cells and memory cells before and after recovery were detected, and the results are shown in Fig. 2B. As can be seen from the results in Fig. 2B, the recovery process does not significantly alter the CAR-positive rate of the cells or the proportion of memory cells, wherein TSM refers to CD62L+/CD45RO- cells, and TCM refers to CD62L+/CD45RO+ cells.

Furthermore, the killing ability of the cells after recovery was detected. Briefly, the Molecule 2 cells were co-incubated with Huh-7 cells. After incubation, the mortality rate of target cells killed by T cells was calculated. Mortality rate = N_{D}/ N_{L}*100%. Here, N_{L} is the number of target cells collected in 20 seconds during FACS detection, and N_{D} is the number of dead target cells among them.

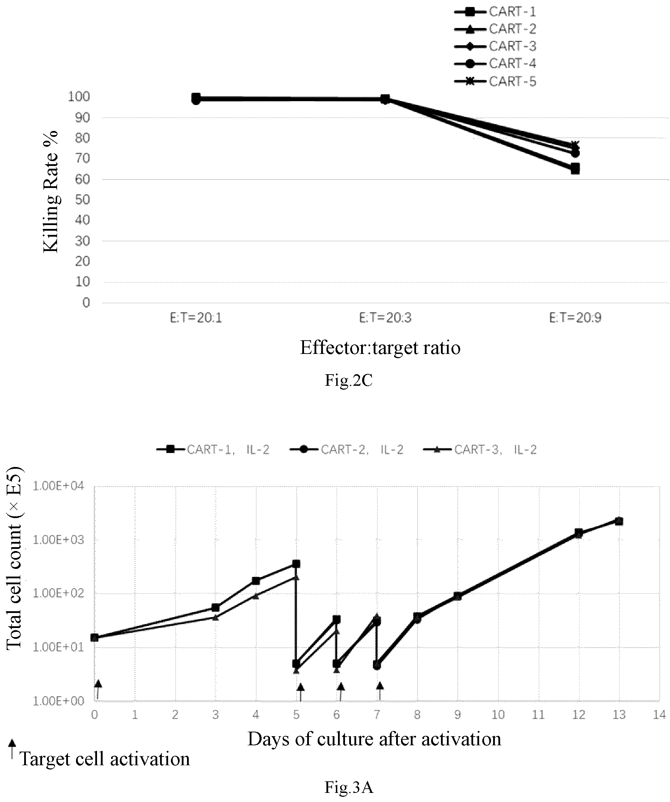

The mortality rate results are shown in Fig. 2C. Fig. 2C shows the mortality results of target cells Huh-7, with the horizontal axis E:T representing the ratio of effector cells (Molecule 2 cells) to target cells (tumor cells). The results in Fig. 2C demonstrate that the cells after recovery still retain killing capacity against target cells.

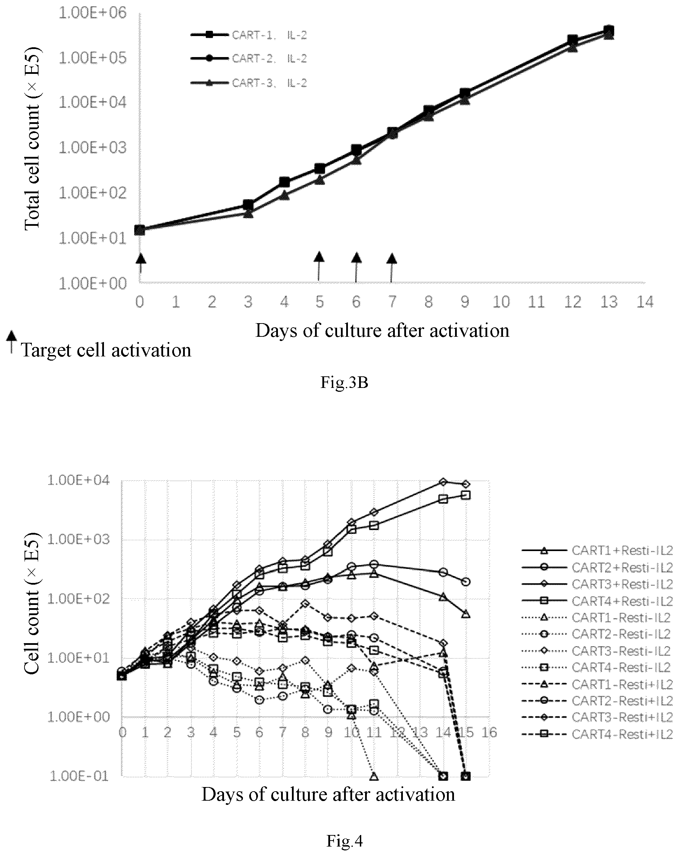

Moreover, the expansion ability of the Molecule 2 cells described in Example 1 after cryopreservation and recovery was also detected, and the results are shown in Figs. 3A and 3B. Briefly, the cells were cultured in Xvivo-15 medium (containing 5% SR + 100 IU/ml rhIL-2). On day 0, day 5, day 6, and day 7, Huh-7 target cells were co-incubated with the Molecule 2 cells to activate them. As can be seen from the results in Figs. 3A and 3B, after recovery, Molecule 2 cells can still be continuously activated and expanded. Specifically, Fig. 3A shows the number of cells after expansion of a portion of the cells (approximately 5x10⁵cells) taken before each activation, while Fig. 3B shows the total number of expanded cells calculated.

### Example 4 Expression of Cytokines by Engineered Immune Cells

The impact of the expression of IL-21 and/or IL-15 by the Molecule 2 cells on their survival was further investigated. The results are shown in Fig. 4. Four different batches of cells, referred to as CART1, CART2, CART3, and CART4, were detected. "+Resti" represents co-incubation with target cells (Huh-7 cells expressing GPC3), "-Resti" represents no co-incubation with target cells, "+IL2" represents the addition of extra recombinant human IL-2 (rhIL-2) to the culture medium, and "-IL2" represents no addition of extra IL-2 to the culture medium.

As shown in the results of Fig. 4, when Molecule 2 cells were cultured *in vitro* in a medium without rhIL-2 and without co-incubation with target cells, they died rapidly. When the cells were cultured *in vitro* in a medium added with 100 IU/ml rhIL-2 but without co-incubation with target cells, the expansion ability and survival rate of the cells was slightly increased. However, when the cells were co-incubated with target cells, even without the addition of extra rhIL-2 to the medium, the expansion ability and survival rate of the cells was still increased.

The above results indicate that when the specific nucleic acid molecule of the present invention is introduced and integrated into the genome of T cells, the prepared CAR-T cells not only express a CAR targeting the tumor-associated antigen but also express exogenous IL-21 and IL-15. Unexpectedly, compared with conventional CAR-T cells, in the presence of target cells, the CAR-T cells of the present invention can possess excellent expansion ability and survival rate without the addition of IL-2. On Day 14, in the presence of only target cells and no addition of exogenous IL-2, the number of CAR-T cells of the present invention was approximately 1-2 orders of magnitude higher than that under the condition of the absence of target cells but with the addition of exogenous IL-2.

In addition, although the CAR-T cells of the present invention express IL-21 and IL-15, in the absence of target cells, the expansion ability of the CAR-T cells of the present invention is limited, thus ensuring safety and suggesting no or a very low or tumor genic risk.

### Example 5 Engineered Immune Cells Effectively Kill Tumor Cells In Vitro

A subcutaneous tumor mouse model was established by subcutaneously injecting 1x10⁷ HepG-2 cells (purchased from PharmaLegacy, Shanghai) into NCG mice (purchased from Gempharmatech Co., Ltd).

About 14 days later, when the tumor grows to a volume of about 100 mm³, 100 µl of the Molecule 2 T cells at a concentration of 1x10⁸/mL were injected into the tail vein of 3 mice among the mouse models, and these mice are referred to as the treatment group. Another 2 model mice were injected with 100 µl of control T cells at a concentration of 1x10⁸/mL via tail veins (the control T cells express a CAR targeting CD19, wherein the scFv domain targeting CD19 was derived from the previously reported antibody FMC63). These mice were referred to as the irrelevant CART control group. In addition, 2 model mice were injected with only the solvent and were referred to as the vehicle control group.

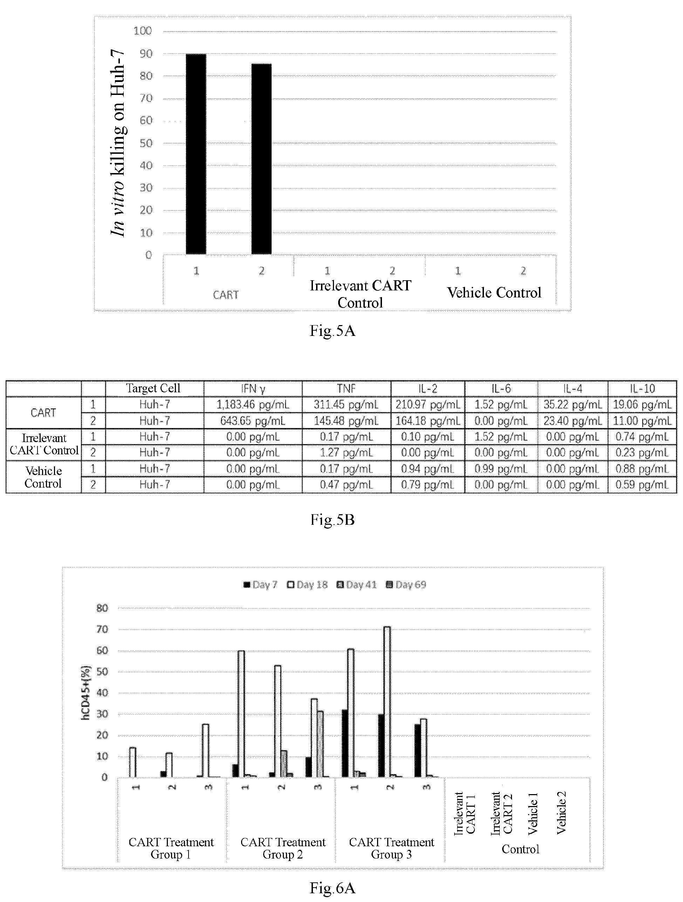

18 days after the injection of the cells or the solvent, peripheral blood cells were collected from 2 mice in each group and co-incubated with Huh-7 target cells *in vitro.* The results are shown in Fig. 5A. The cells derived from the treatment group exhibited a significant killing effect on Huh-7 target cells, while the cells derived from the irrelevant CART control group or the vehicle control group exhibited no killing effect on Huh-7 target cells.

Furthermore, the levels of various cytokines in the cell culture supernatant after co-incubation were measured. The results are shown in Fig. 5B. After the co-incubation, the expression levels of the detected cytokines in the cells derived from the treatment group were upregulated. Conversely, after the co-incubation, the content of the detected cytokines in the culture supernatant of the cells derived from the irrelevant CART control group or the vehicle control group was almost zero.

### Example 6 Engineered Immune Cells Effectively Inhibit Tumor Growth In Vivo

A subcutaneous tumor mouse model was established by subcutaneously injecting 1x10⁷ HepG-2 cells (purchased from PharmaLegacy, Shanghai) into NCG mice (purchased from Gempharmatech Co., Ltd).

About 14 days later, when the tumor grows to a volume of about 100 mm³, 100 µl of the Molecule 2 T cells at a concentration of 1x10⁸/mL were injected into the tail vein of 3 mice among the mouse models, and these mice are referred to as the treatment group (3 batches, 3 mice per batch, 9 mice in total). Another 2 model mice were injected with 100 µl of control T cells (CD19 CART cells) at a concentration of 1x10⁸/mL via tail veins. These mice were referred to as the irrelevant CART control group. In addition, 2 model mice were injected with only the solvent and were referred to as the vehicle control group.

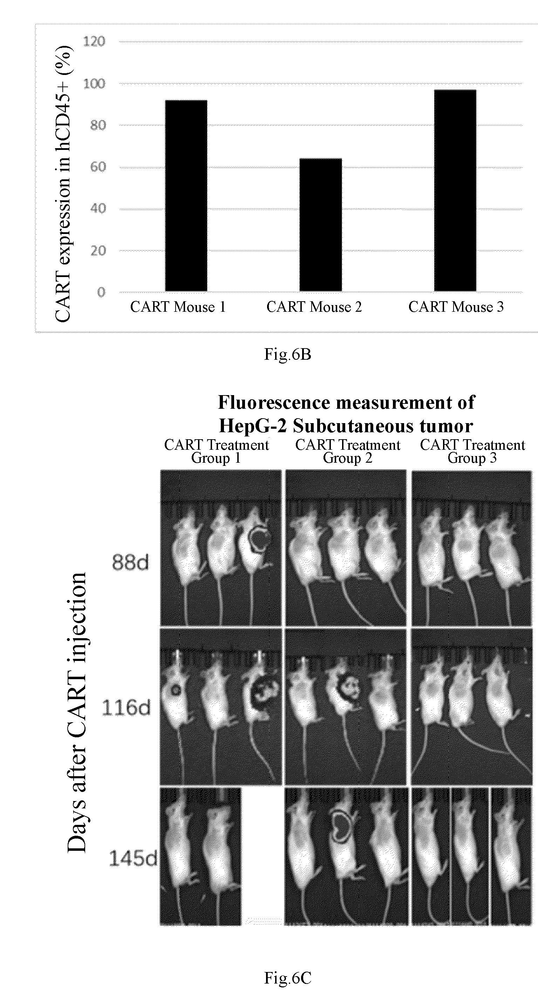

The proportion of human cells (hCD45⁺) in the peripheral blood of mice in each group was detected to evaluate the effectiveness of transplantation, and the results are shown in Fig. 6A. As shown in Fig. 6A, in the 3 treatment groups, the proportion of human cells was low on Day 7, reached a peak on Day 18, and then declined from Day 41 to Day 69. Among them, the CAR expression of human cells in the peripheral blood of 3 mice in the third treatment group was detected, and the results are shown in Fig. 6B. It can be seen that most of the human cells (hCD45⁺) derived from the treatment group expressed CAR.

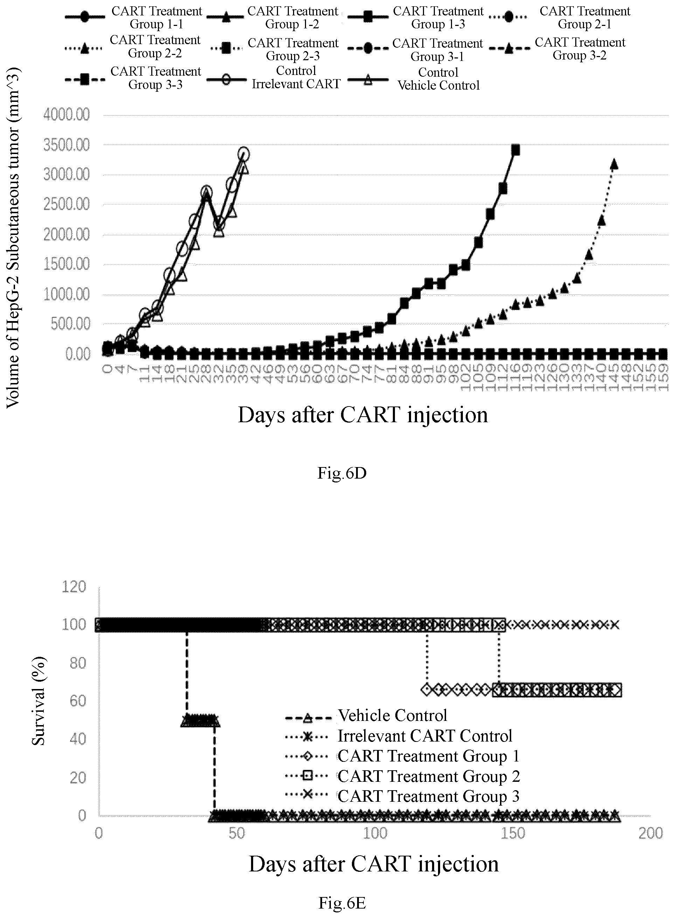

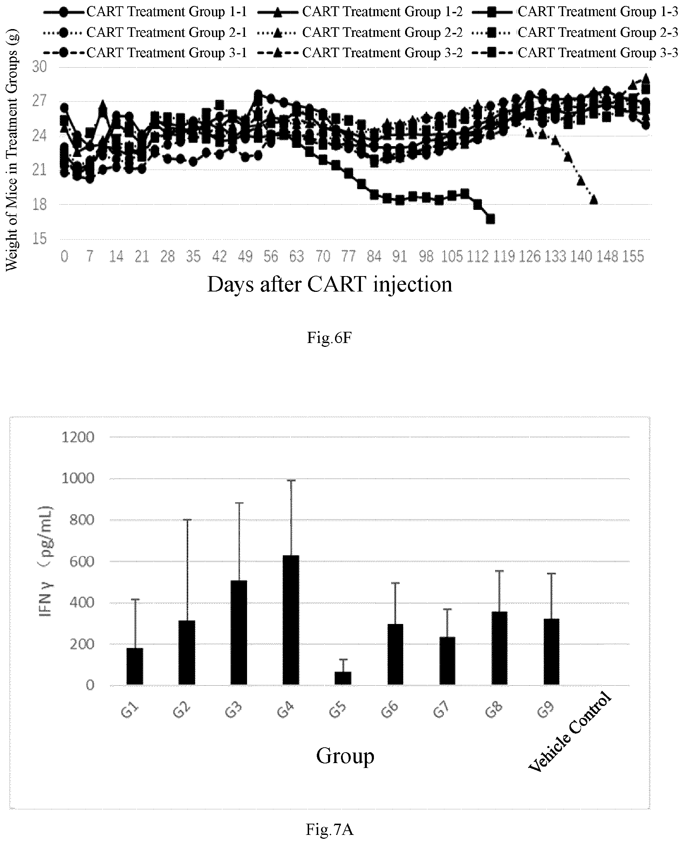

In addition, the tumor growth (Figs. 6C and 6D), mouse survival rate (Fig. 6E), and mouse body weight changes (Fig. 6F) of the treatment group, irrelevant CART control group, and vehicle control group were detected.

As shown in the results of Fig. 6D, in the mice of the irrelevant CART control group and the vehicle control group, the tumor volume increased rapidly. In the 9 mice of the treatment group, the tumor volume had no significant difference from that of the control group on Day 7 after cell injection, but from Day 11, the growth rate of the tumor volume decreased rapidly. In 7 of the 9 mice in the treatment group, the tumor volume shrank to zero on Day 28 and remained zero until at least Day 159 after cell injection.

Fluorescence detection was carried out on Day 88, Day 116, and Day 145 after cell injection. As shown in the results of Fig. 6C, 1 mouse in each of the first treatment group and the second treatment group had tumor relapse and died on Day 145 (first treatment group) or showed a strong fluorescence value (second treatment group), but the other 7 mice in the treatment groups showed a fluorescence value of zero on Day 145. Surprisingly, one mouse of the first treatment group showed a low fluorescence value on Day 116 (though the visible tumor volume was zero), but the fluorescence value went to zero on Day 145, suggesting that the tumor relapse activated memory T cells which are able to eliminate the recurrent tumor cells.

As shown in the results of Fig. 6E, the mice in the irrelevant CART control group and the vehicle control group died on Day 41 after injection. In contrast, the survival rates of the mice in the first treatment group, the second treatment group, and the third treatment group were 66%, 66%, and 100% respectively on Day 148 after cell injection.

As shown in the results of Fig. 6F, among the 9 mice in the treatment groups, except for the 2 dead mice, the body weights of the other 7 mice increased steadily.

### Example 7 Expression of Cytokines by Engineered Immune Cells In Vivo

A subcutaneous tumor mouse model was established by subcutaneously injecting 1x10⁷ HepG-2 cells (purchased from PharmaLegacy, Shanghai) into NCG mice (purchased from Gempharmatech Co., Ltd).

About 14 days later, when the tumor grows to a volume of about 100 mm³, 100 µl of the Molecule 2 cells at a concentration of 1x10⁸/mL were injected into the tail vein of 36 mice (divided into 9 groups, G1-G9, with 4 mice in each group) among the mouse models, and these mice are referred to as the treatment group. In addition, 4 model mice were injected with only the solvent and were referred to as the vehicle control group.

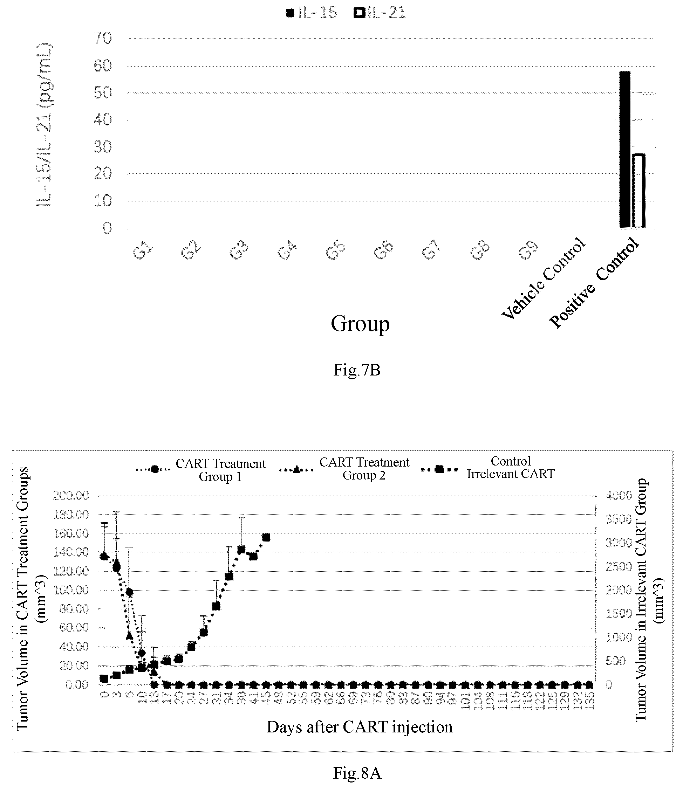

The expression of IFN-γ, IL-15, and IL-21 in the peripheral blood of mice in each group were detected respectively. The results are shown in Figs. 7A and 7B. On Day 10 after injection of cells, the peripheral blood of the mice was collected, and the contents of IFN-γ, IL-15, and IL-21 in each peripheral blood sample were detected by ELISA. As shown in the results of Fig. 7A, the expression of IFN-γ could be detected in the peripheral blood of each mouse, but was not expressed in the vehicle control group. As shown in the results of Fig. 7B, except for the positive control group (derived from a kit known to contain IL-15 and IL-21 in the supernatant), no expression of IL-15 or IL-21 could be detected in the peripheral blood of mice in each group, indicating that the engineered immune cells of the present application do not express a large amount of IL-15 or IL-21 *in vivo* and thus have high safety.

### Example 8 In Vivo Tumor-inhibiting Effect of Engineered Immune Cells

Hepatocellular cancer PDX model mice LI1035-R16P8 (Crown Bioscience (Taicang) Co., Ltd.) were used. On Day 37 after tumor inoculation, the subcutaneous tumor volume was approximately 100 mm³-150 mm³. The mice were randomly divided into 3 groups, with 4 mice in each group. 2 groups of mice were injected with 100 µl of Molecule 2 cells at a concentration of 1×10⁸/mL via tail veins (2 different batches), and these mice were referred to as the first treatment group and the second treatment group. Another group of 4 model mice were injected with 100 µl of control T cells (expressing CAR targeting CD19, i.e., CD19-CART) at a concentration of 1×10⁸/mL via tail veins, and this group was referred to as the irrelevant CART control group. The results are shown in Fig. 8A. As shown in Fig. 8A, the tumor volume of mice in the irrelevant CART control group increased rapidly, and the mouse died at approximately Day 45. In the mice of the first treatment group and the second treatment group, the tumor volume was controlled from Day 3. On Day17, the tumor volume shrank to zero and remained so until Day 135.

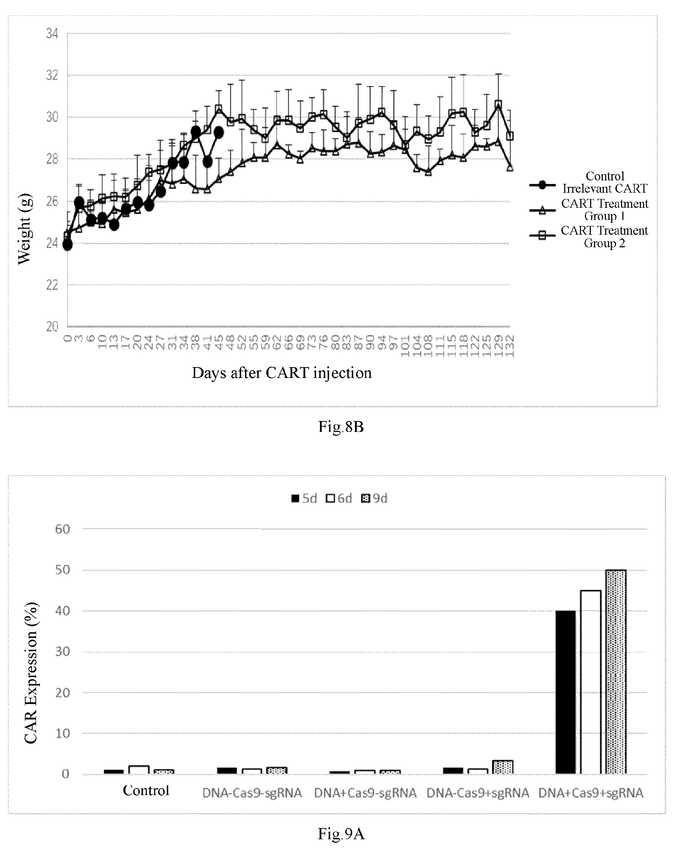

In addition, the body weight of the mice were measured, and the results are shown in Fig. 8B. It can be seen that the body weight of the mice in each group increased steadily.

### Example 9 Immune Cells without Integrated Exogenous Nucleic Acid Sequences Cannot Kill Target Cells

A chemically synthesized sgRNA targeting the human TRAC gene was designed (5'TCAGGGTTCTGGATATCTGT (SEQ ID NO: 25, with 3 sulfur and oxygen methylation modifications at both the 5' and 3' ends of the sgRNA, purchased from GenScript Biotech, Nanjing)). The Cas9 protein was purchased from Yiqiao China (Product No.: 40572-A08B). The sgRNA and Cas9 protein were prepared as ribonucleoprotein RNP for subsequent cell modification.

Plasmid Molecule 2 was introduced into human T-cells that had been activated for two days with Dynabead (from Thermofisher, containing anti-CD3 antibody and anti-CD28 antibody) by electrotransfection, without introduction of Cas9 or sgRNA, thereby obtaining cells in the DNA-Cas9-sgRNA group (i.e., only containing the plasmid).

Plasmid Molecule 2 was introduced into human T-cells that had been activated for two days with Dynabead (from Thermofisher, containing anti-CD3 antibody and anti-CD28 antibody), and Cas9 was simultaneously introduced into these cells, thereby obtaining cells in the DNA+Cas9-sgRNA group (i.e., containing the plasmid and Cas9, but not containing sgRNA).

Plasmid Molecule 2 was introduced into human T-cells that had been activated for two days with Dynabead (from Thermofisher, containing anti-CD3 antibody and anti-CD28 antibody), and sgRNA was simultaneously introduced into these cells, thereby obtaining cells in the DNA-Cas9+sgRNA group (i.e., containing the plasmid and sgRNA, but not containing Cas9).

Plasmid Molecule 2 was introduced into human T-cells that had been activated for two days with Dynabead (from Thermofisher, containing anti-CD3 antibody and anti-CD28 antibody), and the prepared RNP was simultaneously introduced into these cells, thereby obtaining cells in the DNA+Cas9+sgRNA group.

On Day 5, Day 6 and Day 9 after electrotransfection, the expression of CAR in each group of cells was measured respectively. The results are shown in Fig. 9A. It can be seen that the proportion of CAR-positive cells was relatively high only in those cells transfected with plasmid, Cas9, and sgRNA simultaneously. This indicates that the plasmid cannot stably express exogenous nucleic acid molecules in long-term when it is not integrated into the T-cell genome.

In addition, the killing ability of each group of cells was also measured. Briefly, cells in each group were co-incubated with Huh-7 cells. After incubation, the mortality rate of target cells killed by T cells was calculated. Mortality rate = N_{D}/ N_{L}* 100%. Here, N_{L} is the number of target cells collected in 20 seconds during FACS detection, and N_{D} is the number of dead target cells among them.

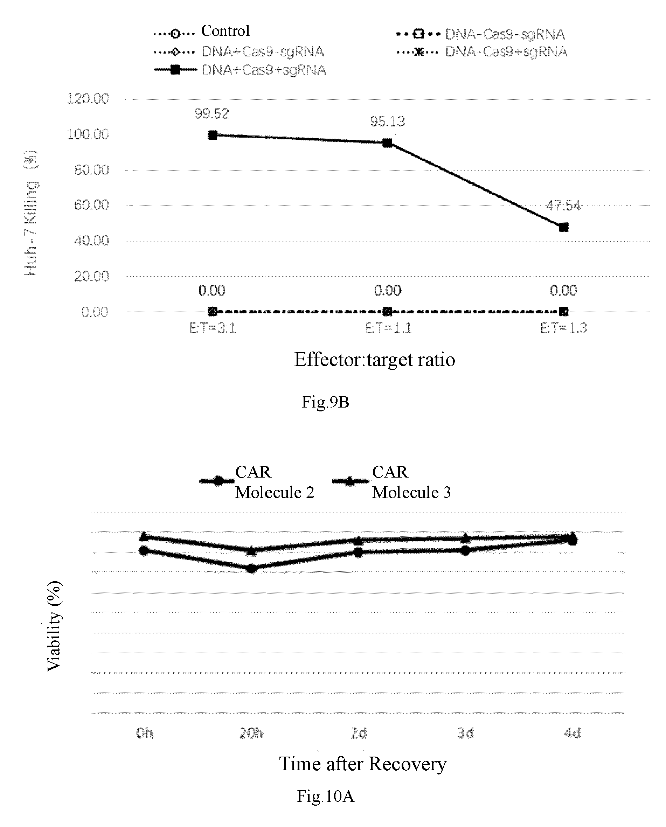

The mortality rate results are shown in Fig. 9B. Fig. 9B shows the mortality rate results of the target cells Huh-7. The abscissa E:T represents the ratio of effector cells to target tumor cells. As shown in Fig. 9B, only those cells transfected with the plasmid, Cas9, and sgRNA simultaneously exhibited a good killing effect on the target cells.

### Example 10 Performance of Engineered Immune Cells

A chemically synthesized sgRNA targeting the human TRAC gene was designed (5'TCAGGGTTCTGGATATCTGT (SEQ ID NO: 25, with 3 sulfur and oxygen methylation modifications at both the 5' and 3' ends of the sgRNA, purchased from GenScript Biotech, Nanjing)). The Cas9 protein was purchased from Yiqiao China (Product No.: 40572-A08B). The sgRNA and Cas9 protein were prepared as ribonucleoprotein RNP for subsequent cell modification.

Plasmid Molecule 3 was introduced into human T-cells that had been activated for 3 days with Dynabead (from Thermofisher, containing anti-CD3 antibody and anti-CD28 antibody) by electrotransfection, and the prepared RNP was simultaneously introduced into these cells. The nucleic acid molecule encoding Molecule 3 was integrated into the human T cell TRAC gene and could be expressed through the endogenous promoter of the TRAC gene. The obtained cells are Molecule 3 cells. The obtained cells were detected.

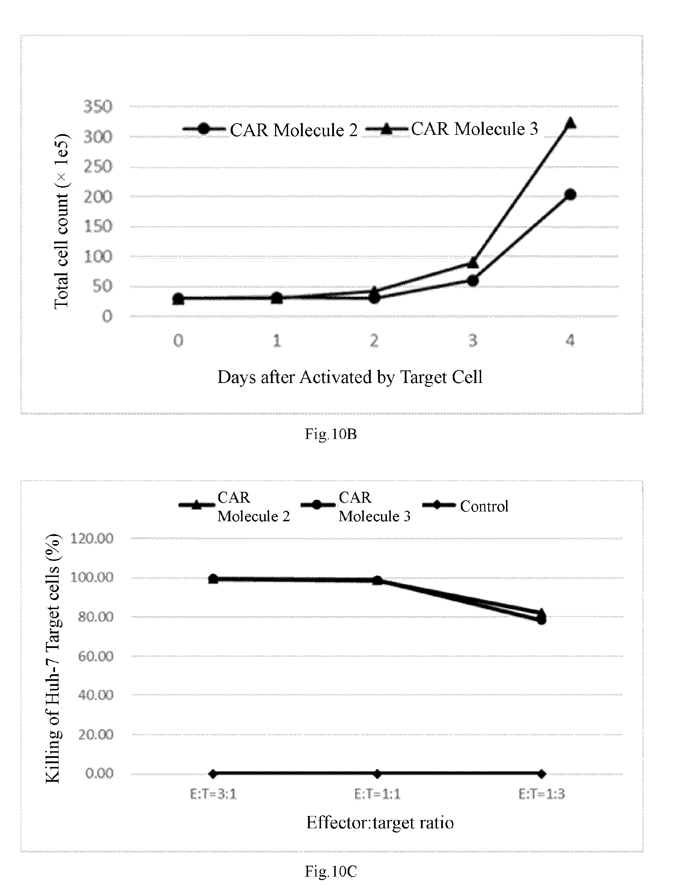

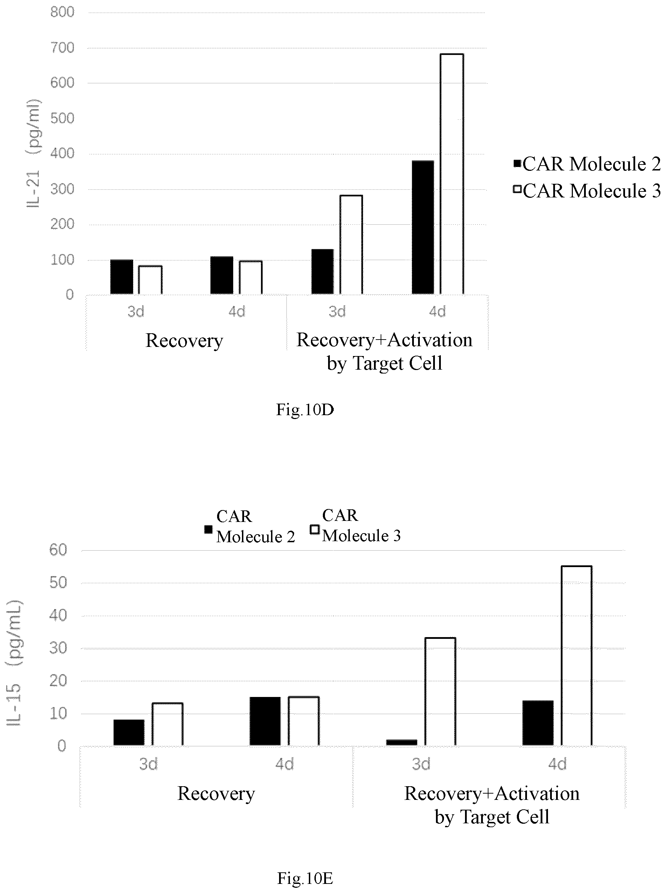

The performance of the Molecule 2 cells and Molecule 3 cells was compared in terms of viability after cryopreservation and recovery, *in-vitro* tumor cell killing ability, expansion ability, and cytokine expression. The results are shown in Fig. 10.

Fig. 10A shows that the viabilities of Molecule 2 cells and Molecule 3 cells after cryopreservation and recovery were comparable.

Fig. 10B shows that when co-incubated with the target cell Huh-7 and activated by this target cell, the expansion abilities of Molecule 2 cells and Molecule 3 cells were comparable.

Fig. 10C shows that when co-incubated with the target cell Huh-7, the killing abilities of Molecule 2 cells and Molecule 3 cells against the target cell Huh-7 were comparable.

In addition, the abilities of Molecule 2 cells and Molecule 3 cells to express cytokines IL-15 and IL-21 were detected. The results are shown in Fig. 10D and Fig. 10E respectively. Without co-incubation with target cells, both Molecule 2 cells and Molecule 3 cells can express IL-21, and the levels of IL-21 expression were similar. However, after co-incubated with and activation by the Huh-7 target cells, although the levels of IL-21 expression in both Molecule 2 cells and Molecule 3 cells increased, the level of IL-21 expression in Molecule 3 cells increased significantly more (Fig. 10D). Without co-incubation with target cells, both Molecule 2 cells and Molecule 3 cells can express IL-15, and the levels of IL-15 expression were similar. However, after co-incubated with and activation by the Huh-7 target cells, although the levels of IL-15 expression in both Molecule 2 cells and Molecule 3 cells increased, the level of IL-15 expression in Molecule 3 cells increased significantly more (Fig. 10E).

### Example 11 Comparison of In Vivo Tumor-inhibiting Effects of Engineered Immune Cells

Furthermore, the *in vivo* tumor-inhibiting abilities of the Molecule 2 cells and Molecule 3 cells described in Example 10 were compared.

A subcutaneous tumor mouse model was established by subcutaneously injecting 1x10⁷ HepG-2 cells (purchased from PharmaLegacy, Shanghai) into NCG mice (purchased from Gempharmatech Co., Ltd).

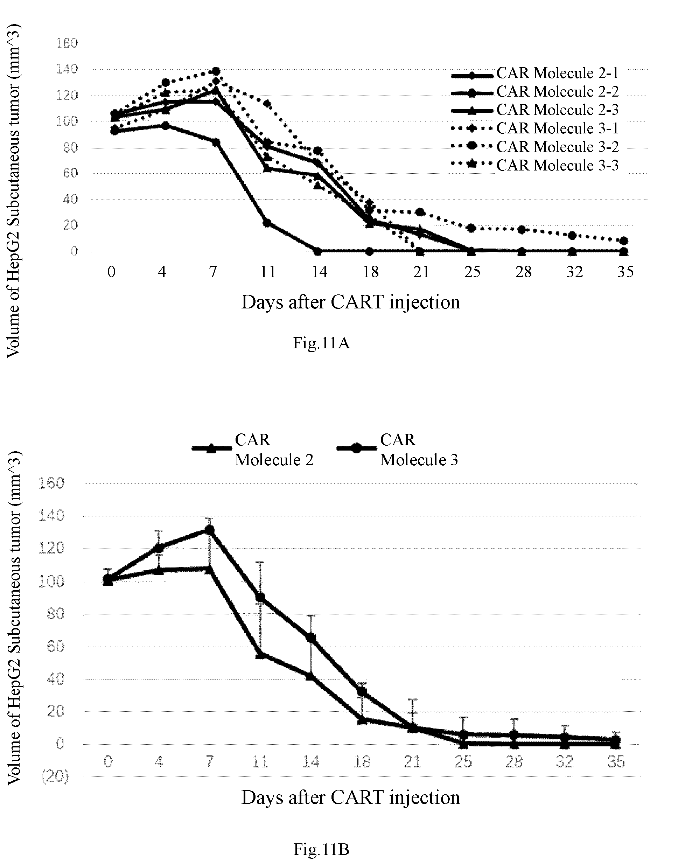

About 14 days later, when the tumor grows to a volume of about 100 mm³, 100 µl of the Molecule 2 T cells at a concentration of 1x10⁸/mL were injected into the tail vein of 3 mice among the mouse models, and these mice are referred to as the CAR Molecule 2 cell treatment group. Another 3 mice were injected with 100 µl of Molecule 3 cells at a concentration of 1×10⁸/mL via the tail vein, and these mice were referred to as the CAR Molecule 3 cell treatment group. The results are shown in Fig. 11A and Fig. 11B (the fitted data lines for groups of each type of cell). It can be seen that the *in vivo* tumor-inhibiting effects of the CAR Molecule 2 cell treatment group and the CAR Molecule 3 cell treatment group were comparable.

### Example 12 In Vivo Tumor-inhibiting Effect under the Regulation of Exogenous Promoters

Using the same plasmid backbones and homologous arms as plasmid Molecules 2 and 3, plasmids containing the following exogenous protein-coding genes were constructed:
BCMA CAR-2A-IL15 (SEQ ID NO: 27);
BCMA CAR-2A-IL15-2A-IL21 (SEQ ID NO: 28);
PGK-BCMA CAR-2A-IL15-2A-IL21 (SEQ ID NO: 29);
PGK-GPC3 CAR-2A-IL15-2A-IL21 (SEQ ID NO: 30);
GPC3 CAR-2A-IL15-2A-IL21,
wherein BCMA CAR is a chimeric antigen receptor targeting BCMA; PGK is the exogenous PGK promoter (SEQ ID NO: 26), and other elements are as defined above.

The plasmids were transfected into T-cells by electrotransfection using the experimental methods described above to prepare the corresponding CAR-T cells. The *in vivo* tumor-inhibiting experimental method was the same as that in Example 11. The inhibitory effects of each CAR-T cell on RPMI-8226 multiple myeloma and HepG2 liver cancer were tested respectively.

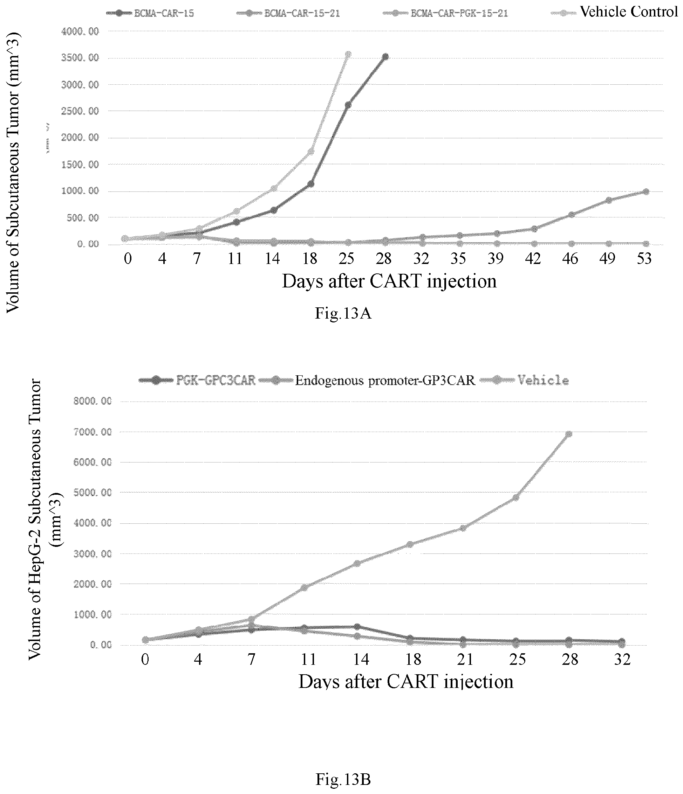

The results are shown in Figs. 13A and 13B. In the *in vivo* tumor-inhibiting experiment for RPMI-8226 multiple myeloma, the CAR-T cells expressing IL-15 and IL-21 under the regulation of the exogenous PGK promoter (PGK-BCMA CAR-2A-IL15-2A-IL21) showed the best tumor-killing effect, and their tumor-inhibiting effect was superior to that of the CAR-T cells expressing only IL15 or both IL15 and IL21 under the regulation of the endogenous promoter. In the *in vivo* tumor-inhibiting experiment for HepG2 liver cancer, the CAR-T cells expressing GPC3 CAR, IL-15, and IL-21 under the regulation of the exogenous PGK promoter (PGK-GPC3 CAR-2A-IL15-2A-IL21) showed basically the same tumor-killing effect as that of those under the regulation of the endogenous promoter. The results suggest that the exogenous promoter can be used to express the nucleic acid molecules of the present invention.

The above detailed description is provided for explanation and example purposes and is not intended to limit the scope of the appended claims. Variations of the embodiments listed in the present application are apparent to those skilled in the art and are reserved within the scope of the appended claims and their equivalents.

## Claims

1. An isolated nucleic acid molecule comprising: a nucleic acid sequence encoding a chimeric antigen receptor (CAR); a nucleic acid sequence encoding IL-15 or a functionally active fragment thereof; and a nucleic acid sequence encoding IL-21 or a functionally active fragment thereof.

2. The nucleic acid molecule according to claim 1, wherein the nucleic acid molecule further comprises a nucleic acid sequence encoding an additional exogenous protein selected from the group consisting of: IL-2 or a functionally active fragment thereof, IL-7 or a functionally active fragment thereof, a cytokine, BiTE, and a combination thereof.

3. The nucleic acid molecule according to claim 1 or 2, which comprises tandem expression units, wherein the expression units comprise:
(E1) a first expression unit for expressing the chimeric antigen receptor;
(E2) a second expression unit for expressing IL-15 or a functionally active fragment thereof or a fusion protein thereof;
(E3) a third expression unit for expressing IL-21 or a functionally active fragment thereof or a fusion protein thereof; and
(E4) optionally, a fourth expression unit for expressing an additional exogenous protein, wherein the positions of the first, second, third, and fourth expression units are randomly interchangeable.

4. The nucleic acid molecule according to claim 3, wherein each independent expression unit is driven by an exogenous or endogenous promoter operably linked thereto, or is driven by a promoter further upstream and has a nucleic acid sequence of a cleavable moiety (such as a 2A nucleic acid sequence or an IRES nucleic acid sequence) at the 5' end.

5. The nucleic acid molecule according to claim 3, wherein the number of the first, second, and third expression units are each independently 1, 2, or 3.

6. The nucleic acid molecule according to claim 3, wherein the number of the fourth expression unit is 0, 1, 2, 3, 4, or 5.

7. The nucleic acid molecule according to claim 3, wherein the nucleic acid molecule encodes 1, 2, or 3 identical or different CAR molecules.

8. The nucleic acid molecule according to any one of claims 1-7, which has a structure shown in the following Formula I:
ARMS-P1-CAR1-P2/L1-Z1-P3/L2-Z2-(P4/L3-Z3)m-ARM3 (I)
wherein,
ARM5 is absent or a 5' homologous arm;
ARM3 is absent or a 3' homologous arm;
P1 is absent, a splicing acceptor, or a first exogenous promoter;
CAR1 is a nucleic acid sequence encoding a first chimeric antigen receptor (CAR);
P2/L1 is a second exogenous promoter P2 or a nucleic acid sequence L1 encoding a cleavable moiety;
one of Z1 and Z2 is a nucleic acid sequence encoding IL-15 or a functionally active fragment thereof or a fusion protein thereof, and the other is a nucleic acid sequence encoding IL-21 or a functionally active fragment thereof or a fusion protein thereof;
L2 is a nucleic acid sequence L2 encoding a cleavable moiety;
P3 is a third exogenous promoter;
P4/L3 are each independently a fourth exogenous promoter P4 or a nucleic acid sequence L3 encoding a cleavable moiety;
Z3 is a nucleic acid sequence encoding an additional exogenous protein; and
m is 0, 1, 2, 3, 4, or 5.

9. The nucleic acid molecule according to claim 8, which has a structure shown in the following Formula II:
ARM5-P1-CAR1-L1-Z1-L2-Z2-ARM3 (II)
wherein ARM5, P1, CAR1, L1, Z1, L2, Z2, ARM3, and P2 are as defined above.

10. The nucleic acid molecule according to claim 8 or 9, wherein P1 is an exogenous promoter, such as a PGK promoter; and/or P2 is a PGK promoter.

11. The nucleic acid molecule according to any one of claims 8-10, wherein the 5' homologous arm and the 3' homologous arm are homologous to a target region in the genome of an immune cell, thereby enabling the knock-in of the nucleic acid sequence between the homologous arms into a predetermined site.

12. The nucleic acid molecule according to any one of claims 1-11, wherein the CAR comprises a target-binding domain targeting a tumor associated antigen, a target-binding domain targeting a viral antigen, a target-binding domain targeting an immune-related antigen, or a combination thereof.

13. The nucleic acid molecule according to claim 12, wherein the tumor associated antigen is selected from the group consisting of: GPC3, CD19, BCMA, GCC (GUCY2C), Her2, Claudin18.2, and Mesothelin; and the viral antigen is selected from EBV-gp350 and HBV s protein.

14. A vector comprising the nucleic acid molecule according to any one of claims 1-13.

15. The vector according to claim 14, wherein the vector is a plasmid.

16. The vector according to claim 14, wherein the plasmid is derived from a microorganism, and the content of genomic DNA of the microorganism accounts for less than 10 wt%, preferably ≤5 wt%, and more preferably ≤1 wt% of the total DNA in the plasmid.

17. A method for preparing a modified immune cell, wherein the method comprises transfecting an immune cell to be modified with the nucleic acid molecule according to any one of claims 1-13, or the vector according to any one of claims 14-16, wherein the method is an electrotransfection method based on a donor plasmid in combination with a targeting nuclease, or an electrotransfection method based on a donor plasmid.

18. The method according to claim 17, wherein the method comprises:
(a) providing a donor plasmid, wherein the donor plasmid comprises a nucleic acid molecule of claim 1, the plasmid is derived from a microorganism and the content of genomic DNA of the microorganism accounts for less than 10 wt% (preferably ≤5 wt%, and more preferably ≤1 wt%) of the total DNA in the plasmid; and
(b) transfecting an immune cell to be modified with the plasmid, thereby allowing the cell to comprise and/or express the nucleic acid molecule.

19. The method according to claim 18, wherein in step (b), the transfection comprises electrotransfection.

20. The method according to claim 18, wherein in step (b), the transfection is carried out in the presence of a gene editing system, thereby integrating the nucleic acid molecule into a specific location in the genome of the cell.

21. The method according to claim 20, wherein the gene editing system comprises a targeting nuclease and a guide RNA.

22. The method according to claim 21, wherein the targeting nuclease includes transcription activator-like effector nuclease (TALEN), zinc-finger nuclease (ZFN), transposase, integrase, and Cas protein, preferably Cas 9 protein.

23. The method according to claim 22, wherein the transposase includes PiggyBac (PB) transposase, and/or Sleeping Beauty (SB) transposase.

24. The method according to claim 20, wherein the gene editing system comprises a ribonucleoprotein complex (RNP), and the RNP comprises the Cas protein and the guide RNA.

25. The method according to claim 17, which comprises: transfecting the immune cell to be modified with a transfection composition comprising the nucleic acid molecule according to any one of claims 1-13 or the vector according to any one of claims 14-16, thereby allowing the cell to comprise and/or express the nucleic acid molecule, wherein at least a portion of the nucleic acid molecule or the vector is obtained from a host cell; and in the portion of the nucleic acid molecule or the portion of the vector obtained from the host cell, the content of genomic DNA from the host cell is about 10% (w/w) (preferably ≤5 wt%, and more preferably ≤1 wt%) or less.

26. The method according to claim 25, wherein the immune cell includes a T lymphocyte, B lymphocyte, NK cell, macrophage, dendritic cell, monocyte, granulocyte, and/or mast cell.

27. The method according to claim 25 or 26, wherein the immune cell includes a peripheral blood lymphocyte.

28. The method according to any one of claims 25-27, wherein the immune cell is a primary cell.

29. The method according to any one of claims 25-28, wherein the content of the genomic DNA of the host cell is less than about 1% (w/w).

30. The method according to any one of claims 25-29, wherein the content of the genomic DNA of the host cell is determined by qPCR method.

31. A transfection composition, which comprises the nucleic acid molecule according to any one of claims 1-13 or the vector according to any one of claims 14-16, wherein at least a portion of the nucleic acid molecule or the vector is obtained from a host cell; and in the portion of the nucleic acid molecule or the portion of the vector obtained from the host cell, the content of genomic DNA from the host cell is about 10% (w/w) or less.

32. The transfection composition according to claim 31, wherein the content of the genomic DNA of the host cell is less than about 1% (w/w).

33. The transfection composition according to claim 31, wherein the content of the genomic DNA of the host cell is less than about 9‰ (w/w).

34. An immune cell prepared by the method according to any one of claims 17-30.

35. A modified immune cell comprising or expressing the nucleic acid molecule according to any one of claims 1-13 or the vector according to any one of claims 14-16.

36. The immune cell according to claim 35, wherein one or more additional genes is knocked out or knocked down in the modified immune cell.

37. The immune cell according to claim 36, wherein the additional gene is different from a target gene into which the nucleic acid molecule or a functionally active fragment thereof is integrated.

38. The immune cell according to claim 36 or 37, wherein the one or more additional genes include an immune checkpoint gene.

39. The immune cell according to any one of claims 36-38, wherein the one or more additional genes include PD-1, CD95 and/or CD52.

40. A pharmaceutical composition comprising the nucleic acid molecule according to any one of claims 1-13, the vector according to any one of claims 14-16, and/or the cell according to any one of claims 34-39.

41. Use of the nucleic acid molecule according to any one of claims 1-13, the vector according to any one of claims 14-16, the cell according to any one of claims 34-39, and/or the pharmaceutical composition according to claim 40 for the preparation of a medicament.

42. The use according to claim 41, wherein the medicament is used for the prevention, treatment, and/or alleviation of a cancer.

43. A method for preventing, treating, and/or alleviating a disease or disorder in a subject, comprising administering an effective amount of the nucleic acid molecule according to any one of claims 1-13, the vector according to any one of claims 14-16, the cell according to any one of claims 34-39, and/or the pharmaceutical composition according to claim 40 to the subject.

44. The method according to claim 43, wherein the disease or disorder is a cancer or viral infection.

45. The nucleic acid molecule according to any one of claims 1-13, the vector according to any one of claims 14-16, the cell according to any one of claims 34-39, and/or the pharmaceutical composition according to claim 40 for use in preventing, treating, and/or alleviating a disease or disorder in a subject.
